# EUROPEAN PATENT APPLICATION

(11) **EP 3 858 440 A2**
(43) Date of publication of application: **04.08.2021**
(21) Application number: 20210797.5
(22) Date of filing: 18.11.2016
(51) Int. Cl.: A61P 37/06, A61K 31/00, A61K 38/00, A61K 47/00, A61K 33/00, A61K 9/00, A61K 9/12

(54) **B-1A LYMPHOCYTE AND/OR MACROPHAGE TARGETING AND ACTIVATION TO TREAT MEDICAL CONDITIONS WITH INFLAMMATORY OR AUTOIMMUNE COMPONENTS**

(30) Priority: 18.11.2015 US 201562256814 P
(62) Divisional of application: 16867271.5
(71) Applicant: The Board of Trustees of the Leland Stanford Junior University, Stanford, CA 94305-2038 (US)
(72) Inventor: STEINMAN, Lawrence, Stanford, California 94305 (US); KURNELLAS, Michael, San Mateo, CA California 94404 (US); ROTHBARD, Jonathan, San Francisco, California 94105 (US)
(74) Representative: Hobson, David James

(57) **Abstract**

Methods and formulations to target and activate B-1a lymphocytes and/or macrophages are described. The methods and formulations can be delivered to the respiratory tract to target B-1a lymphocytes and/or macrophages in the pleural cavity. Conditions with inflammatory or autoimmune components can be treated. B-1a lymphocytes and/or macrophages can be targeted and activated using fibrils or fibril-forming peptides.

## Description

### CROSS REFERENCE TO RELATED APPLICATION

**This** application claims priority to U.S. Provisional Patent Application No. 62/256,814 filed November 18, 2015, the entire contents of which are incorporated by reference herein.

### FIELD OF THE DISCLOSURE

Methods and formulations to target and activate B-1a lymphocytes and/or macrophages are described. The methods and formulations can be delivered to the respiratory tract to target B-1a lymphocytes and/or macrophages in the pleural cavity. Conditions with inflammatory or autoimmune components can be treated. B-1a lymphocytes and/or macrophages can be targeted and activated using fibrils or fibril-forming peptides.

### BACKGROUND OF THE DISCLOSURE

B cells of the immune system produce antibodies. Historically, all B cells were thought to produce antibodies following exposure to a pathogen. It is now known, however, that B2 cells produce antibodies following exposure to a pathogen while B1 cells produce antibodies in the absence of exposure to pathogen. B1 cells also differ functionally from B2 cells in efficiently presenting antigen to T cells, and in displaying evidence of tonic signaling in the absence of specific stimulation.

The antibodies produced by B1 cells (referred to as "natural" or "resting" immunoglobulins (e.g., resting IgM or IgA)) differ from other antibodies in that they are more broadly reactive and repertoire-selected. It is now known that resting immunoglobulins play an important role in early defense against bacterial and viral infections, and also play a role in a wide variety of diseases, through recognition of self-antigens and binding of cellular debris.

B1 cells can be further subdivided into B-1a lymphocytes and B-1b lymphocytes. Both types have the marker profile CD20⁺CD27⁺CD43⁺CD70⁻. B-1a lymphocytes are CD5⁺ while B-1b lymphocytes are CD5⁻. B-1a and B-1b precursor cells also differ in CD138 expression levels.

Macrophages are white blood cells produced by the division of monocytes. Monocytes and macrophages are phagocytes, and play a role in innate immunity (non-specific immune defenses) as well as helping to initiate adaptive immunity (specific defense mechanisms). These cells phagocytose (engulf and then digest) cellular debris and pathogens either as stationary or as mobile cells. When activated by pathogens or by other mechanisms, macrophages stimulate and recruit lymphocytes and other immune cells to respond to the insult.

Although macrophages play a vital role in host immune defenses, activated macrophages are also involved in the progression of a number of diseases and disorders. Activated macrophages elicit massive leukocyte infiltration and flood the surrounding tissue with inflammatory mediators, pro-apoptotic factors, and matrix degrading proteases. These actions can result in inflammation that can dismantle tissues to the point of inflicting serious injury. Tissue destruction perpetrated by macrophage-induced inflammation has been associated with the development of tumors, autoimmune disorders, and other conditions.

### SUMMARY OF THE DISCLOSURE

The current disclosure provides that targeting and activating B-1a lymphocytes and/or macrophages along the respiratory tract can treat medical conditions having inflammatory or autoimmune components. B-1a lymphocytes and/or macrophages can be effectively targeted and activated using fibrils or fibril-forming peptides.

### BRIEF DESCRIPTION OF THE FIGURES

Many of the drawings submitted herein are better understood in color, which is not available in patent application publications at the time of filing. Applicants consider the color versions of the drawings as part of the original submission and reserve the right to present color images of the drawings in later proceedings.

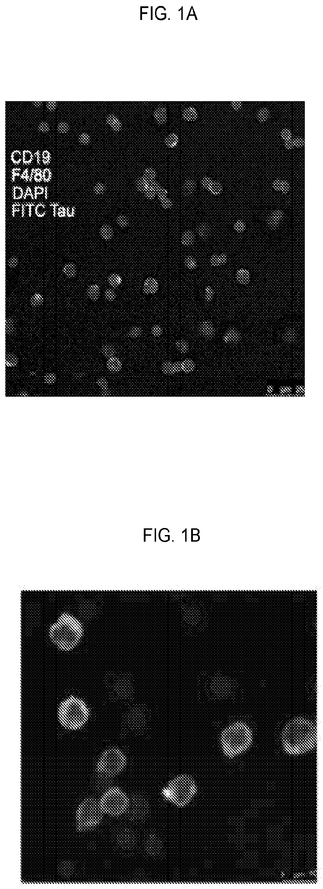

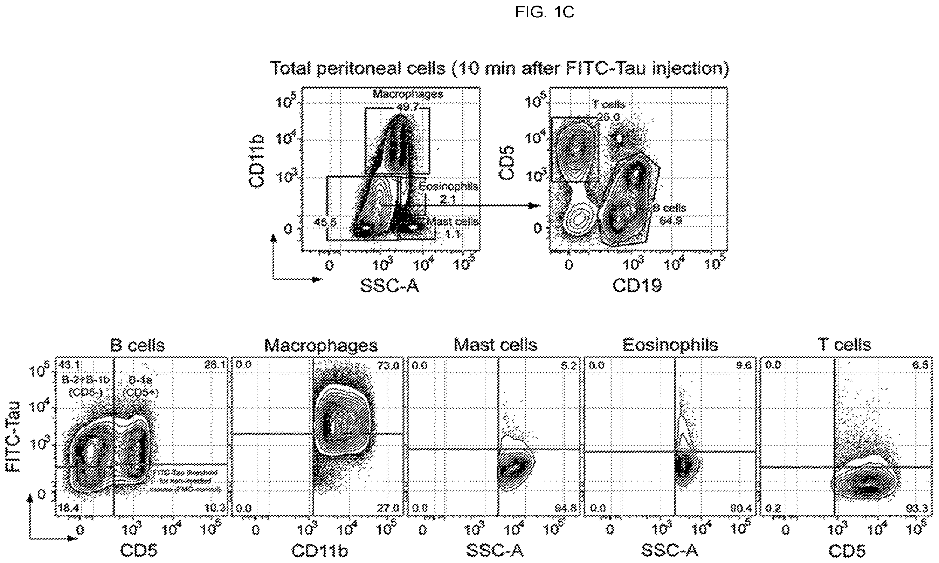

FIGs. 1A-1C Amyloid fibrils composed of Tau 623-628 bind and are endocytosed by B-1a lymphocytes (CD19 CD5) and LPMs (CD11bhi F4/80hi peritoneal MΦs). (FIG. 1A) A composite of a confocal image (40x magnification) and (FIG. 1B) a single Z-cut (63x) from a movie constructed from the set of confocal images of peritoneal cavity cells from wild type mice injected with 10µg FITC-Tau 623-628 and stained with rat anti-mouse CD19 (PE), F4/80 (Alexa Fluor 647), and DAPI. Cells were visualized using a Lei ca TCS SP8 white light laser confocal microscope. (FIG. 1C) Wild type mice were injected with 10µg FITC-Tau 623-628 and peritoneal cells were isolated after 10 minutes and compared with peritoneal cells from an uninjected animal. The cells were washed and stained with rat anti-mouse CD11b (PE), CD19 (Pacific Blue), CD5 (APC), CD3 (PerCP-Cy5.5), and propidium iodide for viability. The gates demarcating the different cell types are shown in the two upper panels (10 minutes post-injection), and the amount of staining with FITC-Tau for each cell type is shown in the five lower panels.

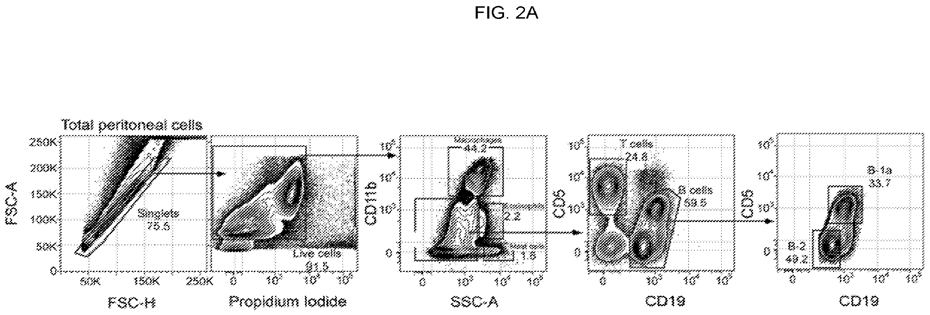

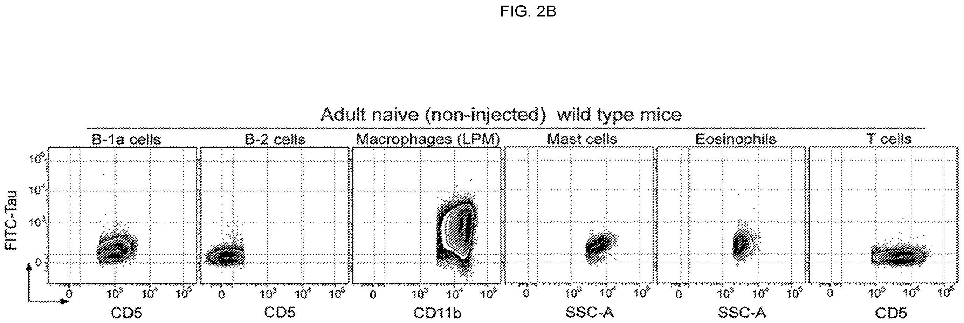

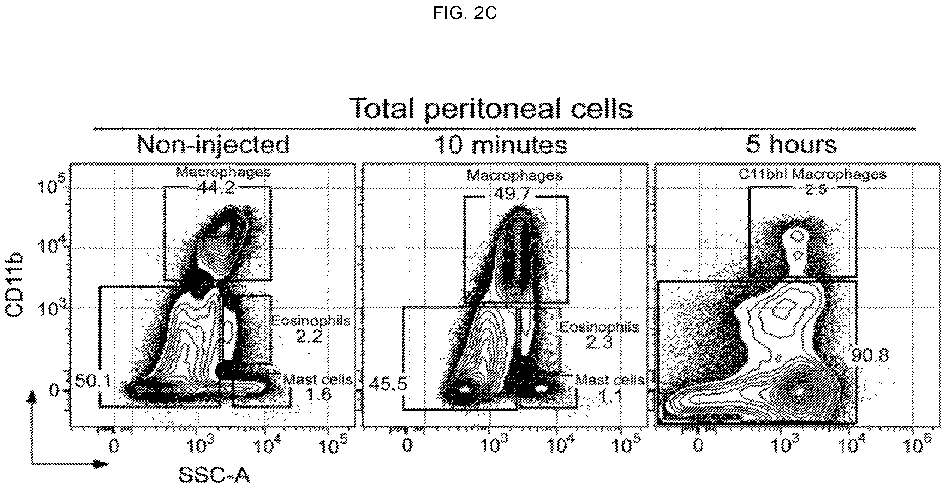

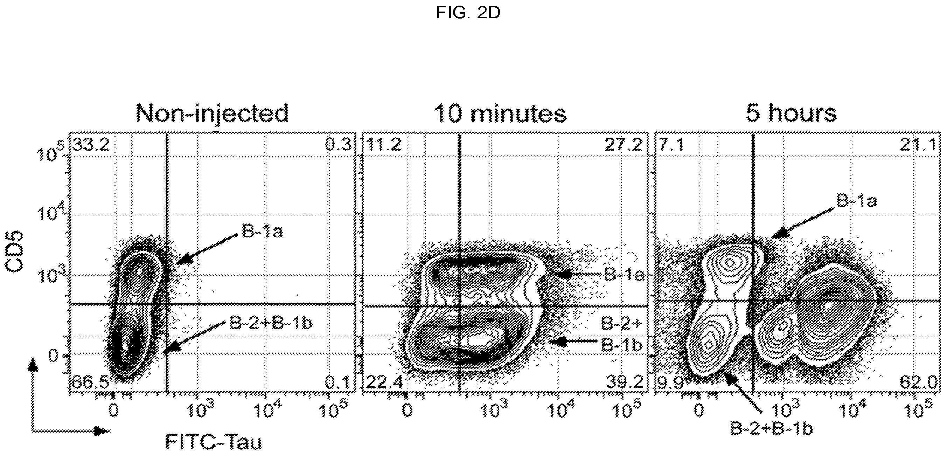

FIGs. 2A-2D. Gating strategy used to identify the various murine peritoneal cell populations. Total cells from peritoneal lavage were stained with rat anti- mouse CD11b (PE), CD19 (Pacific Blue), CD5 (APC), CD3 (PerCP-Cy5.5), and propidium iodide (viability marker). (FIG. 2A) Peritoneal cells from unimmunized mice were sequentially gated to identify macrophages (CD11b), eosinophils (CD11bSSC^{hi}), mast cells (CD11b-SSC^{hi}), T cells (CD11b⁻CD5^{hi}CD19⁻), B-1a (CD19^{hi}CD5⁺) and B-2 (CD19⁺CD5⁻) cells. (FIG. 2B) The various peritoneal cell subsets from unimmunized mice were analyzed for their levels of auto-fluorescence emitted into the FITC channel to establish the negative threshold (i.e. FITC-Tau^{negative}), which was used to determine the binding of FITC-Tau shown in FIG. 1. (FIG. 2C) Total live peritoneal cells 10min and 5h after intraperitoneal injection of 10µg FITC-Tau were processed and stained as described above and gated to identify macrophages, eosinophils, and mast cells. CD11b^{hi} macrophages are dramatically decreased 5h post-injection, i.e. from >40% (left panel) to <3% (right panel). (FIG. 2D) Panels show peritoneal B cells (B-1 and B-2) 10min and 5h after FITC-Tau injection. Ten minutes after injection majority (>70%) of peritoneal B-1a and B-2 cells endocytose FITC-Tau (middle panel). In contrast, 5h after injection the percentage of peritoneal B-1a and B-2 cells are dramatically decreased, and the remaining resident B-1a and B-2 cells are negative for FITC-Tau (right panel).

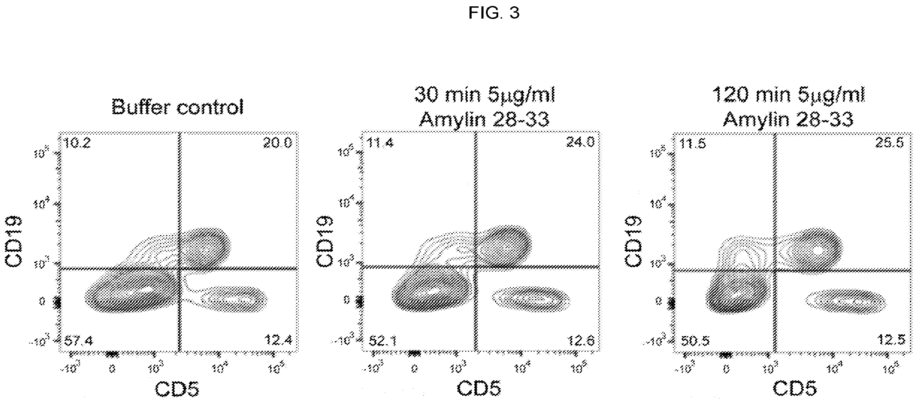

FIG. 3. Incubation of Amylin 28-33 with peritoneal cells in vitro does not affect cell viability. Peritoneal cells were isolated by lavage of C57BL/6 mice, aliquoted 500,000 cells per well of a 96 well round bottomed plate and treated with 1.0, 0.5, 0.25, 0.125 µg/well of Amylin 28-33 for 30 minutes or 120 minutes in DMEM with 10% FCS. At the end of the incubation the cells were spun, supernatant aspirated, and the cells were stained with rat anti-mouse CD11b (Pacific Blue), CD5 (PE), CD19 (APC), and incubated for 30 minutes on ice. The cells were washed once, aspirate transferred to FACS tubes, PI added, and analyzed by flow cytometry.

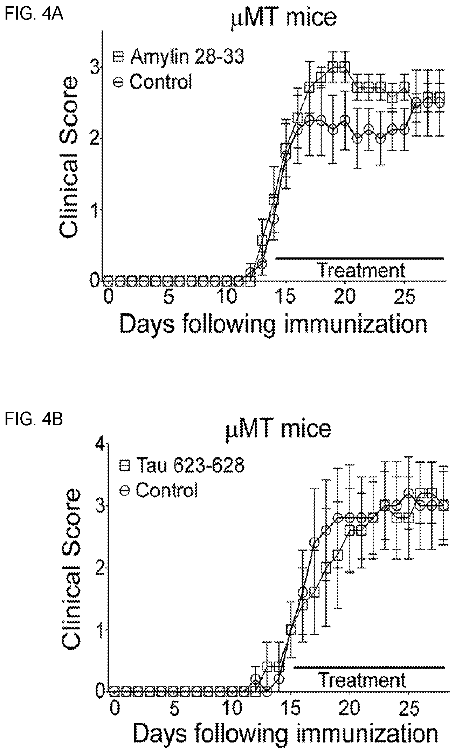

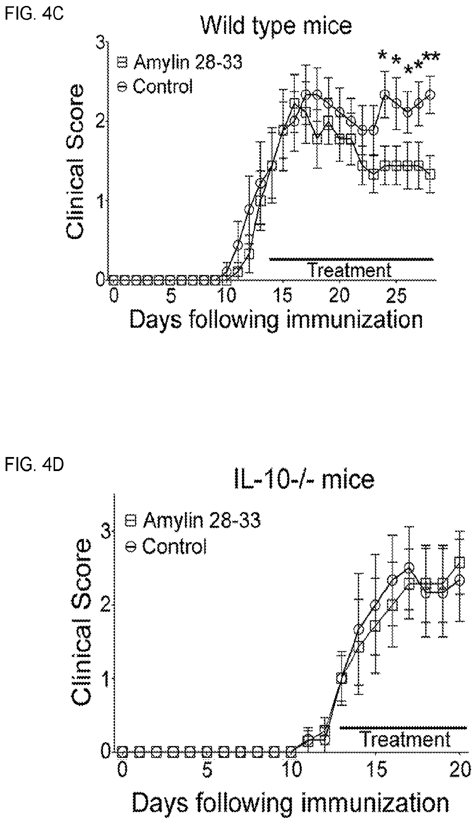

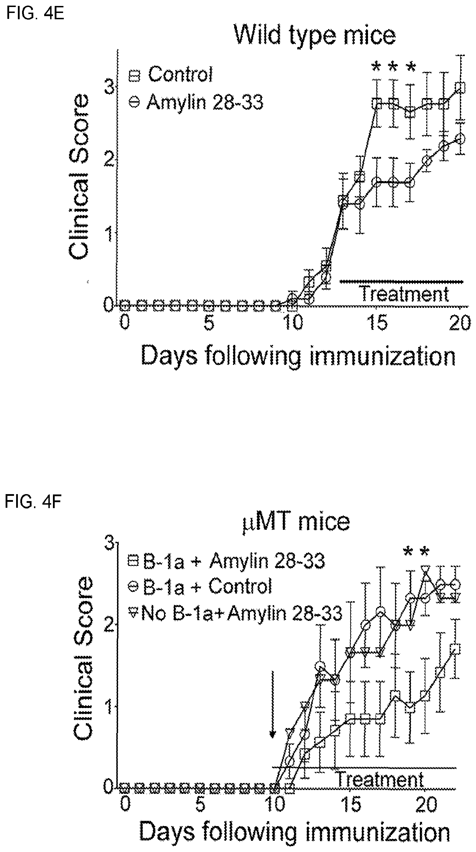

FIGs. 4A-4F. B-1a lymphocytes and IL-10 are necessary for therapeutic efficacy of amyloidogenic peptides. µMT mice were treated daily with intraperitoneal (i.p.) injections of 10µg (FIG. 4A) Amylin 28-33 (n=10) or (FIG. 4B) Tau 623-628 (n=10) at onset of symptoms. (FIG. 4C) Wild type EAE mice were treated daily i.p. with 10µg Amylin 28-33 (n=10). (FIG. 4D) IL-10 deficient (n=7) and (FIG. 4E) wild type (n=10) mice were treated daily with 10µg Amylin 28-33. Values in graph represent mean +/- S.E.M. *p<0.05 and **p<0.005 by Mann-Whitney U test. All experiments were repeated at least twice. (FIG. 4F) Adoptive transfer of 3.5x10⁵ B-1a cells into µMT mice prior to the signs of EAE were treated daily i.p. with 10µg Amylin 28-33 or control buffer (n=6). Mice without transfer of cells were treated with 10µg Amylin 28-33. Values in graph represent mean +/- S.E.M. *p<0.05 by Mann-Whitney U test.

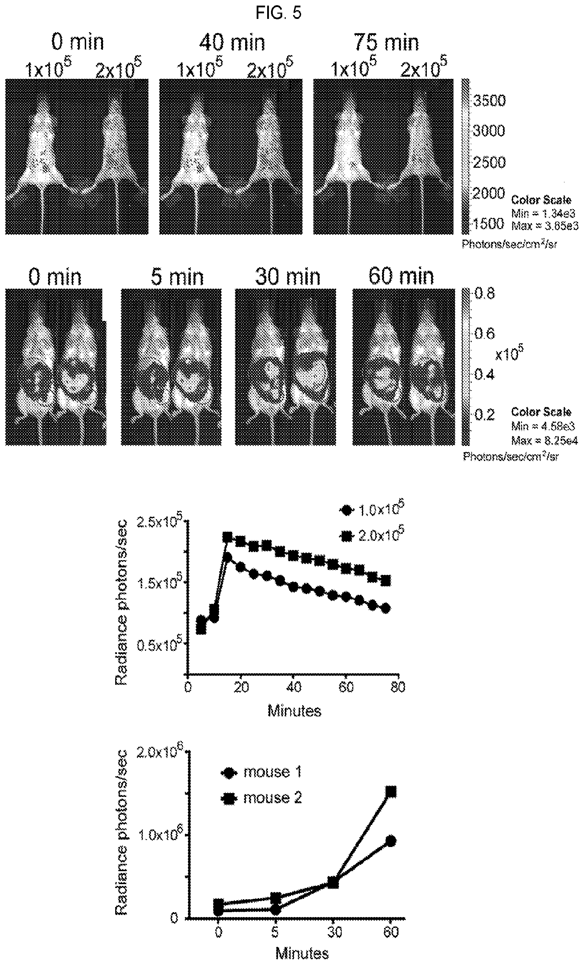

FIG. 5. Real time measurement of trafficking of adoptively transferred B-1a lymphoytes and LPMs using bioluminescence induced by amyloidogenic peptides. (FIG. 5, top left) 1x10⁵ and 2x10⁵ B-1a sorted cells were injected i.p. into C57BL/6 albino mice and the substrate luciferin, 5 minutes later bioluminescence images were obtained using CCD camera serially every 5 minutes. Bioluminescent signal was detectable from the peritoneum area diminishing with time and relocalizing in the inguinal lymph nodes area. (FIG. 5, top right) Quantification of B-1a Luc⁺ cell distribution by measuring light emission from the C57BL/6 albino mice over time after injection of Tau 623-628. (FIG. 5, bottom left) 1x10⁶ luc⁺ LPMs were injected i.p. into C57BL/6 albino mice and luciferase was re-injected every 30 minutes before an image was obtained. The MΦs egressed from the peritoneum (larger circle) and migrated to different tissues including the inguinal lymph nodes (smaller circle). (FIG. 5, bottom right) Quantification of MΦs cells migrating to the lymph nodes from the peritoneum by measuring the light emission. BLI measured from the lymph nodes increased by 10 fold at 60 min compared to the initial measurement at 5 min.

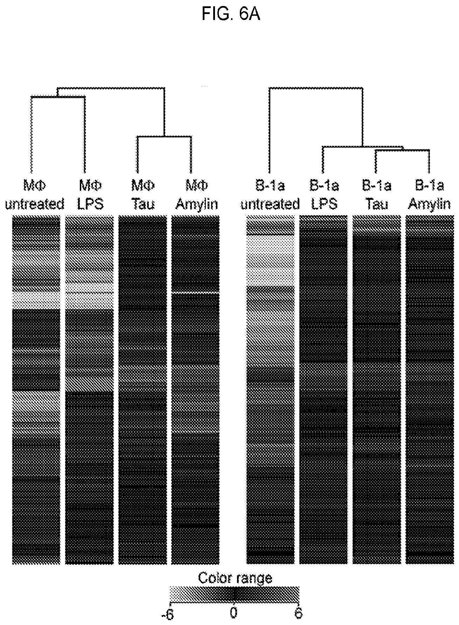

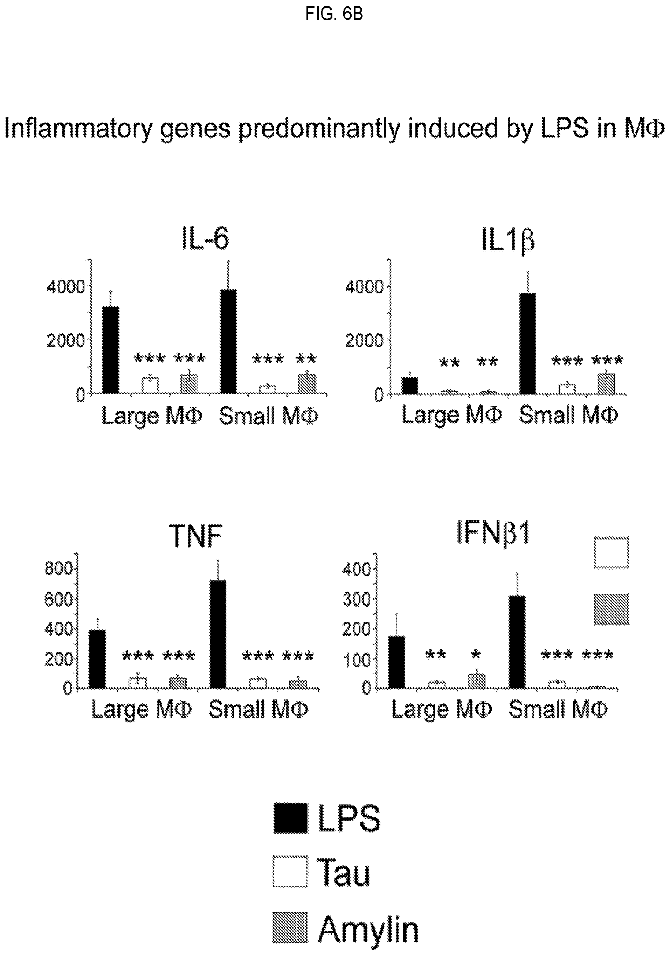

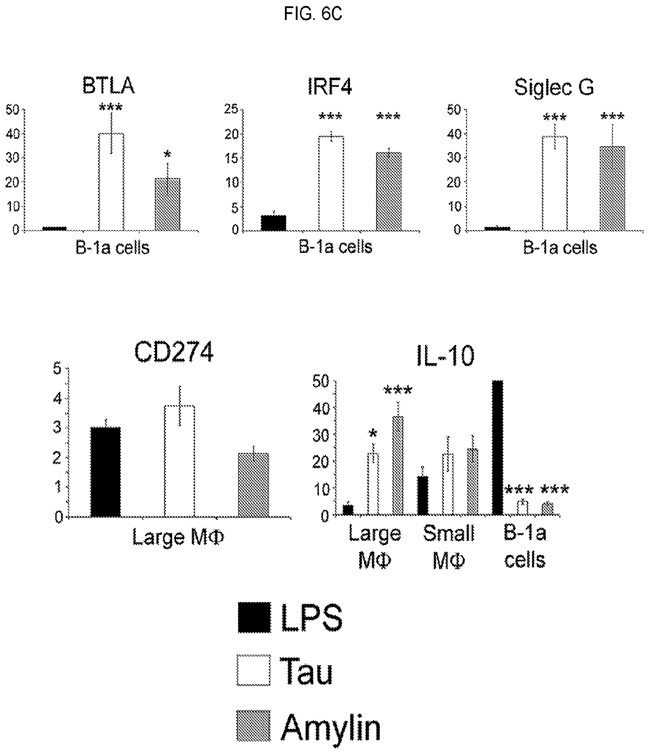

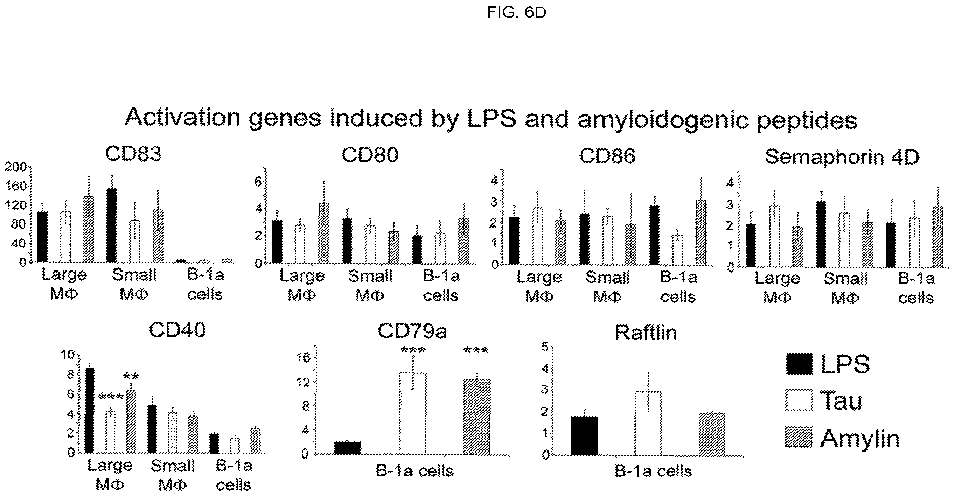

FIGs. 6A-6D. Amyloid fibrils, composed of either Tau 623-628 or Amylin 28-33, induce a different pattern of gene expression than LPS in B-1a lymphocytes and peritoneal MΦs, SPM and LPM. (FIG. 6A) Differential gene expression (720 annotated genes) expressed as a heatmap induced by LPS and the two types of amyloid fibrils. RNA isolated from purified B-1a lymphocytes and CD11b ^{high} MΦs isolated from groups of three C57BL/6 mice injected with either 10µg LPS, Amylin 28-33, Tau 623-628, or buffer. Each of the RNA samples was hybridized to a microarray plate (SurePrint G3 Mouse; Agilent Technologies) and quantified and analyzed using GeneSpring and Ingenuity software. Measurement by qPCR of gene induction compared to cells from uninjected animals of sets of genes representing (FIG. 6B) inflammatory cytokines, (FIG. 6C) immune suppressive genes, or (FIG. 6D) activation genes. Graphs represent the results of three separate measurements. The full set of data has been deposited in the Geo databank.

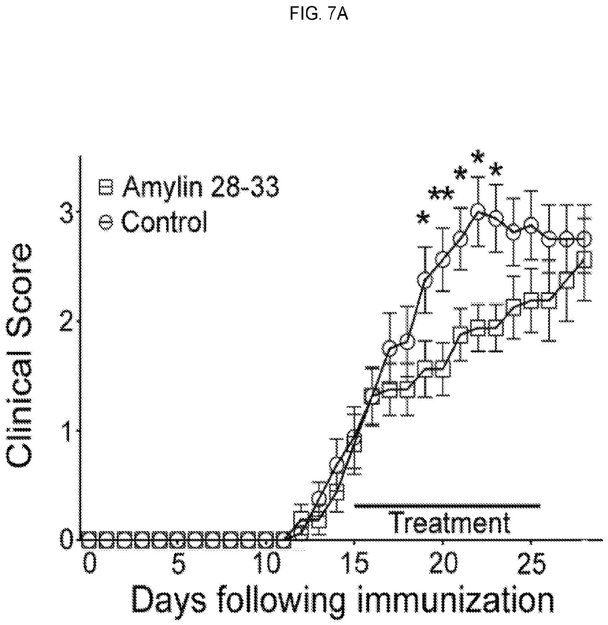

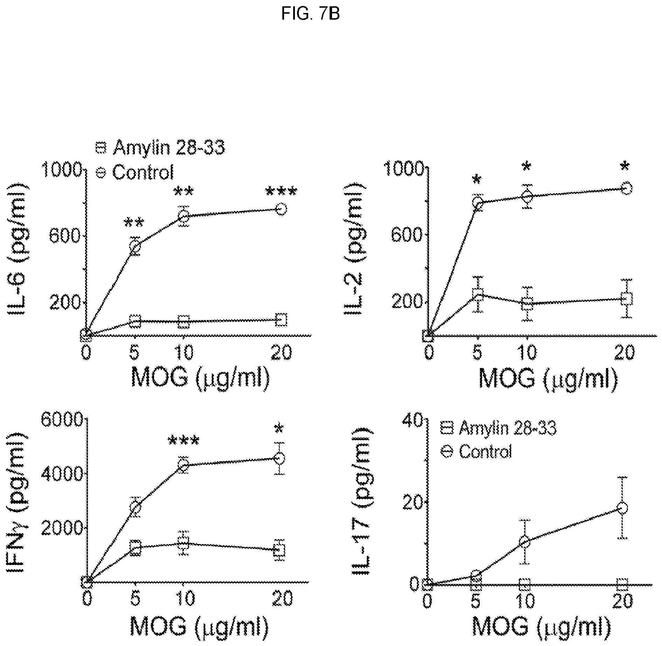

FIGs. 7A and 7B. Intranasal delivery of Amylin 28-33 reduces the clinical signs of EAE. (FIG. 7A) Mice with EAE were treated daily intranasally with 10µg Amylin 28-33 (n=16) for 10 days at onset of symptoms. Values in graph represent mean +/- S.E.M. *p<0.05 and **p<0.005 by Mann-Whitney U test. Experiments were repeated twice. (FIG. 7B) Splenocytes from EAE mice treated with 10µg Amylin 28-33 were stimulated with 0, 5, 10 and 20µg/ml MOG₃₅₋₅₅ and the levels of cytokines IL-6, IFNγ, IL-2, and IL-17 were measured (n=3). Values in graph represent mean +/- S.E.M. *p<0.01, **p<0.001, and ***p<0.0001 by student's t test.

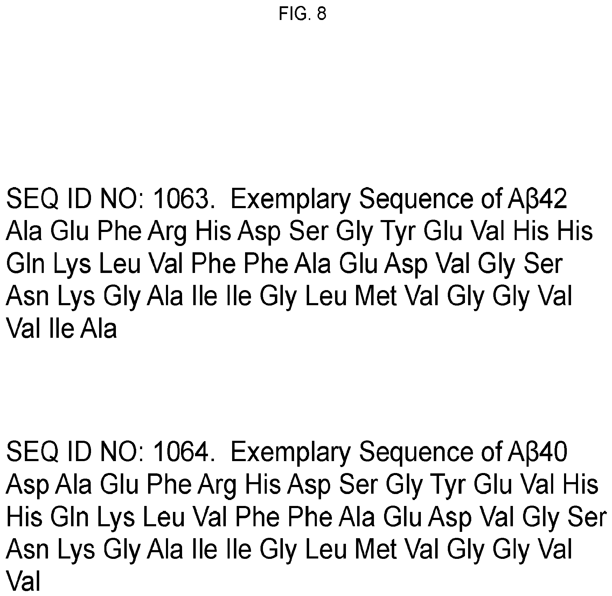

FIG. 8. Exemplary sequences of Aβ42 and Aβ40 (SEQ ID NOs: 1063 and 1064).

### DETAILED DESCRIPTION

B cells of the immune system produce antibodies. Historically, all B cells were thought to produce antibodies following exposure to a pathogen. It is now known, however, that B2 cells produce antibodies following exposure to a pathogen while B1 cells produce antibodies in the absence of exposure to pathogen. B1 cells also differ functionally from B2 cells in efficiently presenting antigen to T cells, and in displaying evidence of tonic signaling in the absence of specific stimulation.

The antibodies produced by B1 cells (referred to as "natural" or "resting" immunoglobulins (e.g., resting IgM or IgA)) differ from other antibodies in that they are more broadly reactive and repertoire-selected. It is now known that resting immunoglobulins play an important role in early defense against bacterial and viral infections, and also play a role in a wide variety of diseases, through recognition of self-antigens and binding of cellular debris.

B1 cells can be further subdivided into B-1a lymphocytes and B-1b lymphocytes. Both types have the marker profile CD20⁺CD27⁺CD43⁺CD70⁻. B-1a lymphocytes are CD5⁺ while B-1b lymphocytes are CD5⁻. B-1a and B-1b precursor cells also differ in CD138 expression levels.

Macrophages are white blood cells produced by the division of monocytes. Monocytes and macrophages are phagocytes, and play a role in innate immunity (non-specific immune defenses) as well as helping to initiate adaptive immunity (specific defense mechanisms). These cells phagocytose (engulf and then digest) cellular debris and pathogens either as stationary or as mobile cells. When activated by pathogens or by other mechanisms, macrophages stimulate and recruit lymphocytes and other immune cells to respond to the insult.

Although macrophages play a vital role in host immune defenses, activated macrophages are also involved in the progression of a number of diseases and disorders. Activated macrophages elicit massive leukocyte infiltration and flood the surrounding tissue with inflammatory mediators, pro-apoptotic factors, and matrix degrading proteases. These actions can result in inflammation that can dismantle tissues to the point of inflicting serious injury. Tissue destruction perpetrated by macrophage-induced inflammation has been associated with the development of tumors, autoimmune disorders, and other conditions.

The current disclosure provides that targeting and activating B-1a lymphocytes and/or macrophages along the respiratory tract can treat medical conditions having inflammatory or autoimmune components. B-1a lymphocytes and/or macrophages can be effectively targeted and activated using fibrils or fibril-forming peptides.

**The** respiratory tract is the structure involved in the exchange of gases between the atmosphere and the blood stream. The respiratory tract encompasses the upper airways, including the oropharynx and larynx, followed by the lower airways, which include the trachea followed by bifurcations into the bronchi and bronchioli. The upper and lower airways are called the conducting airways. The terminal bronchioli then divide into respiratory bronchioli which lead to the ultimate respiratory zone, the alveoli or deep lung where the exchange of gases occurs. The alveolar surface area is the largest in the respiratory system where active agent (e.g., drug) absorption occurs.

The pleura is a membrane which surrounds the lungs and has a two-layer structure including an outer or parietal pleura that is normally attached to the chest wall and an inner or visceral pleura that covers the lungs and adjoining structures. The space between the inner and outer pleurae is referred to as the pleural cavity, pleural space, or intrapleural space.

Particular embodiments disclosed herein include administration of formulations including active agents to various portions of the respiratory tract. Particular embodiments include administration to the respiratory tract to target and activate B-1a lymphocytes and/or macrophages in the pleural cavity.

Pulmonary administration refers to administration of formulations so that they reach the lungs and in particular embodiments the alveolar regions of the lung. In particular embodiments, pulmonary administration occurs by inhalation or administration through the nose or mouth.

The geometry of the airways is a major barrier for active agent dispersal within the lungs. There are three basic mechanisms of deposition: impaction, sedimentation, and Brownian motion (Padfield. 1987. In: D. Ganderton & T. Jones eds. Drug Delivery to the Respiratory Tract, Ellis Harwood, Chicherster, U.K.). Impaction occurs when particles are unable to stay within the air stream, particularly at airway branches. They are adsorbed onto the mucus layer covering bronchial walls and cleaned out by mucocilliary action. Impaction most often occurs with particles over 5 µm in diameter. Smaller particles (<5 µm) stay within the air stream more readily and can be transported deep into the lungs. Sedimentation often occurs in the lower respiratory system where airflow is slower. Very small particles (<0.6 µm) can deposit by Brownian motion. In particular embodiments, this deposition is undesirable because it is not targeted to the alveoli (Worakul & Robinson. 2002. In: Polymeric Biomaterials, 2nd ed. S. Dumitriu ed. Marcel Dekker. New York).

Formulations can be delivered to various portions of the respiratory tract in, for example, droplets, dry powder forms, foams, gels, mists, particles, solutions, sprays, suspensions, and/or vapors. In particular embodiments, formulations can be aerosolized.

An aerosol refers to any preparation of a fine mist of particles, typically less than 10 microns in diameter. In particular embodiments, the mean diameter for aqueous formulations of aerosol particles is, for example between 0.1 and 30 microns, between 0.5 and 20 microns, between 0.5 and 10 microns or 5 microns.

Aerosols for the delivery of active agents to the respiratory tract are described in, for example, Adjei and Garren, J. Pharm. Res., 7: 565-569 (1990); Zanen and Lamm, J. Int. J. Pharm., 114: 111-115 (1995); Gonda, Critical Reviews in Therapeutic Drug Carrier Systems, 6:273-313 (1990); and Moren, "Aerosol dosage forms and formulations," in: Aerosols in Medicine, Principles, Diagnosis and Therapy, Moren, et al., Eds. Esevier, Amsterdam, 1985.

Aerosols are typically formed by atomizing a solution or suspension under pressure through a nebulizer, a pressurized can, a continuous sprayer, or through the use of a metered dose inhaler ("MDI") or pressurized metered dose inhaler (pMDI).

Nebulizers create a fine mist from a solution or suspension, which is inhaled by a subject. Nebulized saline solutions have long been delivered chronically to the lungs with small amounts of active agents, such as beta agonists, corticosteroids, or antibiotics. Often these saline solutions are hypertonic (sodium chloride concentrations greater than 0.9% by weight, often as high as 5% by weight) and generally they are delivered for up to 20 minutes. VENTOLIN® Inhalation Solution (GSK) is an albuterol sulfate solution that can be nebulized for administration to the lungs. A VENTOLIN® solution for nebulization can be prepared by mixing, for example, 1.25-2.5 mg of albuterol sulfate in 0.25-0.5 mL of aqueous solution into sterile normal saline to achieve a total volume of 3 mL. No adverse effects have been found to be associated with the delivery to the lungs by VENTOLIN® nebulization. Nebulizing devices are described in, for example, U.S. Patent No. 5,709,202.

pMDIs typically include a pressurized canister having a meter valve, wherein the canister is filled with a solution or suspension and a propellant. In particular embodiments, the solution or suspension acts as the propellant. In other embodiments, propellants can include hydrofluoroalkane (HFA) propellants (e.g., Proventil® HFA (Schering-Plough Corporation) or FREON® (E. I. Du Pont De Nemours and Co. Corp.). When released from the canister, the formulation is a fine mist, and the propellant and solvent may wholly or partially evaporate due to the decrease in pressure.

Dry powder formulations can also be used. Dry powder formulations (DPFs) with large particle size have improved flowability characteristics, such as less aggregation, easier aerosolization, and potentially less phagocytosis. Dry powder aerosols for inhalation are generally produced using particles with mean diameters primarily in the range of less than 5 microns, although in particular embodiments, the range is between one and ten microns in aerodynamic diameter. Large "carrier" particles (containing no active agent) have been co-delivered with dry powder aerosols to aid in achieving efficient aerosolization among other possible benefits. Particles with degradation and release times ranging from seconds to months can be designed and fabricated by methods known in the art.

In particular embodiments, "aerodynamically light particles" can be used in dry powder formulations. "Aerodynamically light particles" are particles having a mean or tap density less than 0.4 g/cm³. Tap density is a standard measure of the envelope mass density. The envelope mass density of an isotropic particle is defined as the mass of the particle divided by the minimum sphere envelope volume in which it can be enclosed. Features contributing to low tap density include irregular surface texture and porous structure. The tap density of particles of a dry powder may be obtained by the standard USP tap density measurement.

The currently preferred mean diameter for aerodynamically light particles for inhalation is between 3 and 30 microns in diameter, or between 5 and 7 microns. The aerodynamically light particles may be fabricated with the appropriate material, surface roughness, diameter and tap density for localized delivery to selected regions of the respiratory tract such as the deep lung or upper airways. For example, higher density or larger particles may be used for upper airway delivery. Similarly, a mixture of different sized particles, provided with the same or different active agent may be administered to target different regions of the lung in one administration.

In particular embodiments, conductive formulations can be used for administration to the respiratory tract. Conductive formulations are particularly useful to suppress particle exhalation. Solution conductivity is a product of the ionic strength, concentration, and mobility (the latter two contribute to the conductivity of the formulation as a whole). Any form of ionic components (anionic, cationic, or zwitterionic) can be used. These conductive materials may alter the properties of the mucosal lining of the respiratory tract by acting, for example, as a cross-linking agent within the mucus. The ionic components in conductive formulations can interact with the strongly linked anionic glycoproteins within normal tracheobronchial mucus. These interactions influence the state of the air/liquid surface of the airway lining fluid and transiently the nature of the physical entanglements due to covalent and noncovalent interactions, including hydrogen bonding, hydrophobic, and electrostatic interactions.

Substances useful to form conductive formulations are those that are easily ionized in an aqueous or organic solvent environment (also referred to herein as "conductive agents") Examples of such substances include salts, ionic surfactants, charged amino acids, charged proteins, or other charged materials.

Suitable salts include any salt form of sodium, potassium, magnesium, calcium, aluminum, silicon, scandium, titanium, vanadium, chromium, cobalt, nickel, copper, manganese, zinc, tin, and similar elements. Examples include sodium chloride, sodium acetate, sodium bicarbonate, sodium carbonate, sodium sulfate, sodium stearate, sodium ascorbate, sodium benzoate, sodium biphosphate, sodium phosphate, sodium bisulfite, sodium citrate, sodium borate, sodium gluconate, calcium chloride, calcium carbonate, calcium acetate, calcium phosphate, calcium alginite, calcium stearate, calcium sorbate, calcium sulfate, calcium gluconate, magnesium carbonate, magnesium sulfate, magnesium stearate, magnesium trisilicate, potassium bicarbonate, potassium chloride, potassium citrate, potassium borate, potassium bisulfite, potassium biphosphate, potassium alginate, potassium benzoate, magnesium chloride, cupric sulfate, chromium chloride, stannous chloride, and sodium metasilicate.

Suitable ionic surfactants include sodium dodecyl sulfate (SDS) (also known as sodium lauryl sulfate (SLS)), magnesium lauryl sulfate, Polysorbate 20, Polysorbate 80, and similar surfactants.

Suitable charged amino acids include L-lysine. L-arginine, histidine, aspartate, glutamate, glycine, cysteine, and tyrosine.

Suitable charged proteins include calmodulin (CaM), troponin C, and charged phospholipids. Negatively charged phospholipids include phosphatidylinositol, phosphatidylserine, phosphatidylglycerol, phosphatidic acid, cardiolipins, dialkanoyl phosphatidyl glycerols (dipalmitoyl phosphatidyl glycerol and dimyristoyl phosphatidyl glycerol), phosphatidylinositol 4-phosphate (PIP), phosphatidylinositol 4,5-bisphosphate (PIP2), and phosphatidylethanolamines. Positively charged phospholipids include dioleoyl trimethylammonium propane, esters of phosphatidic acids, dipalmitoylphosphatidic acid, and distearoyl-phosphatidic acid with aminoalcohols such as hydroxyethylenediamine.

In particular embodiments, conductive formulations can have conductivity values of greater than 5,000 µS/cm, greater than 10,000 µS/cm, or greater than 20,000 µS/cm. In particular embodiments, conductive formulations can have conductivity values within ranges of 4,000 - 50,000 µS/cm; 10,000 - 40,000 µS/cm; 30,000 - 60,000 µS/cm. In particular embodiments, conductive formulations have a specific conductivity that is greater than the specific conductivity of isotonic saline.

Particular embodiments of conductive formulations include salts, such as saline (0.15 M NaCl or 0.9%) solution, CaCl₂ solution, CaCl₂ in saline solution, or saline solution containing ionic surfactants, such as SDS or SLS. In particular embodiments, the formulation includes saline solution and CaCl₂. Suitable concentration ranges of the salt or other conductive/charged compounds can vary from 0.01% to 20%, 0.1% to 10% or 0.1 to 7% (weight of conductive or charged compound/total weight of formulation). In particular embodiments, the formulation contains a hypertonic saline solution (i.e. sodium chloride concentration greater than 0.9% by weight).

In particular embodiments, the formulations include a mucoactive or mucolytic agent, which is a substance that can modify mucus production, secretion, composition and/or interactions with the epithelium. Examples of mucoactive or mucolytic agents include MUCSAC and MUC5B mucins, DNA, N-acetylcysteine (NAC), cysteine, nacystelyn, dornase alfa, gelsolin, heparin, heparin sulfate. P2Y2 agonists (e.g. UTP, INS365), and nedocromil sodium.

Targeting and activating B-1a lymphocytes and/or macrophages (e.g., pleural cavity B-1a lymphtocytes and/or macrophages) through administration of formulations to the respiratory tract can treat numerous medical conditions with inflammatory or autoimmune components.

Conditions with inflammatory or autoimmune components include allergic airway disease, Alpers' disease, Alzheimer's disease (AD), amyloid nephropathy, amyloid neuropathy, amyotrophic lateral sclerosis (ALS), asthma, Batten disease, cancer, cardiac ischemia-reperfusion injury, celiac disease, cerebro-oculo-facio-skeletal syndrome, chemotherapy-associated cognitive impairment and dementia, chronic hepatitis, chronic inflammatory demyelinating polyneuropathy, chronic obstructive pulmonary disease (COPD), collagen induced arthritis, corticobasal degeneration, Creutzfeldt-Jakob disease, Crohn's disease, depression-induced dementia, Friedreich's ataxia, frontotemporal dementia, Gerstmann-Straussler-Scheinker disease, glaucoma, Graves' disease, HIV-Related cognitive impairment, Huntington's disease (HD), immune thrombocytopenia purpura, inflammatory bowel disease, insulin resistance, ischemia/reperfusion injury, juvenile arthritis, Lacunar syndromes, Lewy body disease, lupus, macular degeneration, mild cognitive impairment, monomelic amyotrophy, motor neuron diseases (MND), multiple sclerosis, multiple system atrophy, multiple system atrophy with orthostatic hypotension (Shy-Drager syndrome), myasthenia gravis, neurodegeneration with brain iron accumulation, opsoclonus myoclonus, Parkinson's disease (PD), post-encephalitic dementia, post traumatic stress disorder, syndromeposterior cortical atrophy, prion diseases, primary antiphospholipid syndrome, primary progressive aphasia, primary Sjogren's syndrome, progressive multifocal leukoencephalopathy, progressive supranuclear palsy, pseudodementia, retinal ischemia-reperfusion injury, retinitis pigmentosa, rheumatoid arthritis, spinal cord injury, spinal muscular atrophy (SMA), spinocerebellar ataxia (SCA), stroke, systemic lupus erythematosus, traumatic brain injury, type 1 diabetes, type 2 diabetes, vascular dementia, and Wernicke-Korsakoff's syndrome.

Ischemia, for example, is a restriction in blood supply that causes tissue damage in affected areas due to insufficient supply of oxygen and glucose to maintain cellular metabolism. Inadequate blood flow can be caused by vasoconstriction, artery blockage, low blood pressure, septic shock, anemia, heart failure or organ transplant. Depending on the type of tissue affected, irreversible damage may take occur within 3-5 minutes (e.g., in the brain or heart) or within 10-20 minutes in less aerobically intense organs (e.g., skin). Ischemia leads to the buildup of metabolic waste and cell leakage, and symptoms may include angina, inflammation, mottling or discoloration of skin.

After ischemia, reperfusion injury can occur when blood flow is reintroduced, where the return of oxygen can lead to overproduction of free radicals and reactive oxygen species (ROS), and causing oxidative damage to tissues, for example caused by Nox2 activity. Reperfusion can lead to cardiac arrhythmia, accelerated cell self-destruction, and exaggerated inflammation of tissue already inflamed due to ischemia as white blood cells overreact to tissue damage.

Asthma is a respiratory disease in which bronchial inflammation triggered by an allergic reaction or infection with a bacterium or virus becomes chronic to thereby cause increased airway hyperresponsiveness and reversible airway narrowing, leading to symptoms such as attacks of wheezing, cough, apnea, and chest tightness. These symptoms are more common at night or in the early morning. As the responsiveness of the airway increases, the symptoms become more severe and continuous, and daily variation in pulmonary function increases.

Chronic obstructive pulmonary disease (COPD) is a chronic lung disease that leads to breathing difficulties. It can be chronic bronchitis (a long term, productive cough) or emphysema (lung destruction), or a combination of both. It is most commonly caused by smoking, with other risk factors including inhalation of gases or fumes, exposure to secondhand smoke, or high levels of pollution. Symptoms include cough, fatigue, respiratory infections, shortness of breath, and wheezing.

Cancer (neoplasia) is characterized by deregulated cell growth and cell division. Examples of cancer with inflammatory components include acoustic neuroma, adenocarcinoma, astrocytoma, basal cell cancer, bile duct cancer, bladder cancer, brain cancer, breast cancer, bronchogenic cancer, central nervous system cancer, cervical cancer, chondrosarcoma, choriocarcinoma, chronic lymphocytic leukemia, colon cancer, craniopharyogioma, ependymoma, Ewing's tumor, fibrosarcoma, glandular cancer, glioma, hairy cell leukemia, hemangioblastoma, hepatocellular carcinoma, hepatoma, kidney cancer, leiomyosarcoma, liver cancer, liposarcoma, lung cancer, melanoma, medulloblastoma, medullary cancer, medullary thyroid cancer, menangioma, mesothelioma, myxosarcoma, neuroblastoma, non-Hodgkin's lymphoma, oligodendroglioma, osteogenic sarcoma, ovarian cancer, papillary adenocarcinomas, papillary thyroid cancer, pancreatic cancer, pheochromocytomas papillary cancer, pineal cancer, prolymphocytic leukemia, prostate cancer, renal cell cancer, retinoblastoma, rhabdomyosarcoma, sarcoma, sebaceous gland cancer, seminoma, skin cancer, squamous cell cancer, sweat gland cancer, synovioma, testicular cancer, and/or Wilms' tumor.

Effective treatments against cancer can decrease the number of cancer cells, decrease the number of metastases, decrease tumor volume, induce apoptosis of cancer cells, induce cancer cell death, induce chemo- or radio-sensitivity in cancer cells, inhibit angiogenesis near cancer cells, inhibit cancer cell proliferation, and/or inhibit tumor growth. Effective treatments against cancer can also increase life expectancy, prolong a subject's life, reduce cancer-associated pain, and/or reduce relapse or re-occurrence of the cancer following treatment. In particular embodiments effective treatments against cancer prevent, reduce, or delay the number or severity of metastatic tumors.

Rheumatoid arthritis is a chronic syndrome characterized by usually symmetric inflammation of the peripheral joints, potentially resulting in progressive destruction of articular and periarticular structures, with or without generalized manifestations. Onset of rheumatoid arthritis is usually insidious, with progressive joint involvement, but may be abrupt, with simultaneous inflammation in multiple joints. Tenderness in nearly all inflamed joints is the most sensitive physical finding. Synovial thickening, the most specific physical finding, eventually occurs in most involved joints. Symmetric involvement of small hand joints (especially proximal interphalangeal and metacarpophalangeal), foot joints (metatarsophalangeal), wrists, elbows, and ankles is typical, but initial manifestations may occur in any joint.

Multiple sclerosis (MS) is an inflammatory neurological disease characterized by various symptoms and signs of CNS dysfunction, with remissions and recurring exacerbations. The most common presenting symptoms are paresthesia in one or more extremities, in the trunk, or on one side of the face; weakness or clumsiness of a leg or hand; or visual disturbances, e.g. partial blindness and pain in one eye (retrobulbar optic neuritis), dimness of vision, or scotomas. Other common early symptoms are ocular palsy resulting in double vision (diplopia), transient weakness of one or more extremities, slight stiffness or unusual fatigability of a limb, minor gait disturbances, difficulty with bladder control, vertigo, and mild emotional disturbances; all indicate scattered CNS involvement and often occur months or years before the disease is recognized. Excess heat may accentuate symptoms and signs.

Relapsing remitting MS (RR MS) is characterized clinically by relapses and remissions that occur over months to years, with partial or full recovery of neurological deficits between attacks. Such patients manifest 1 attack, or relapse, per year. Over 10 to 20 years, 50% of RR MS patients develop secondary progressive MS (SP MS) which is characterized by incomplete recovery between attacks and accumulation of neurologic deficits resulting in increasing disability.

Diagnosis of MS is indirect, by deduction from clinical, radiographic (brain plaques on magnetic resonance [MR] scan), and to a lesser extent laboratory (oligoclonal bands on CSF analysis) features.

Huntington's disease (HD) is a neurodegenerative and inflammatory genetic disorder that affects muscle coordination and leads to cognitive decline and psychiatric problems. Clinical symptoms of HD include abnormal and/or unusual movements, anxiety, behavioral disturbances, chorea, cognitive impairment, confusion, difficulty swallowing, disorientation, fidgeting, hallucinations, head turning to shift eye position, involuntary grimaces, jerking movements of the arms, legs, face, and other body parts,
irritability, lack of coordination, personality changes, memory loss, moodiness, paranoia, psychosis, restlessness, rigidity, small unintentionally initiated or uncompleted motions, slow movements, speech changes, speech impairment, suicidal thoughts, and suicide attempts, tremor, and weight loss.

Parkinson's disease (PD) is a degenerative and inflammatory disorder of the central nervous system. Four motor symptoms are considered hallmarks of PD: tremor, rigidity, slowness of movement, and postural instability. Later in disease progression, thinking and behavioral problems may arise and can range from mild to severe, with dementia commonly occurring in the advanced stages of the disease. Depression is the most common psychiatric symptom. Other common symptoms include disorders of speech, cognition, mood, behavior, and thought. Cognitive disturbances further include executive dysfunction, which can include problems with planning, cognitive flexibility, abstract thinking, rule acquisition, initiating appropriate actions and inhibiting inappropriate actions, selecting relevant sensory information, fluctuations in attention, slowed cognitive speed, and memory loss. Other symptoms include sleep disturbances.

Amyotrophic lateral sclerosis (ALS) is a fatal motor neuron disease, affecting both the first and second order motor neurons. Early clinical symptoms of ALS are typically weakness and/or muscle atrophy. Other early symptoms include trouble swallowing (dysphagia), cramping, or stiffness of affected muscles; muscle weakness affecting an arm or a leg; and/or slurred and nasal speech or trouble forming words (dysarthria), and in some cases dementia. Twitches of muscles that can be seen under the skin (fasciculations) are observed, and an abnormal reflex commonly called Babinski's sign indicates upper motor neuron damage.

The rate of progression of ALS can be measured using an outcome measure called the "ALS Functional Rating Scale Revised (ALSFRS-R)", a 12-item instrument administered as a clinical interview or patient-reported questionnaire that produces a score between 48 (normal function) and 0 (severe disability). A survey-based study amongst clinicians showed that they rated a 20% change in the slope of the ALSFRS-R clinically meaningful (Castrillo-Viguera, et al., Amyotroph Lateral Scler, 11(1-2):178-80 (2010)).

Traumatic brain injury (TBI) occurs when an external mechanical force, typically head trauma, causes brain dysfunction. TBI can have wide-ranging physical and psychological effects. Some symptoms appear immediately while others may not appear until days or weeks after the traumatic event. Symptoms of TBI include loss of consciousness; a state of being dazed, confused or disoriented; memory or concentration problems; headache, dizziness or loss of balance; nausea or vomiting; sensory problems such as blurred vision, ringing in the ears or a bad taste in the mouth; sensitivity to light or sound; mood changes or mood swings; feeling depressed or anxious; fatigue or drowsiness; difficulty sleeping; sleeping more than usual, agitation, combativeness or other unusual behavior; slurred speech; inability to awaken from sleep; weakness or numbness in fingers and toes; loss of coordination; convulsions or seizures, dilation of one or both pupils of the eyes; and/or clear fluids draining from the nose or ears. In children, additional symptoms include change in eating or nursing habits; persistent crying and inability to be consoled; unusual or easy irritability; change in ability to pay attention; change in sleep habits; sad or depressed mood; and/or loss of interest in favorite toys or activities.

Effective treatments against conditions with an inflammatory or autoimmune component described herein can be identified by observing a statistically-significant improvement in a symptom associated with the condition in a clinical and/or research setting. For conditions where particular symptoms are not described herein, any clinically-relevant model of the condition known and accepted by clinicians and researchers in the relevant field can be used to assess the effectiveness of a treatment.

In particular embodiments, inflammatory or autoimmune conditions with a central nervous system component can be evaluated using tests for cognitive impairment, and/or neuropsychiatric morbidities, such as disorders of cognitive function, memory, mood, behavior, thought, REM Sleep Behavior Disorder, apathy, fatigue, indifference and lack of social engagement, and dullness. Methods of measuring and monitoring these aspects are known in the art and include, for example, serial position testing which focuses on human memory processes (Surprenant, Perception and Psychophysics, 63(4): 737-745 (2001)), word superiority testing which focuses on human speech and language (Krueger, Memory & Cognition, 20(6):685-694 (1992)), the Brown-Peterson test which focuses on human short-term memory (Nairne, et al., Quarterly Journal of Experimental Psychology A: Human Experimental Psychology, 52:241-251 (1999)), memory span testing (May, et al., Memory & Cognition, 27(5):759-767 (1999)), visual search testing (Wolfe, et al., Journal of Experimental Psychology: Human Perception and Performance, 15(3):419-433 (1989)), and knowledge representation (e.g., semantic network) testing. Additional tests examine processing speed, reaction time, i.e. clock speed; flexibility and ability to adapt to changes in task rules; attention, focus and concentration; problem solving; memory; and verbal fluency. Representative tests and instruments include traditional IQ tests like the WAIS and Progressive Ravens Matrices, and the battery of tests available through Luminosity (Lumos Labs, Inc.).

In particular embodiments, medical conditions with inflammatory or autoimmune components can be treated by targeting and activating B-1a lymphocytes and/or macrophages. In particular embodiments, targeting and activation of B-1a lymphocytes and/or macrophages is evidenced by an increase in IL-10 expression by the B-1a lymphocytes and/or macrophages. IL-10 is an anti-inflammatory cytokine which can inhibit the production of proinflammatory cytokines.

In particular embodiments targeting and activating B-1a lymphocytes and/or macrophages leads to suppression of proinflammatory cytokines. Examples of proinflammatory cytokines include IL-6, TNF-α, IL-2, and IFN-γ. Suppression of proinflammatory cytokines can mean a statistically significant reduction in the expression of one or more proinflammatory cytokines.

In particular embodiments targeting and activation of B-1a lymphocytes and/or macrophages is evidenced by migration of the B-1a lymphocytes and/or macrophages to lymph nodes. In particular embodiments, targeting and activation of B-1a lymphocytes and/or macrophages is evidenced by an increase in IL-10 expression and migration of the B-1a lymphocytes and/or macrophages to lymph nodes. In particular embodiments, targeting and activation of B-1a lymphocytes and/or macrophages can be evidence by decreased production of proinflammatory cytokines. In particular embodiments, targeting and activation of B-1a lymphocytes and/or macrophages is evidenced by a statistically-significant reduction in a symptom associated with a medical condition.

In particular embodiments B-1a lymphocytes and/or macrophages are targeted and activated using fibrils or fibril forming peptides. In particular embodiments, a fibril refers to an aggregation of proteins or peptides into a fibrous formation, whereby many copies of the proteins or peptides attach to one another to form insoluble fibers. In particular embodiments a fibril forming peptide can be referred to as amyloidogenic. Examples of proteins and/or peptides that naturally form fibrils include amyloid beta, tau, and amylin.

In particular embodiments, fibrils or fibril-forming peptides can include amyloid beta (Aβ) peptides. Aβ peptides include the sequences set forth in SEQ ID NO: 1063 and SEQ ID NO: 1064 (FIG. 8) and fragments and derivatives thereof. Aβ is the main component of amyloid plaques. Aβ is formed after sequential cleavage of the amyloid precursor protein (APP), a transmembrane glycoprotein of undetermined function. APP can be processed by α-, β- and γ-secretases; Aβ protein is generated by successive action of the β- and γ secretases. The γ secretase, which produces the C-terminal end of the Aβ peptide, cleaves within the transmembrane region of APP and can generate a number of isoforms of 36-43 amino acid residues in length. The most common isoforms are Aβ 1-40 and Aβ 1-42 ("Aβ40" and "Aβ42"); the shorter form is typically produced by cleavage that occurs in the endoplasmic reticulum, while the longer form is produced by cleavage in the trans-Golgi network. Aβ40 is more common of the two, but Aβ42 is more fibrillogenic.

In particular embodiments, fibrils or fibril-forming peptides can be active fragments of Aβ. Active fragments of Aβ peptide share a functional or binding property with full length Aβ peptide. In particular embodiments an active fragment of Aβ can be any fragment of the full-length Aβ that can self-polymerize to form fibrils or aggregates. In particular embodiments an active fragment of Aβ can be any length from 5 to 43 amino acids in length. Epitopic fragments of Aβ peptides are fragments that retain the ability to bind to one or more anti-Aβ monoclonal antibody. Aβ is intrinsically unstructured, meaning that in solution it does not acquire a compact tertiary fold but rather populates a set of structures. By NMR-guided simulations, Aβ40 and Aβ42 also seem to feature highly different conformational states, with the C-terminus of Aβ42 being more structured than Aβ40.

In particular embodiments, fibrils or fibril-forming peptides can be active fragments of amylin. Amylin is a peptide hormone that is secreted with insulin from the pancreas. In particular embodiments, the fibrils or fibril forming peptides can be residues 28-33 of the amylin protein, known as amylin 28-33 (SEQ ID NO: 1053). In particular embodiments, the fibrils or fibril-forming peptides can be amyloidogenic analogs of amylin 28-33. Amyloidogenic analogs of amylin 28-33 can include SEQ ID NO: 1062 and SEQ ID NO: 449 (Kurnellas, et al. (2014) JEM 211(9): 1847).

In particular embodiments, fibril formation or aggregation of a peptide or protein can be measured using techniques including the thioflavin T assay, dynamic light scattering, and size exclusion chromatography. The thioflavin T assay can be used to measure fibril formation by treating a protein/peptide sample with thioflavin T, and then measuring fluorescence by microscopy or spectroscopy.

In particular embodiments, fibril refers to a fibrous aggregate of four or more peptide molecules linked through non-covalent bonds. In particular embodiments, the aggregates include one hundred or more, one thousand or more, five thousand or more, or ten thousand or more peptide molecules. In particular embodiments a fibril-forming or amyloidogenic peptide is a peptide that, either spontaneously or upon exposure to certain conditions such as temperature or salinity, oligomerizes through non-covalent interactions to form fibrils.

In particular embodiments, fibril-forming peptides include hexapeptides with amino acids of L-configuration, D-configuration or a mixture of configurations. In particular embodiments, fibril-forming peptides are peptides that generate a Rosetta energy of binding of -23 kcal/mol or less (i.e., more negative). Rosetta is a software suite that is used for computational modeling of proteins and peptides. The cut-off value of -23 kcal/mol for fibril-forming potential is based on an experimentally validated model (see Goldschmidt et al (2010) PNAS 107(5): 3487-3492, for methods of Rosetta energy calculation for the hexapeptides).

In particular embodiments the peptides include 1, 2, or 3 amino acids having polar basic side chains. The polar basic side chains may have a terminal amino or a terminal imidazole group. In other cases, the peptides include 1 polar acidic side chain. In other cases, the peptides include 0, 1, 2, 3, 4, or 5 amino acids having hydrophobic side chains. In still other cases, the peptides include 0, 1, 2, 3, 4, or 5 amino acids having polar uncharged side chains, wherein the peptide has a positive charge. In particular embodiments, the fibrils or fibril-forming peptides do not contain proline.

**The** carboxy terminus of the peptides is typically either a carboxylic acid (i.e., --COOH) or an amide (i.e., --C(O)NR₂, where R is a substituent such as alkyl or hydrogen). The amino terminus is typically either an amine (i.e., N(R')₂, where R' is a substituent such as alkyl or hydrogen) or an acetate group (i.e., --C(O)R", where R" is a substituent such as methyl, ethyl, or longer alkyl).

In particular embodiments, fibril-forming peptides may include one or more of the following hexapeptides, where each indicated amino acid is either an L-amino acid or a D-amino acid (symbol for hexapeptide indicated as its SEQ ID NO: identifier in parenthesis after the hexamer): SVNVDL (SEQ ID NO: 1); SLNVDV (SEQ ID NO: 2); SVDVNL (SEQ ID NO: 3); DLSVVL (SEQ ID NO: 4); SVNLDV (SEQ ID NO: 5); SVVNDV (SEQ ID NO: 6); DVSLVN (SEQ ID NO: 7); DVSVLN (SEQ ID NO: 8); SDLVNV (SEQ ID NO: 9); SLNVVS (SEQ ID NO: 10); LNVDSV (SEQ ID NO: 11); NDLSVV (SEQ ID NO: 12); VDNLVS (SEQ ID NO: 13); VNDVSL (SEQ ID NO: 14); VSDNVL (SEQ ID NO: 15); LNDVVS (SEQ ID NO: 16); LSVDVN (SEQ ID NO: 17); NSVDLV (SEQ ID NO: 18); VDVLNS (SEQ ID NO: 19); VNDSVL (SEQ ID NO: 20); VNVSLD (SEQ ID NO: 21); LDVNSV (SEQ ID NO: 22); LNVVDS (SEQ ID NO: 23); LSDVVN (SEQ ID NO: 24); LVVNDS (SEQ ID NO: 25); NSLDVV (SEQ ID NO: 26); VLVDNS (SEQ ID NO: 27); VNLSDV (SEQ ID NO: 28); VNVDLS (SEQ ID NO: 29); DNVSVD (SEQ ID NO: 30); LSDNVV (SEQ ID NO: 31); LSVVDN (SEQ ID NO: 32); VLDVSN (SEQ ID NO: 33); VNDLVS (SEQ ID NO: 34); VNVSDL (SEQ ID NO: 35); VVLSDN (SEQ ID NO: 36); LVSVNL (SEQ ID NO: 37); LVNVSV (SEQ ID NO: 38); SLNVSV (SEQ ID NO: 39); LVSVNS (SEQ ID NO: 40); SVDVNV (SEQ ID NO: 41); LVVSVL (SEQ ID NO: 42); VNLVVS (SEQ ID NO: 43); SNLVSV (SEQ ID NO: 44); SVNVLS (SEQ ID NO: 45); VLVSVL (SEQ ID NO: 46); LVNVSL (SEQ ID NO: 47); SVNVDS (SEQ ID NO: 48); VLSVNV (SEQ ID NO: 49); NLVVSV (SEQ ID NO: 50); SVVLNV (SEQ ID NO: 51); LSVVNL (SEQ ID NO: 52); SNLVVS (SEQ ID NO: 53); SVVLDV (SEQ ID NO: 54); LVSLNV (SEQ ID NO: 55); VSLNVV (SEQ ID NO: 56); VKVQIY (SEQ ID NO: 57); QVVIYK (SEQ ID NO: 58); KVIQVY (SEQ ID NO: 59); VYVKIY (SEQ ID NO: 60); QIVVYK (SEQ ID NO: 61); QVIKVY (SEQ ID NO: 62); VQVKIY (SEQ ID NO: 63); QVVKIY (SEQ ID NO: 64); QVIVYK (SEQ ID NO: 65); QIVKVY (SEQ ID NO: 66); QKIVVY (SEQ ID NO: 67); QKVVYI (SEQ ID NO: 68); KVQVYI (SEQ ID NO: 69); QVVKYI (SEQ ID NO: 70); KVQIYV (SEQ ID NO: 71); VKIQVY (SEQ ID NO: 72); VIQKVY (SEQ ID NO: 73); KVVIYK (SEQ ID NO: 74); QIVKYV (SEQ ID NO: 75); QKVIYV (SEQ ID NO: 76); KQVVIY (SEQ ID NO: 77); KIQVYV (SEQ ID NO: 78); KVYVQI (SEQ ID NO: 79); VVQIYK (SEQ ID NO: 80); KQVIVY (SEQ ID NO: 81); VQIKVY (SEQ ID NO: 82); QKIVYV (SEQ ID NO: 83); VIQVYK (SEQ ID NO: 84); KVVIQY (SEQ ID NO: 85); KVQVIY (SEQ ID NO: 86); QYVVIK (SEQ ID NO: 87); YVVIQK (SEQ ID NO: 88); KIVVQY (SEQ ID NO: 89); YQVIVK (SEQ ID NO: 90); KIYVQV (SEQ ID NO: 91); KVVIYQ (SEQ ID NO: 92); KYVVQI (SEQ ID NO: 93); YQIVVK (SEQ ID NO: 94); YVVIQY (SEQ ID NO: 95); KQVIYV (SEQ ID NO: 96); KYVQVI (SEQ ID NO: 97); KYVVIQ (SEQ ID NO: 98); QKVVIY (SEQ ID NO: 99); QYVIVK (SEQ ID NO: 100); IVQKVY (SEQ ID NO: 101); KYIVVQ (SEQ ID NO: 102); QVIKYV (SEQ ID NO: 103); YIVVQK (SEQ ID NO: 104); YQVVIK (SEQ ID NO: 105); IKVQVY (SEQ ID NO: 106); KVVQYI (SEQ ID NO: 107); KYVQIV (SEQ ID NO: 108); VKQVYI (SEQ ID NO: 109); VQVIYK (SEQ ID NO: 110); VVIQKY (SEQ ID NO: 111); IQVVYK (SEQ ID NO: 112); KIVVYQ (SEQ ID NO: 113); KYQVIV (SEQ ID NO: 114); KYVIVQ (SEQ ID NO: 115); VVYIQK (SEQ ID NO: 116); IVQVYK (SEQ ID NO: 117); KQIVYV (SEQ ID NO: 118); KYVIQV (SEQ ID NO: 119); QKVIVY (SEQ ID NO: 120); QYIKVV (SEQ ID NO: 121); QYIVKV (SEQ ID NO: 122); VQIVYK (SEQ ID NO: 123); VVQKYI (SEQ ID NO: 124); VYQIVK (SEQ ID NO: 125); YVQVIK (SEQ ID NO: 126); QIVYVK (SEQ ID NO: 127); IKVYQV (SEQ ID NO: 128); KVVYIQ (SEQ ID NO: 129); VQKYIV (SEQ ID NO: 130); VQKYVI (SEQ ID NO: 131); KVVQIY (SEQ ID NO: 132); YVIKIY (SEQ ID NO: 133); QVIVYV (SEQ ID NO: 134); VVIKVY (SEQ ID NO: 135); KVVIYI (SEQ ID NO: 136); VYVVIK (SEQ ID NO: 137); YIVQVY (SEQ ID NO: 138); YVIQVY (SEQ ID NO: 139); YVIVVY (SEQ ID NO: 140); KVIVYI (SEQ ID NO: 141); YVIKVY (SEQ ID NO: 142); VQVIVK (SEQ ID NO: 143); YVVKIY (SEQ ID NO: 144); YVVQVI (SEQ ID NO: 145); QIVVYQ (SEQ ID NO: 146); VVQIVK (SEQ ID NO: 147); VQIVVK (SEQ ID NO: 148); VIVVYK (SEQ ID NO: 149); IVQVYI (SEQ ID NO: 150); YVVIQV (SEQ ID NO: 151); VYQVVI (SEQ ID NO: 152); KYIQVY (SEQ ID NO: 153); KYVVIY (SEQ ID NO: 154); KYVQIY (SEQ ID NO: 155); VVQVIK (SEQ ID NO: 156); QKIVVK (SEQ ID NO: 157); KYVIVY (SEQ ID NO: 158); KQVVIK (SEQ ID NO: 159); YVQIYV (SEQ ID NO: 160); YKVQVY (SEQ ID NO: 161); IKVQIY (SEQ ID NO: 162); KIVVQK (SEQ ID NO: 163); VIKVVI (SEQ ID NO: 164); VYIKVV (SEQ ID NO: 165); VYIVQV (SEQ ID NO: 166); IVYVQI (SEQ ID NO: 167); KVQIYK (SEQ ID NO: 168); IYVIVY (SEQ ID NO: 169); VIYVIV (SEQ ID NO: 170); KYQVYI (SEQ ID NO: 171); VIQKVV (SEQ ID NO: 172); VKIVYV (SEQ ID NO: 173); VKQIVV (SEQ ID NO: 174); YIVKQY (SEQ ID NO: 175); YIVVQY (SEQ ID NO: 176); YKIQVY (SEQ ID NO: 177); YQVVIY (SEQ ID NO: 178); YVVKQY (SEQ ID NO: 179); VIVKVQ (SEQ ID NO: 180); QKVVIK (SEQ ID NO: 181); GMVVVG (SEQ ID NO: 182); GVVVMG (SEQ ID NO: 183); GVVMVG (SEQ ID NO: 184); GGVVVM (SEQ ID NO: 185); GVVGVM (SEQ ID NO: 186); GVVVGM (SEQ ID NO: 187); GVMVVG (SEQ ID NO: 188); GGVVMV (SEQ ID NO: 189); GGVMVV (SEQ ID NO: 190); GMVVGV (SEQ ID NO: 191); GVVMGV (SEQ ID NO: 192); GMVGVV (SEQ ID NO: 193); GVMVGV (SEQ ID NO: 194); GVVGMV (SEQ ID NO: 195); GGMVVV (SEQ ID NO: 196); GVGVMV (SEQ ID NO: 197); GVGVVM (SEQ ID NO: 198); MVVVGM (SEQ ID NO: 199); GMGVVV (SEQ ID NO: 200); MVVVGG (SEQ ID NO: 201); GVGMVV (SEQ ID NO: 202); GVVGGV (SEQ ID NO: 203); MVGVVG (SEQ ID NO: 204); VVMVGG (SEQ ID NO: 205); MGVVVG (SEQ ID NO: 206); MVGVGV (SEQ ID NO: 207); VGVMVG (SEQ ID NO: 208); MVVGVG (SEQ ID NO: 209); VVGVMG (SEQ ID NO: 210); VMGVVG (SEQ ID NO: 211); VGVGVM (SEQ ID NO: 212); VMVGVG (SEQ ID NO: 213); MGGVVV (SEQ ID NO: 214); VGMVVG (SEQ ID NO: 215); VVGVGM (SEQ ID NO: 216); VGGVVM (SEQ ID NO: 217); VVGMVG (SEQ ID NO: 218); VGVVMG (SEQ ID NO: 219); MVGGVV (SEQ ID NO: 220); VGMVGV (SEQ ID NO: 221); VMVVGG (SEQ ID NO: 222); VVGGVM (SEQ ID NO: 223); VGGVMV (SEQ ID NO: 224); VVVGMG (SEQ ID NO: 225); VVGMGV (SEQ ID NO: 226); MGVGVV (SEQ ID NO: 227); VGVVGM (SEQ ID NO: 228); VGGMVV (SEQ ID NO: 229); VVVMGG (SEQ ID NO: 230); VVMGGV (SEQ ID NO: 231); VVVGGM (SEQ ID NO: 232); VVGGMV (SEQ ID NO: 233); VGMGVV (SEQ ID NO: 234); VMGGVV (SEQ ID NO: 235); VGGVGV (SEQ ID NO: 236); VGVMGV (SEQ ID NO: 237); VGVGMV (SEQ ID NO: 238); and VMVGGV (SEQ ID NO: 239); ANSTSV (SEQ ID NO: 240); ANSVSG (SEQ ID NO: 241); ANSVSS (SEQ ID NO: 242); AQNSNV (SEQ ID NO: 243); AQNVNS (SEQ ID NO: 244); AQNVTS (SEQ ID NO: 245); AQSQSV (SEQ ID NO: 246); AQSSSV (SEQ ID NO: 247); AQSTSV (SEQ ID NO: 248); AQSVNS (SEQ ID NO: 249); AQSVQS (SEQ ID NO: 250); AQSVSQ (SEQ ID NO: 251); AQSVSS (SEQ ID NO: 252); AQSVST (SEQ ID NO: 253); ASNNNV (SEQ ID NO: 254); ASNQNQ (SEQ ID NO: 255); ASNQNV (SEQ ID NO: 256); ASNQTQ (SEQ ID NO: 257); ASNSNV (SEQ ID NO: 258); ASNSTV (SEQ ID NO: 259); ASNTNS (SEQ ID NO: 260); ASNTNV (SEQ ID NO: 261); ASNTSV (SEQ ID NO: 262); ASNVNG (SEQ ID NO: 263); ASNVNQ (SEQ ID NO: 264); ASNVNS (SEQ ID NO: 265); ASNVNT (SEQ ID NO: 266); ASNVTG (SEQ ID NO: 267); ASNVTT (SEQ ID NO: 268); ASSNSV (SEQ ID NO: 269); ASSVSG (SEQ ID NO: 270); ASSVSN (SEQ ID NO: 271); ATNVNS (SEQ ID NO: 272); ATNVTS (SEQ ID NO: 273); ATSQSQ (SEQ ID NO: 274); ATSQSV (SEQ ID NO: 275); ATSTSG (SEQ ID NO: 276); ATSTSV (SEQ ID NO: 277); ATSVSG (SEQ ID NO: 278); ATSVSS (SEQ ID NO: 279); AVNQNS (SEQ ID NO: 280); AVNQSQ (SEQ ID NO: 281); AVNSNG (SEQ ID NO: 282); AVNSNS (SEQ ID NO: 283); AVNSNT (SEQ ID NO: 284); AVNTNS (SEQ ID NO: 285); AVSNSG (SEQ ID NO: 286); AVSNSS (SEQ ID NO: 287); AVSQNQ (SEQ ID NO: 288); AVSQSG (SEQ ID NO: 289); AVSQSQ (SEQ ID NO: 290); AVSQTQ (SEQ ID NO: 291); AVSSNQ (SEQ ID NO: 292); AVSSNS (SEQ ID NO: 293); AVSSSQ (SEQ ID NO: 294); AVSTSG (SEQ ID NO: 295); GANTVS (SEQ ID NO: 296); GAQTSS (SEQ ID NO: 297); GASNQS (SEQ ID NO: 298); GASQQS (SEQ ID NO: 299); GASSQQ (SEQ ID NO: 300) GGQVTS (SEQ ID NO: 301); GGSNQV (SEQ ID NO: 302); GNNVQS (SEQ ID NO: 303); GNQVTS (SEQ ID NO: 304); GNSNQV (SEQ ID NO: 305); GNSQQQ (SEQ ID NO: 306); GNSQQS (SEQ ID NO: 307); GNSQQV (SEQ ID NO: 308); GNSSTV (SEQ ID NO: 309); GNSTQS (SEQ ID NO: 310); GNSTQV (SEQ ID NO: 311); GNSTVS (SEQ ID NO: 312); GNSVQS (SEQ ID NO: 313); GNSVSS (SEQ ID NO: 314); GNSVST (SEQ ID NO: 315); GNSVTS (SEQ ID NO: 316); GQNTVS (SEQ ID NO: 317); GQNVAS (SEQ ID NO: 318); GQNVQS (SEQ ID NO: 319); GQNVSS (SEQ ID NO: 320); GQNVTS (SEQ ID NO: 321); GQQQSQ (SEQ ID NO: 322); GQQTSS (SEQ ID NO: 323); GQQTSV (SEQ ID NO: 324); GQQVAS (SEQ ID NO: 325); GQQVNS (SEQ ID NO: 326); GQQVQS (SEQ ID NO: 327); GQQVSG (SEQ ID NO: 328); GQQVSQ (SEQ ID NO: 329); GQQVSS (SEQ ID NO: 330); GQQVST (SEQ ID NO: 331); GQQVTS (SEQ ID NO: 332); GQSGQV (SEQ ID NO: 333); GQSNQA (SEQ ID NO: 334); GQSNQS (SEQ ID NO: 335); GQSNQV (SEQ ID NO: 336); GQSQAQ (SEQ ID NO: 337); GQSQQQ (SEQ ID NO: 338); GQSQQS (SEQ ID NO: 339); GQSQSQ (SEQ ID NO: 340); GQSSQQ (SEQ ID NO: 341); GQSSQS (SEQ ID NO: 342); GQSSQV (SEQ ID NO: 343); GQSTQS (SEQ ID NO: 344); GQSTQV (SEQ ID NO: 345); GQSVAG (SEQ ID NO: 346); GQSVAQ (SEQ ID NO: 347); GQSVAS (SEQ ID NO: 348); GQSVQN (SEQ ID NO: 349); GQSVQQ (SEQ ID NO: 350); GQSVQS (SEQ ID NO: 351); GQSVSG (SEQ ID NO: 352); GQSVSN (SEQ ID NO: 353); GQSVSQ (SEQ ID NO: 354); GQSVSS (SEQ ID NO: 355); GQSVST (SEQ ID NO: 356); GQSVTS (SEQ ID NO: 357); GSNQQV (SEQ ID NO: 358); GSNQVQ (SEQ ID NO: 359); GSNSTV (SEQ ID NO: 360); GSNSVQ (SEQ ID NO: 361); GSNSVT (SEQ ID NO: 362); GSNTAV (SEQ ID NO: 363); GSNTQV (SEQ ID NO: 364); GSNTVA (SEQ ID NO: 365); GSNTVS (SEQ ID NO: 366); GSNVAS (SEQ ID NO: 367); GSNVQQ (SEQ ID NO: 368); GSNVQS (SEQ ID NO: 369); GSNVQT (SEQ ID NO: 370); GSNVTS (SEQ ID NO: 371); GSQQSV (SEQ ID NO: 372); GSQQTQ (SEQ ID NO: 373); GSQQTV (SEQ ID NO: 374); GSQTSS (SEQ ID NO: 375); GSQTSV (SEQ ID NO: 376); GSQVAS (SEQ ID NO: 377); GSQVNS (SEQ ID NO: 378); GSQVQS (SEQ ID NO: 379); GSQVSS (SEQ ID NO: 380); GSQVST (SEQ ID NO: 381); GSQVTG (SEQ ID NO: 382); GSQVTS (SEQ ID NO: 383); GSSAQS (SEQ ID NO: 384); GSSGQV (SEQ ID NO: 385); GSSNQA (SEQ ID NO: 386); GSSNQT (SEQ ID NO: 387); GSSNQV (SEQ ID NO: 388); GSSNSV (SEQ ID NO: 389); GSSNTV (SEQ ID NO: 390); GSSNVT (SEQ ID NO: 391); GSSQAQ (SEQ ID NO: 392); GSSQQA (SEQ ID NO: 393); GSSQQV (SEQ ID NO: 394); GSSQSQ (SEQ ID NO: 395); GSSSQQ (SEQ ID NO: 396); GSSSQS (SEQ ID NO: 397); GSSSQV (SEQ ID NO: 398); GSSTQA (SEQ ID NO: 399); GSSTQG (SEQ ID NO: 400); GSSTQT (SEQ ID NO: 401); GSSTQV (SEQ ID NO: 402); GSSTSV (SEQ ID NO: 403); GSSTVN (SEQ ID NO: 404); GSSVNS (SEQ ID NO: 405); GSSVQN (SEQ ID NO: 406); GSSVQT (SEQ ID NO: 407); GSSVSG (SEQ ID NO: 408); GSSVSS (SEQ ID NO: 409); GSSVST (SEQ ID NO: 410); GSSVTN (SEQ ID NO: 411); GTNSSV (SEQ ID NO: 412); GTNSVS (SEQ ID NO: 413); GTNTVS (SEQ ID NO: 414); GTNVQS (SEQ ID NO: 415); GTNVSS (SEQ ID NO: 416); GTNVTS (SEQ ID NO: 417); GTQQSQ (SEQ ID NO: 418); GTQSTS (SEQ ID NO: 419); GTQSTV (SEQ ID NO: 420); GTQTSA (SEQ ID NO: 421); GTQTSV (SEQ ID NO: 422); GTQVQS (SEQ ID NO: 423); GTQVSG (SEQ ID NO: 424); GTQVSN (SEQ ID NO: 425); GTQVSS (SEQ ID NO: 426); GTQVST (SEQ ID NO: 427); GTSAQS (SEQ ID NO: 428); GTSGQV (SEQ ID NO: 429); GTSNQA (SEQ ID NO: 430); GTSNQS (SEQ ID NO: 431); GTSNQT (SEQ ID NO: 432); GTSNQV (SEQ ID NO: 433); GTSNSV (SEQ ID NO: 434); GTSNVS (SEQ ID NO: 435); GTSQQA (SEQ ID NO: 436); GTSQQS (SEQ ID NO: 437); GTSQQV (SEQ ID NO: 438); GTSQSQ (SEQ ID NO: 439); GTSQSV (SEQ ID NO: 440); GTSSNV (SEQ ID NO: 441); GTSTQA (SEQ ID NO: 442); GTSTQS (SEQ ID NO: 443); GTSTQT (SEQ ID NO: 444); GTSTQV (SEQ ID NO: 445); GTSTSV (SEQ ID NO: 446); GTSVAG (SEQ ID NO: 447); GTSVAS (SEQ ID NO: 448); GTSVNS (SEQ ID NO: 449); GTSVNT (SEQ ID NO: 450); GTSVQG (SEQ ID NO: 451); GTSVQN (SEQ ID NO: 452); GTSVQQ (SEQ ID NO: 453); GTSVQS (SEQ ID NO: 454); GTSVQT (SEQ ID NO: 455); GTSVSG (SEQ ID NO: 456); GTSVSN (SEQ ID NO: 457); GTSVSS (SEQ ID NO: 458); GTSVST (SEQ ID NO: 459); GTSVTS (SEQ ID NO: 460); GVNSQS (SEQ ID NO: 461); GVNSST (SEQ ID NO: 462); GVNSTS (SEQ ID NO: 463); GVNTQS (SEQ ID NO: 464); GVNTSS (SEQ ID NO: 465); GVQNTS (SEQ ID NO: 466); GVQQSQ (SEQ ID NO: 467); GVQSNQ (SEQ ID NO: 468); GVQSNS (SEQ ID NO: 469); GVQSSG (SEQ ID NO: 470); GVQSSQ (SEQ ID NO: 471); GVQSTS (SEQ ID NO: 472); GVQSTT (SEQ ID NO: 473); GVQTNS (SEQ ID NO: 474); GVQTQS (SEQ ID NO: 475); GVQTSG (SEQ ID NO: 476); GVQTSS (SEQ ID NO: 477); GVSGQG (SEQ ID NO: 478); GVSGQT (SEQ ID NO: 479); GVSNAS (SEQ ID NO: 480); GVSNNV (SEQ ID NO: 481); GVSNQG (SEQ ID NO: 482); GVSNQN (SEQ ID NO: 483); GVSNQQ (SEQ ID NO: 484); GVSNQS (SEQ ID NO: 485); GVSNQT (SEQ ID NO: 486); GVSNSS (SEQ ID NO: 487); GVSNST (SEQ ID NO: 488); GVSNTS (SEQ ID NO: 489); GVSQAQ (SEQ ID NO: 490); GVSQNQ (SEQ ID NO: 491); GVSQNS (SEQ ID NO: 492); GVSQQG (SEQ ID NO: 493); GVSQQS (SEQ ID NO: 494); GVSQQT (SEQ ID NO: 495); GVSQSG (SEQ ID NO: 496); GVSQSQ (SEQ ID NO: 497); GVSQSS (SEQ ID NO: 498); GVSSAQ (SEQ ID NO: 499); GVSSAS (SEQ ID NO: 500); GVSSGQ (SEQ ID NO: 501); GVSSNG (SEQ ID NO: 502); GVSSNQ (SEQ ID NO: 503); GVSSNS (SEQ ID NO: 504); GVSSNT (SEQ ID NO: 505); GVSSQG (SEQ ID NO: 506); GVSSQN (SEQ ID NO: 507); GVSSQQ (SEQ ID NO: 508); GVSSSG (SEQ ID NO: 509); GVSSSQ (SEQ ID NO: 510); GVSSSS (SEQ ID NO: 511); GVSSTN (SEQ ID NO: 512); GVSTAS (SEQ ID NO: 513); GVSTNS (SEQ ID NO: 514); GVSTQG (SEQ ID NO: 515); GVSTQN (SEQ ID NO: 516); GVSTQQ (SEQ ID NO: 517); GVSTQS (SEQ ID NO: 518); GVSTQT (SEQ ID NO: 519); GVSTSN (SEQ ID NO: 520); GVSTSQ (SEQ ID NO: 521); GVSTSS (SEQ ID NO: 522); GVSTST (SEQ ID NO: 523); GVTNSS (SEQ ID NO: 524); GVTSNS (SEQ ID NO: 525); GVTSSN (SEQ ID NO: 526); NGSTSV (SEQ ID NO: 527); NGSVTS (SEQ ID NO: 528); NNSVSS (SEQ ID NO: 529); NQSQSQ (SEQ ID NO: 530); NQSVSN (SEQ ID NO: 531); NQSVSQ (SEQ ID NO: 532); NQSVSS (SEQ ID NO: 533); NSSNSV (SEQ ID NO: 534); NSSQSQ (SEQ ID NO: 535); NSSTVG (SEQ ID NO: 536); NSSVSG (SEQ ID NO: 537); NSSVSN (SEQ ID NO: 538); NSSVSS (SEQ ID NO: 539); NSSVTG (SEQ ID NO: 540); NTNVNS (SEQ ID NO: 541); NTQVSS (SEQ ID NO: 542); NTSGSV (SEQ ID NO: 543); NTSQSQ (SEQ ID NO: 544); NTSTSS (SEQ ID NO: 545); NTSTSV (SEQ ID NO: 546); NTSVSS (SEQ ID NO: 547); NVSGST (SEQ ID NO: 548); NVSNSG (SEQ ID NO: 549); NVSQSG (SEQ ID NO: 550); NVSQSQ (SEQ ID NO: 551); NVSQSS (SEQ ID NO: 552); NVSSSG (SEQ ID NO: 553); NVSSSS (SEQ ID NO: 554); NVSSTG (SEQ ID NO: 555); NVSTSN (SEQ ID NO: 556); NVSTSS (SEQ ID NO: 557); NVSTST (SEQ ID NO: 558); QNSTSV (SEQ ID NO: 559); QNSVSG (SEQ ID NO: 560); QQSQSQ (SEQ ID NO: 561); QQSVAG (SEQ ID NO: 562); QQSVAQ (SEQ ID NO: 563); QQSVAS (SEQ ID NO: 564); QQSVAT (SEQ ID NO: 565); QQSVSG (SEQ ID NO: 566); QQSVSN (SEQ ID NO: 567); QQSVSS (SEQ ID NO: 568); QSNQVQ (SEQ ID NO: 569); QSNTAV (SEQ ID NO: 570); QSNTNV (SEQ ID NO: 571); QSSNSV (SEQ ID NO: 572); QSSQAQ (SEQ ID NO: 573); QSSQSQ (SEQ ID NO: 574); QSSQSV (SEQ ID NO: 575); QSSTSV (SEQ ID NO: 576); QSSVSG (SEQ ID NO: 577); QSSVSN (SEQ ID NO: 578); QSSVSQ (SEQ ID NO: 579); QSSVSS (SEQ ID NO: 580); QSSVTG (SEQ ID NO: 581); QSSVTS (SEQ ID NO: 582); QTSQSQ (SEQ ID NO: 583); QTSSSQ (SEQ ID NO: 584); QTSSSV (SEQ ID NO: 585); QTSVAG (SEQ ID NO: 586); QTSVAS (SEQ ID NO: 587); QTSVSG (SEQ ID NO: 588); QTSVSN (SEQ ID NO: 589); QTSVSS (SEQ ID NO: 590); QTSVST (SEQ ID NO: 591); QTSVTS (SEQ ID NO: 592); QVSNSG (SEQ ID NO: 593); QVSNSS (SEQ ID NO: 594); QVSNST (SEQ ID NO: 595); QVSQAQ (SEQ ID NO: 596); QVSQTG (SEQ ID NO: 597); QVSQTQ (SEQ ID NO: 598); QVSSAQ (SEQ ID NO: 599); QVSSSG (SEQ ID NO: 600); QVSSSQ (SEQ ID NO: 601); QVSSSS (SEQ ID NO: 602); QVSSTQ (SEQ ID NO: 603); QVSTSG (SEQ ID NO: 604); QVSTSN (SEQ ID NO: 605); QVSTSS (SEQ ID NO: 606); QVSTST (SEQ ID NO: 607); SAQQSQ (SEQ ID NO: 608); SAQQTQ (SEQ ID NO: 609); SASQQQ (SEQ ID NO: 610); SASQSQ (SEQ ID NO: 611); SGNSTV (SEQ ID NO: 612); SGNTSV (SEQ ID NO: 613); SGNVST (SEQ ID NO: 614); SGNVTS (SEQ ID NO: 615); SGQQTQ (SEQ ID NO: 616); SGQQTV (SEQ ID NO: 617); SGQTSV (SEQ ID NO: 618); SGQVSS (SEQ ID NO: 619); SGQVTG (SEQ ID NO: 620); SGQVTQ (SEQ ID NO: 621); SGQVTT (SEQ ID NO: 622); SGSNTV (SEQ ID NO: 623); SGSTNV (SEQ ID NO: 624); SGSTQV (SEQ ID NO: 625); SGSVAS (SEQ ID NO: 626); SGSVNT (SEQ ID NO: 627); SGSVQG (SEQ ID NO: 628); SGSVQS (SEQ ID NO: 629); SNNQTV (SEQ ID NO: 630); SNNTAV (SEQ ID NO: 631); SNNTNV (SEQ ID NO: 632); SNNTQV (SEQ ID NO: 633); SNQQSQ (SEQ ID NO: 634); SNQQSV (SEQ ID NO: 635); SNQQTQ (SEQ ID NO: 636); SNQQTV (SEQ ID NO: 637); SNQSTV (SEQ ID NO: 638); SNQTSV (SEQ ID NO: 639); SNQVSG (SEQ ID NO: 640); SNQVSQ (SEQ ID NO: 641); SNQVSS (SEQ ID NO: 642); SNQVST (SEQ ID NO: 643); SNQVTG (SEQ ID NO: 644); SNQVTQ (SEQ ID NO: 645); SNQVTS (SEQ ID NO: 646); SNSGAV (SEQ ID NO: 647); SNSNAV (SEQ ID NO: 648); SNSNGV (SEQ ID NO: 649); SNSNQS (SEQ ID NO: 650); SNSQAG (SEQ ID NO: 651); SNSQAQ (SEQ ID NO: 652); SNSQAS (SEQ ID NO: 653); SNSQAT (SEQ ID NO: 654); SNSQAV (SEQ ID NO: 655); SNSQGQ (SEQ ID NO: 656); SNSQGV (SEQ ID NO: 657); SNSQQA (SEQ ID NO: 658); SNSQQG (SEQ ID NO: 659); SNSQQQ (SEQ ID NO: 660); SNSQQS (SEQ ID NO: 661); SNSQQT (SEQ ID NO: 662); SNSQQV (SEQ ID NO: 663); SNSQSG (SEQ ID NO: 664); SNSQST (SEQ ID NO: 665); SNSQSV (SEQ ID NO: 666); SNSSAV (SEQ ID NO: 667); SNSSGV (SEQ ID NO: 668); SNSSQG (SEQ ID NO: 669); SNSSQQ (SEQ ID NO: 670); SNSSQT (SEQ ID NO: 671); SNSSQV (SEQ ID NO: 672); SNSSSQ (SEQ ID NO: 673); SNSSSV (SEQ ID NO: 674); SNSTAG (SEQ ID NO: 675); SNSTGS (SEQ ID NO: 676); SNSTNV (SEQ ID NO: 677); SNSTQA (SEQ ID NO: 678); SNSTQQ (SEQ ID NO: 679); SNSTQS (SEQ ID NO: 680); SNSTQV (SEQ ID NO: 681); SNSTSA (SEQ ID NO: 682); SNSTSV (SEQ ID NO: 683); SNSTVG (SEQ ID NO: 684); SNSVAQ (SEQ ID NO: 685); SNSVAS (SEQ ID NO: 686); SNSVAT (SEQ ID NO: 687); SNSVGQ (SEQ ID NO: 688); SNSVGS (SEQ ID NO: 689); SNSVGT (SEQ ID NO: 690); SNSVNS (SEQ ID NO: 691); SNSVNT (SEQ ID NO: 692); SNSVQQ (SEQ ID NO: 693); SNSVQS (SEQ ID NO: 694); SNSVSG (SEQ ID NO: 695); SNSVSQ (SEQ ID NO: 696); SNSVSS (SEQ ID NO: 697); SNSVST (SEQ ID NO: 698); SNSVTG (SEQ ID NO: 699); SQNVAS (SEQ ID NO: 700); SQNVNS (SEQ ID NO: 701); SQQQSQ (SEQ ID NO: 702); SQQQSV (SEQ ID NO: 703); SQQQTQ (SEQ ID NO: 704); SQQQTV (SEQ ID NO: 705); SQQSTV (SEQ ID NO: 706); SQQVSG (SEQ ID NO: 707); SQQVSN (SEQ ID NO: 708); SQQVSS (SEQ ID NO: 709); SQQVST (SEQ ID NO: 710); SQQVTG (SEQ ID NO: 711); SQQVTN (SEQ ID NO: 712); SQQVTS (SEQ ID NO: 713); SQQVTT (SEQ ID NO: 714); SQSNAS (SEQ ID NO: 715); SQSNAV (SEQ ID NO: 716); SQSNGV (SEQ ID NO: 717); SQSNQT (SEQ ID NO: 718); SQSNQV (SEQ ID NO: 719); SQSNSV (SEQ ID NO: 720); SQSQAQ (SEQ ID NO: 721); SQSQAS (SEQ ID NO: 722); SQSQAV (SEQ ID NO: 723); SQSQQQ (SEQ ID NO: 724); SQSQQV (SEQ ID NO: 725); SQSQSQ (SEQ ID NO: 726); SQSSAV (SEQ ID NO: 727); SQSSGV (SEQ ID NO: 728); SQSSQG (SEQ ID NO: 729); SQSSQV (SEQ ID NO: 730); SQSSSQ (SEQ ID NO: 731); SQSSSV (SEQ ID NO: 732); SQSTAV (SEQ ID NO: 733); SQSTQS (SEQ ID NO: 734); SQSTSG (SEQ ID NO: 735); SQSTSV (SEQ ID NO: 736); SQSVAG (SEQ ID NO: 737); SQSVAN (SEQ ID NO: 738); SQSVAQ (SEQ ID NO: 739); SQSVAS (SEQ ID NO: 740); SQSVAT (SEQ ID NO: 741); SQSVGG (SEQ ID NO: 742); SQSVGN (SEQ ID NO: 743); SQSVGQ (SEQ ID NO: 744); SQSVGS (SEQ ID NO: 745); SQSVGT (SEQ ID NO: 746); SQSVNG (SEQ ID NO: 747); SQSVNS (SEQ ID NO: 748); SQSVQG (SEQ ID NO: 749); SQSVQN (SEQ ID NO: 750); SQSVQQ (SEQ ID NO: 751); SQSVQS (SEQ ID NO: 752); SQSVQT (SEQ ID NO: 753); SQSVSG (SEQ ID NO: 754); SQSVSN (SEQ ID NO: 755); SQSVSS (SEQ ID NO: 756); SQSVST (SEQ ID NO: 757); SSNGTV (SEQ ID NO: 758); SSNQNQ (SEQ ID NO: 759); SSNQNV (SEQ ID NO: 760); SSNQTQ (SEQ ID NO: 761); SSNQTV (SEQ ID NO: 762); SSNSTV (SEQ ID NO: 763); SSNTNV (SEQ ID NO: 764); SSNTQV (SEQ ID NO: 765); SSNTVG (SEQ ID NO: 766); SSNVAQ (SEQ ID NO: 767); SSNVAS (SEQ ID NO: 768); SSNVGT (SEQ ID NO: 769); SSNVNG (SEQ ID NO: 770); SSNVNQ (SEQ ID NO: 771); SSNVNS (SEQ ID NO: 772); SSNVNT (SEQ ID NO: 773); SSNVQS (SEQ ID NO: 774); SSNVQT (SEQ ID NO: 775); SSNVTG (SEQ ID NO: 776); SSNVTQ (SEQ ID NO: 777); SSNVTS (SEQ ID NO: 778); SSQNTV (SEQ ID NO: 779); SSQQSV (SEQ ID NO: 780); SSQQTQ (SEQ ID NO: 781); SSQQTV (SEQ ID NO: 782); SSQSTQ (SEQ ID NO: 783); SSQTSV (SEQ ID NO: 784); SSQVNS (SEQ ID NO: 785); SSQVSN (SEQ ID NO: 786); SSQVSQ (SEQ ID NO: 787); SSQVSS (SEQ ID NO: 788); SSQVST (SEQ ID NO: 789); SSQVTG (SEQ ID NO: 790); SSQVTQ (SEQ ID NO: 791); SSQVTS (SEQ ID NO: 792); SSQVTT (SEQ ID NO: 793); SSSNAV (SEQ ID NO: 794); SSSNQV (SEQ ID NO: 795); SSSNSV (SEQ ID NO: 796); SSSQAQ (SEQ ID NO: 797); SSSQNQ (SEQ ID NO: 798); SSSQQQ (SEQ ID NO: 799); SSSQQT (SEQ ID NO: 800); SSSQQV (SEQ ID NO: 801); SSSQSQ (SEQ ID NO: 802); SSSQSV (SEQ ID NO: 803); SSSSGQ (SEQ ID NO: 804); SSSSGV (SEQ ID NO: 805); SSSSNV (SEQ ID NO: 806); SSSSQQ (SEQ ID NO: 807); SSSSQS (SEQ ID NO: 808); SSSTAV (SEQ ID NO: 809); SSSTNV (SEQ ID NO: 810); SSSTSV (SEQ ID NO: 811); SSSVAQ (SEQ ID NO: 812); SSSVAS (SEQ ID NO: 813); SSSVGG (SEQ ID NO: 814); SSSVGQ (SEQ ID NO: 815); SSSVGS (SEQ ID NO: 816); SSSVNG (SEQ ID NO: 817); SSSVNS (SEQ ID NO: 818); SSSVNT (SEQ ID NO: 819); SSSVQG (SEQ ID NO: 820); SSSVQN (SEQ ID NO: 821); SSSVQS (SEQ ID NO: 822); SSSVQT (SEQ ID NO: 823); SSSVSG (SEQ ID NO: 824); SSSVSN (SEQ ID NO: 825); SSSVSQ (SEQ ID NO: 826); SSSVSS (SEQ ID NO: 827); SSSVST (SEQ ID NO: 828); STNGSV (SEQ ID NO: 829); STNSGV (SEQ ID NO: 830); STNSNV (SEQ ID NO: 831); STNTNV (SEQ ID NO: 832); STNTQV (SEQ ID NO: 833); STNVGS (SEQ ID NO: 834); STNVNG (SEQ ID NO: 835); STNVNS (SEQ ID NO: 836); STNVSG (SEQ ID NO: 837); STNVSS (SEQ ID NO: 838); STNVTG (SEQ ID NO: 839); STNVTS (SEQ ID NO: 840); STQQSG (SEQ ID NO: 841); STQQSQ (SEQ ID NO: 842); STQQSV (SEQ ID NO: 843); STQQTQ (SEQ ID NO: 844); STQQTS (SEQ ID NO: 845); STQQTV (SEQ ID NO: 846); STQSNV (SEQ ID NO: 847); STQSTV (SEQ ID NO: 848); STQTSS (SEQ ID NO: 849); STQTSV (SEQ ID NO: 850); STQVNS (SEQ ID NO: 851); STQVSG (SEQ ID NO: 852); STQVSN (SEQ ID NO: 853); STQVST (SEQ ID NO: 854); STQVTG (SEQ ID NO: 855); STQVTN (SEQ ID NO: 856); STQVTQ (SEQ ID NO: 857); STQVTS (SEQ ID NO: 858); STQVTT (SEQ ID NO: 859); STSGNV (SEQ ID NO: 860); STSNAS (SEQ ID NO: 861); STSNGV (SEQ ID NO: 862); STSNQV (SEQ ID NO: 863); STSNSV (SEQ ID NO: 864); STSQAQ (SEQ ID NO: 865); STSQGV (SEQ ID NO: 866); STSQNQ (SEQ ID NO: 867); STSQQQ (SEQ ID NO: 868); STSQQV (SEQ ID NO: 869); STSQSQ (SEQ ID NO: 870); STSSAV (SEQ ID NO: 871); STSSGV (SEQ ID NO: 872); STSSQQ (SEQ ID NO: 873); STSSQS (SEQ ID NO: 874); STSSQV (SEQ ID NO: 875); STSSSQ (SEQ ID NO: 876); STSTAV (SEQ ID NO: 877); STSTGV (SEQ ID NO: 878); STSTQA (SEQ ID NO: 879); STSTQG (SEQ ID NO: 880); STSTQT (SEQ ID NO: 881); STSTQV (SEQ ID NO: 882); STSTSQ (SEQ ID NO: 883); STSTSV (SEQ ID NO: 884); STSVAG (SEQ ID NO: 885); STSVAN (SEQ ID NO: 886); STSVAQ (SEQ ID NO: 887); STSVAS (SEQ ID NO: 888); STSVAT (SEQ ID NO: 889); STSVGG (SEQ ID NO: 890); STSVGN (SEQ ID NO: 891); STSVGQ (SEQ ID NO: 892); STSVGS (SEQ ID NO: 893); STSVNG (SEQ ID NO: 894); STSVNN (SEQ ID NO: 895); STSVNS (SEQ ID NO: 896); STSVQG (SEQ ID NO: 897); STSVQN (SEQ ID NO: 898); STSVQQ (SEQ ID NO: 899); STSVQS (SEQ ID NO: 900); STSVQT (SEQ ID NO: 901); STSVSG (SEQ ID NO: 902); STSVSN (SEQ ID NO: 903); STSVSQ (SEQ ID NO: 904); STSVSS (SEQ ID NO: 905); STSVST (SEQ ID NO: 906); SVNGST (SEQ ID NO: 907); SVNGTS (SEQ ID NO: 908); SVNQAQ (SEQ ID NO: 909); SVNQQQ (SEQ ID NO: 910); SVNSGT (SEQ ID NO: 911); SVNSNQ (SEQ ID NO: 912); SVNSNS (SEQ ID NO: 913); SVNSTG (SEQ ID NO: 914); SVNSTS (SEQ ID NO: 915); SVNTGS (SEQ ID NO: 916); SVNTSG (SEQ ID NO: 917); SVQQSQ (SEQ ID NO: 918); SVQQST (SEQ ID NO: 919); SVQQTQ (SEQ ID NO: 920); SVQQTS (SEQ ID NO: 921); SVQQTT (SEQ ID NO: 922); SVQSNQ (SEQ ID NO: 923); SVQSNS (SEQ ID NO: 924); SVQSSG (SEQ ID NO: 925); SVQSSQ (SEQ ID NO: 926); SVQSSS (SEQ ID NO: 927); SVQSTG (SEQ ID NO: 928); SVQSTQ (SEQ ID NO: 929); SVQSTS (SEQ ID NO: 930); SVQTSG (SEQ ID NO: 931); SVQTSN (SEQ ID NO: 932); SVQTSS (SEQ ID NO: 933); SVQVSN (SEQ ID NO: 934); SVSGNT (SEQ ID NO: 935); SVSGQS (SEQ ID NO: 936); SVSNAQ (SEQ ID NO: 937); SVSNAS (SEQ ID NO: 938); SVSNGS (SEQ ID NO: 939); SVSNGT (SEQ ID NO: 940); SVSNQG (SEQ ID NO: 941); SVSNQS (SEQ ID NO: 942); SVSNQT (SEQ ID NO: 943); SVSNST (SEQ ID NO: 944); SVSNTG (SEQ ID NO: 945); SVSQAQ (SEQ ID NO: 946); SVSQAS (SEQ ID NO: 947); SVSQGQ (SEQ ID NO: 948); SVSQNQ (SEQ ID NO: 949); SVSQQG (SEQ ID NO: 950); SVSQQQ (SEQ ID NO: 951); SVSQQS (SEQ ID NO: 952); SVSQSG (SEQ ID NO: 953); SVSQSS (SEQ ID NO: 954); SVSQTQ (SEQ ID NO: 955); SVSSAQ (SEQ ID NO: 956); SVSSAS (SEQ ID NO: 957); SVSSGG (SEQ ID NO: 958); SVSSGS (SEQ ID NO: 959); SVSSGT (SEQ ID NO: 960); SVSSNG (SEQ ID NO: 961); SVSSNQ (SEQ ID NO: 962); SVSSNS (SEQ ID NO: 963); SVSSNT (SEQ ID NO: 964); SVSSQG (SEQ ID NO: 965); SVSSQQ (SEQ ID NO: 966); SVSSQS (SEQ ID NO: 967); SVSSQT (SEQ ID NO: 968); SVSSQV (SEQ ID NO: 969); SVSSSG (SEQ ID NO: 970); SVSSSQ (SEQ ID NO: 971); SVSSSS (SEQ ID NO: 972); SVSTAG (SEQ ID NO: 973); SVSTAS (SEQ ID NO: 974); SVSTAT (SEQ ID NO: 975); SVSTGS (SEQ ID NO: 976); SVSTNS (SEQ ID NO: 977); SVSTNT (SEQ ID NO: 978); SVSTQQ (SEQ ID NO: 979); SVSTQS (SEQ ID NO: 980); SVSTQT (SEQ ID NO: 981); SVSTSG (SEQ ID NO: 982); SVSTSN (SEQ ID NO: 983); SVSTSS (SEQ ID NO: 984); SVSTST (SEQ ID NO: 985); TASQSQ (SEQ ID NO: 986); TASQVQ (SEQ ID NO: 987); TGSNSV (SEQ ID NO: 988); TGSNVS (SEQ ID NO: 989); TGSSNV (SEQ ID NO: 990); TGSVNS (SEQ ID NO: 991); TGSVSN (SEQ ID NO: 992); TNSGSV (SEQ ID NO: 993); TNSGVS (SEQ ID NO: 994); TNSQVQ (SEQ ID NO: 995); TNSSGV (SEQ ID NO: 996); TNSVGS (SEQ ID NO: 997); TNSVSG (SEQ ID NO: 998); TQSQSQ (SEQ ID NO: 999); TQSQVQ (SEQ ID NO: 1000); TQSTVS (SEQ ID NO: 1001); TQSVSN (SEQ ID NO: 1002); TQSVSS (SEQ ID NO: 1003); TSNGVS (SEQ ID NO: 1004); TSNVGS (SEQ ID NO: 1005); TSNVSG (SEQ ID NO: 1006); TSSGNV (SEQ ID NO: 1007); TSSGVN (SEQ ID NO: 1008); TSSNGV (SEQ ID NO: 1009); TSSNSV (SEQ ID NO: 1010); TSSNVG (SEQ ID NO: 1011); TSSQSQ (SEQ ID NO: 1012); TSSSVQ (SEQ ID NO: 1013); TSSVGN (SEQ ID NO: 1014); TSSVNG (SEQ ID NO: 1015); TSSVSG (SEQ ID NO: 1016); TSSVSS (SEQ ID NO: 1017); TSSVTS (SEQ ID NO: 1018); TVNSGS (SEQ ID NO: 1019); TVNSSG (SEQ ID NO: 1020); TVSGNS (SEQ ID NO: 1021); TVSGSN (SEQ ID NO: 1022); TVSNGS (SEQ ID NO: 1023); TVSNSG (SEQ ID NO: 1024); TVSNST (SEQ ID NO: 1025); TVSNVG (SEQ ID NO: 1026); TVSQNQ (SEQ ID NO: 1027); TVSQQV (SEQ ID NO: 1028); TVSQSG (SEQ ID NO: 1029); TVSQSQ (SEQ ID NO: 1030); TVSSGN (SEQ ID NO: 1031); TVSSNG (SEQ ID NO: 1032); TVSSNQ (SEQ ID NO: 1033); TVSSNS (SEQ ID NO: 1034); TVSSSQ (SEQ ID NO: 1035); TVSTSG (SEQ ID NO: 1036); TVSTSN (SEQ ID NO: 1037); TVSTSS (SEQ ID NO: 1038); TVSTST (SEQ ID NO: 1039); VGSTNS (SEQ ID NO: 1040); VNSTSG (SEQ ID NO: 1041); VNSTSN (SEQ ID NO: 1042); VSSQSQ (SEQ ID NO: 1043); VSSQVQ (SEQ ID NO: 1044); VSSTNG (SEQ ID NO: 1045); VTSNSG (SEQ ID NO: 1046); VTSQSQ (SEQ ID NO: 1047), SVNDLV (SEQ ID NO: 1059); LKVKVL (SEQ ID NO: 1060), NKGAII (SEQ ID NO: 1061), and NVSTSG (SEQ ID NO: 1062).

In particular embodiments, the peptide includes one or more of the following hexapeptides, where each indicated amino acid is either an L-amino acid or a D-amino acid: SVNLDV (SEQ ID NO: 5); VEALYL (SEQ ID NO: 1048); LYQLEN (SEQ ID NO: 1049); VQIVYK (SEQ ID NO: 123); GYVIIK (SEQ ID NO: 1050); SNQNNF (SEQ ID NO: 1051); SSQVTQ (SEQ ID NO: 791); SVLTSL (SEQ ID NO: 1052); SSTNVG (SEQ ID NO: 1053); SVSSSY (SEQ ID NO: 1054); GAILSS (SEQ ID NO: 1055); GAIIGL (SEQ ID NO: 1056); AIIGLM (SEQ ID NO: 1057); MVGGVV (SEQ ID NO: 220); GGVVIA (SEQ ID NO: 1058).

Variants of peptides disclosed herein can also be used, so long as the variant continues to contribute to fibril formation. "Variants" include protein sequences having one or more amino acid additions, deletions, stop positions, or substitutions, as compared to a protein sequence disclosed elsewhere herein.

**An** amino acid substitution can be a conservative or a non-conservative substitution. Variants of protein sequence disclosed herein can include those having one or more conservative amino acid substitutions. A "conservative substitution" or "conservative amino acid substitution" involves a substitution found in one of the following conservative substitutions groups: Group 1: Alanine (Ala; A), Glycine (Gly; G), Serine (Ser; S), Threonine (Thr; T); Group 2: Aspartic acid (Asp; D), Glutamic acid (Glu; E); Group 3: Asparagine (Asn; N), Glutamine (Gln; Q); Group 4: Arginine (Arg; R), Lysine (Lys; K), Histidine (His; H); Group 5: Isoleucine (Ile; I), Leucine (Leu; L), Methionine (Met; M), Valine (Val; V); and Group 6: Phenylalanine (Phe; F), Tyrosine (Tyr; Y), Tryptophan (Trp; W).

Additionally, amino acids can be grouped into conservative substitution groups by similar function, chemical structure, or composition (e.g., acidic, basic, aliphatic, aromatic, or sulfur-containing). For example, an aliphatic grouping may include, for purposes of substitution, G, A, V, L, and I. Other groups including amino acids that are considered conservative substitutions for one another include: sulfur-containing: M and C; acidic: D, E, N, and Q; small aliphatic, nonpolar or slightly polar residues: A, S, T, P, and G; polar, negatively charged residues and their amides: D, N, E, and Q; polar, positively charged residues: H, R, and K; large aliphatic, nonpolar residues: M, L, I, V, and C; and large aromatic residues: F, Y, and W.

Non-conservative substitutions include those that significantly affect: the structure of the peptide backbone in the area of the alteration (e.g., the alpha-helical or beta-sheet structure); the charge or hydrophobicity of the molecule at the target site; or the bulk of the side chain. Non-conservative substitutions which in general are expected to produce the greatest changes in the protein's properties are those in which (i) a hydrophilic residue (e.g. S or T) can be substituted for (or by) a hydrophobic residue (e.g. L, I, F, V, or A); (ii) a C or P can be substituted for (or by) any other residue; (iii) a residue having an electropositive side chain (e.g. K, R, or H) can be substituted for (or by) an electronegative residue (e.g. Q or D); or (iv) a residue having a bulky side chain (e.g. F), can be substituted for (or by) one not having a bulky side chain, (e.g. G). Additional information is found in Creighton (1984) Proteins, W.H. Freeman and Company.

Variants of fibril-forming peptides disclosed herein include proteins that share: 70% sequence identity with any of SEQ ID NOs: 1-1064; 75% sequence identity with any of SEQ ID NOs: 1-1064; 80% sequence identity with any of SEQ ID NOs: 1-1064; 81% sequence identity with any of SEQ ID NOs: 1-1064; 82% sequence identity with any of 1 - 1064; 83% sequence identity with any of SEQ ID NOs: 1-1064; 84% sequence identity with any of SEQ ID NOs: 1-1064; 85% sequence identity with any of SEQ ID NOs: 1-1064; 86% sequence identity with any of SEQ ID NOs: 1-1064; 87% sequence identity with any of SEQ ID NOs: 1-1064; 88% sequence identity with any of SEQ ID NOs: 1-1064; 89% sequence identity with any of SEQ ID NOs: 1-1064; 90% sequence identity with any of SEQ ID NOs: 1-1064; 91% sequence identity with any of SEQ ID NOs: 1-1064; 92% sequence identity with any of SEQ ID NOs: 1-1064; 93% sequence identity with any of SEQ ID NOs: 1-1064; 94% sequence identity with any of SEQ ID NOs: 1-1064; 95% sequence identity with any of SEQ ID NOs: 1-1064; 96% sequence identity with any of SEQ ID NOs: 1-1064; 97% sequence identity with any of SEQ ID NOs: 1-1064; 98% sequence identity with any of SEQ ID NOs: 1-1064; or 99% sequence identity with any of SEQ ID NOs: 1-1064.

"Percent(%) amino acid sequence identity" with respect to the sequences identified herein is defined as the percentage of amino acid residues in a candidate sequence that are identical with the amino acid residues in the reference sequence after aligning the sequences and introducing gaps, if necessary, to achieve the maximum percent sequence identity. Alignment for purposes of determining percent amino acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. For example, % amino acid sequence identity values generated using the WU-BLAST-2 computer program (Altschul et al., Methods in Enzymology, 266:460-480 (1996)) uses several search parameters, most of which are set to the default values. Those that are not set to default values (i.e., the adjustable parameters) are set with the following values: overlap span = 1, overlap fraction = 0.125, word threshold (T) = 11 and scoring matrix BLOSUM62.

In particular embodiments, fibrils formed from the peptides disclosed herein are capable of forming a complex with one or more plasma proteins. A "complex" refers to a molecular entity formed by non-covalent association involving two or more component molecular entities (ionic or uncharged). The bonding between the components is normally weaker than in a covalent bond. Typically, the dissociation constant for a complex ("K_{d}") is equal to or greater than 1 µM.

Nonlimiting examples of such plasma proteins include (symbol for plasma protein indicated in parenthesis after the plasma protein): Apolipoprotein B-100 (A); Complement C3 (B); Complement C1s (C); Beta-2-glycoprotein 1 (D); Clusterin (E); Coagulation Factor V (F); Complement C1r (G); Apolipoprotein A-I (H); ITIH2 (I); Complement 1qB (J); Apolipoprotein A-IV (K); Complement Factor H (L); Fibrinogen beta chain (M); Complement C4-A (N); Transthyretin (0); SerpinG1 Plasma protease C1 inhibitor (P); Fibrinogen alpha chain (Q); Complement C1qA (R); Vitronectin (S); Serpina-1 Alpha-1-antitrypsin (T); VitaminD-binding protein (U); Haptoglobin-related protein (V); ITIH4 (W); Fibrinogen gamma chain (X); SerpinC1 Antithrombin-III (Y); Apolipoprotein A-2 (Z); Complement Factor H-related protein (AA); Gelsolin (BB); Complement factor B (CC); Alpha-2-HS-glycoprotein (DD); Serum paraoxinase/arylesterase 1 (EE); Complement C5 (FF); Apolipoprotein C5 (GG); Apolipoprotein C-II (HH); Apolipoprotein C-I (II); ITIH1 (JJ); von Willebrand factor (KK); Ceruloplasmin (LL); Apolipoprotein E (MM); Filamin-A (NN); Histidine-rich glycoprotein (00); SerpinF2 alpha-2-antiplasmin (PP); Coagulation factor II Prothrombin (QQ); Coagulation factor X (RR); Vitamin K-dependent protein (SS); Apolipoprotein C-III (TT); Alpha-1-acid glycoprotein 2 (UU); Coagulation factor IX (VV); Apolipoprotein M (WW); Serum Albumin (XX).

Examples of peptide fibril/plasma protein complexes that are capable of being formed include (symbol for hexamer followed by symbol for plasma protein): 1B, 2B, 3B, 4B, 5B, 6B, 7B, 8B, 9B, 10B, 11B, 12B, 13B, 14B, 15B, 16B, 17B, 18B, 19B, 20B, 21B, 22B, 23B, 24B, 25B, 26B, 27B, 28B, 29B, 30B, 31B, 32B, 33B, 34B, 35B, 36B, 37B, 38B, 39B, 40B, 41B, 42B, 43B, 44B, 45B, 46B, 47B, 48B, 49B, 50B, 51B, 52B, 53B, 54B, 55B, 56B, 57B, 58B, 59B, 60B, 61B, 62B, 63B, 64B, 65B, 66B, 67B, 68B, 69B, 70B, 71B, 72B, 73B, 74B, 75B, 76B, 77B, 78B, 79B, 80B, 81B, 82B, 83B, 84B, 85B, 86B, 87B, 88B, 89B, 90B, 91B, 92B, 93B, 94B, 95B, 96B, 97B, 98B, 99B, 100B, 101B, 102B, 103B, 104B, 105B, 106B, 107B, 108B, 109B, 110B, 111B, 112B, 113B, 114B, 115B, 116B, 117B, 118B, 119B, 120B, 121B, 122B, 123B, 124B, 125B, 126B, 127B, 128B, 129B, 130B, 131B, 132B, 133B, 134B, 135B, 136B, 137B, 138B, 139B, 140B, 141B, 142B, 143B, 144B, 145B, 146B, 147B, 148B, 149B, 150B, 151B, 152B, 153B, 154B, 155B, 156B, 157B, 158B, 159B, 160B, 161B, 162B, 163B, 164B, 165B, 166B, 167B, 168B, 169B, 170B, 171B, 172B, 173B, 174B, 175B, 176B, 177B, 178B, 179B, 180B, 181B, 182B, 183B, 184B, 185B, 186B, 187B, 188B, 189B, 190B, 191B, 192B, 193B, 194B, 195B, 196B, 197B, 198B, 199B, 200B, 201B, 202B, 203B, 204B, 205B, 206B, 207B, 208B, 209B, 210B, 211B, 212B, 213B, 214B, 215B, 216B, 217B, 218B, 219B, 220B, 221B, 222B, 223B, 224B, 225B, 226B, 227B, 228B, 229B, 230B, 231B, 232B, 233B, 234B, 235B, 236B, 237B, 238B, 239B, 240B, 241B, 242B, 243B, 244B, 245B, 246B; and 247B-1047B; 1C, 2C, 3C, 4C, 5C, 6C, 7C, 8C, 9C, 10C, 11C, 12C, 13C, 14C, 15C, 16C, 17C, 18C, 19C, 20C, 21C, 22C, 23C, 24C, 25C, 26C, 27C, 28C, 29C, 30C, 31C, 32C, 33C, 34C, 35C, 36C, 37C, 38C, 39C, 40C, 41C, 42C, 43C, 44C, 45C, 46C, 47C, 48C, 49C, 50C, 51C, 52C, 53C, 54C, 55C, 56C, 57C, 58C, 59C, 60C, 61C, 62C, 63C, 64C, 65C, 66C, 67C, 68C, 69C, 70C, 71C, 72C, 73C, 74C, 75C, 76C, 77C, 78C, 79C, 80C, 81C, 82C, 83C, 84C, 85C, 86C, 87C, 88C, 89C, 90C, 91C, 92C, 93C, 94C, 95C, 96C, 97C, 98C, 99C, 100C, 101C, 102C, 103C, 104C, 105C, 106C, 107C, 108C, 109C, 110C, 111C, 112C, 113C, 114C, 115C, 116C, 117C, 118C, 119C, 120C, 121C, 122C, 123C, 124C, 125C, 126C, 127C, 128C, 129C, 130C, 131C, 132C, 133C, 134C, 135C, 136C, 137C, 138C, 139C, 140C, 141C, 142C, 143C, 144C, 145C, 146C, 147C, 148C, 149C, 150C, 151C, 152C, 153C, 154C, 155C, 156C, 157C, 158C, 159C, 160C, 161C, 162C, 163C, 164C, 165C, 166C, 167C, 168C, 169C, 170C, 171C, 172C, 173C, 174C, 175C, 176C, 177C, 178C, 179C, 180C, 181C, 182C, 183C, 184C, 185C, 186C, 187C, 188C, 189C, 190C, 191C, 192C, 193C, 194C, 195C, 196C, 197C, 198C, 199C, 200C, 201C, 202C, 203C, 204C, 205C, 206C, 207C, 208C, 209C, 210C, 211C, 212C, 213C, 214C, 215C, 216C, 217C, 218C, 219C, 220C, 221C, 222C, 223C, 224C, 225C, 226C, 227C, 228C, 229C, 230C, 231C, 232C, 233C, 234C, 235C, 236C, 237C, 238C, 239C, 240C, 241C, 242C, 243C, 244C, 245C, 246C; and 247C-1047C; 1F, 2F, 3F, 4F, 5F, 6F, 7F, 8F, 9F, 10F, 11F, 12F, 13F, 14F, 15F, 16F, 17F, 18F, 19F, 20F, 21F, 22F, 23F, 24F, 25F, 26F, 27F, 28F, 29F, 30F, 31F, 32F, 33F, 34F, 35F, 36F, 37F, 38F, 39F, 40F, 41F, 42F, 43F, 44F, 45F, 46F, 47F, 48F, 49F, 50F, 51F, 52F, 53F, 54F, 55F, 56F, 57F, 58F, 59F, 60F, 61F, 62F, 63F, 64F, 65F, 66F, 67F, 68F, 69F, 70F, 71F, 72F, 73F, 74F, 75F, 76F, 77F, 78F, 79F, 80F, 81F, 82F, 83F, 84F, 85F, 86F, 87F, 88F, 89F, 90F, 91F, 92F, 93F, 94F, 95F, 96F, 97F, 98F, 99F, 100F, 101F, 102F, 103F, 104F, 105F, 106F, 107F, 108F, 109F, 110F, 111F, 112F, 113F, 114F, 115F, 116F, 117F, 118F, 119F, 120F, 121F, 122F, 123F, 124F, 125F, 126F, 127F, 128F, 129F, 130F, 131F, 132F, 133F, 134F, 135F, 136F, 137F, 138F, 139F, 140F, 141F, 142F, 143F, 144F, 145F, 146F, 147F, 148F, 149F, 150F, 151F, 152F, 153F, 154F, 155F, 156F, 157F, 158F, 159F, 160F, 161F, 162F, 163F, 164F, 165F, 166F, 167F, 168F, 169F, 170F, 171F, 172F, 173F, 174F, 175F, 176F, 177F, 178F, 179F, 180F, 181F, 182F, 183F, 184F, 185F, 186F, 187F, 188F, 189F, 190F, 191F, 192F, 193F, 194F, 195F, 196F, 197F, 198F, 199F, 200F, 201F, 202F, 203F, 204F, 205F, 206F, 207F, 208F, 209F, 210F, 211F, 212F, 213F, 214F, 215F, 216F, 217F, 218F, 219F, 220F, 221F, 222F, 223F, 224F, 225F, 226F, 227F, 228F, 229F, 230F, 231F, 232F, 233F, 234F, 235F, 236F, 237F, 238F, 239F, 240F, 241F, 242F, 243F, 244F, 245F, 246F; and 247F-1047F; 1G, 2G, 3G, 4G, 5G, 6G, 7G, 8G, 9G, 10G, 11G, 12G, 13G, 14G, 15G, 16G, 17G, 18G, 19G, 20G, 21G, 22G, 23G, 24G, 25G, 26G, 27G, 28G, 29G, 30G, 31G, 32G, 33G, 34G, 35G, 36G, 37G, 38G, 39G, 40G, 41G, 42G, 43G, 44G, 45G, 46G, 47G, 48G, 49G, 50G, 51G, 52G, 53G, 54G, 55G, 56G, 57G, 58G, 59G, 60G, 61G, 62G, 63G, 64G, 65G, 66G, 67G, 68G, 69G, 70G, 71G, 72G, 73G, 74G, 75G, 76G, 77G, 78G, 79G, 80G, 81G, 82G, 83G, 84G, 85G, 86G, 87G, 88G, 89G, 90G, 91G, 92G, 93G, 94G, 95G, 96G, 97G, 98G, 99G, 100G, 101G, 102G, 103G, 104G, 105G, 106G, 107G, 108G, 1090, 110G, 1110, 112G, 113G, 114G, 115G, 116G, 117G, 118G, 119G, 120G, 121G, 122G, 123G, 124G, 125G, 126G, 127G, 128G, 129G, 130G, 131G, 1320, 133G, 134G, 135G, 136G, 137G, 138G, 139G, 140G, 141G, 142G, 143G, 144G, 145G, 146G, 147G, 148G, 149G, 150G, 151G, 152G, 153G, 154G, 155G, 156G, 157G, 158G, 159G, 160G, 161G, 162G, 163G, 164G, 165G, 166G, 167G, 168G, 169G, 170G, 171G, 172G, 173G, 1740, 175G, 176G, 177G, 178G, 179G, 180G, 181G, 182G, 183G, 184G, 185G, 186G, 187G, 188G, 189G, 190G, 191G, 192G, 193G, 194G, 195G, 196G, 197G, 198G, 199G, 200G, 201G, 202G, 203G, 204G, 205G, 206G, 207G, 208G, 209G, 210G, 211G, 212G, 213G, 214G, 215G, 216G, 217G, 218G, 219G, 220G, 221G, 222G, 223G, 224G, 225G, 226G, 227G, 228G, 229G, 230G, 231G, 232G, 233G, 234G, 235G, 236G, 237G, 238G, 239G, 240G, 241G, 242G, 243G, 244G, 245G, 246G; and 247G-1047G; 1M, 2M, 3M, 4M, 5M, 6M, 7M, 8M, 9M, 10M, 11M, 12M, 13M, 14M, 15M, 16M, 17M, 18M, 19M, 20M, 21M, 22M, 23M, 24M, 25M, 26M, 27M, 28M, 29M, 30M, 31M, 32M, 33M, 34M, 35M, 36M, 37M, 38M, 39M, 40M, 41M, 42M, 43M, 44M, 45M, 46M, 47M, 48M, 49M, 50M, 51M, 52M, 53M, 54M, 55M, 56M, 57M, 58M, 59M, 60M, 61M, 62M, 63M, 64M, 65M, 66M, 67M, 68M, 69M, 70M, 71M, 72M, 73M, 74M, 75M, 76M, 77M, 78M, 79M, 80M, 81M, 82M, 83M, 84M, 85M, 86M, 87M, 88M, 89M, 90M, 91M, 92M, 93M, 94M, 95M, 96M, 97M, 98M, 99M, 100M, 101M, 102M, 103M, 104M, 105M, 106M, 107M, 108M, 109M, 110M, 111M, 112M, 113M, 114M, 115M, 116M, 117M, 118M, 119M, 120M, 121M, 122M, 123M, 124M, 125M, 126M, 127M, 128M, 129M, 130M, 131M, 132M, 133M, 134M, 135M, 136M, 137M, 138M, 139M, 140M, 141M, 142M, 143M, 144M, 145M, 146M, 147M, 148M, 149M, 150M, 151M, 152M, 153M, 154M, 155M, 156M, 157M, 158M, 159M, 160M, 161M, 162M, 163M, 164M, 165M, 166M, 167M, 168M, 169M, 170M, 171M, 172M, 173M, 174M, 175M, 176M, 177M, 178M, 179M, 180M, 181M, 182M, 183M, 184M, 185M, 186M, 187M, 188M, 189M, 190M, 191M, 192M, 193M, 194M, 195M, 196M, 197M, 198M, 199M, 200M, 201M, 202M, 203M, 204M, 205M, 206M, 207M, 208M, 209M, 210M, 211M, 212M, 213M, 214M, 215M, 216M, 217M, 218M, 219M, 220M, 221M, 222M, 223M, 224M, 225M, 226M, 227M, 228M, 229M, 230M, 231M, 232M, 233M, 234M, 235M, 236M, 237M, 238M, 239M, 240M, 241M, 242M, 243M, 244M, 245M, 246M; and 247M-1047M; 1J, 2J, 3J, 4J, 5J, 6J, 7.1, 8J, 9J, 10J, 11J, 12J, 13J, 14J, 15J, 16J, 17J, 18J, 19J, 20J, 21J, 22J, 23J, 24.1, 25J, 26J, 27J, 28J, 29J, 30J, 31J, 32J, 33J, 34J, 35J, 36J, 37J, 38J, 39J, 40J, 41J, 42J, 43J, 44J, 45J, 46J, 47J, 48.1, 49J, 50J, 51J, 52J, 53J, 54J, 55J, 56J, 57J, 58J, 59J, 60J, 61J, 62J, 63J, 64J, 65J, 66J, 67J, 68J, 69J, 70J, 71.1, 72J, 73.1, 74J, 75J, 76J, 77J, 78J, 79J, 80J, 81J, 82J, 83J, 84J, 85J, 86J, 87J, 88J, 89J, 90J, 91J, 92J, 93J, 94J, 95J, 96J, 97J, 98J, 99J, 100J, 101J, 102J, 103J, 104J, 105J, 106J, 107J, 108J, 109J, 110J, 111J, 112J, 113J, 114J, 115J, 116J, 117J, 118J, 119J, 120J, 121J, 122J, 123J, 124.1, 125J, 126J, 127J, 128J, 129J, 130J, 131J, 132J, 133J, 134J, 135J, 136J, 137J, 138J, 139J, 140J, 141J, 142J, 143J, 144J, 145J, 146J, 147J, 148J, 149J, 150J, 151J, 152J, 153J, 154J, 155J, 156J, 157J, 158J, 159J, 160J, 161J, 162J, 163J, 164J, 165J, 166J, 167J, 168J, 169J, 170J, 171J, 172J, 173J, 174J, 175J, 176J, 177J, 178J, 179J, 180J, 181J, 182J, 183.1, 184J, 185J, 186.1, 187J, 188J, 189.1, 190J, 191J, 192J, 193J, 194J, 195J, 196J, 197J, 198J, 199J, 200J, 201J, 202J, 203J, 204J, 205J, 206J, 207J, 208J, 209J, 210J, 211J, 212J, 213J, 214J, 215J, 216J, 217J, 218J, 219J, 220J, 221J, 222J, 223J, 224J, 225J, 226J, 227J, 228J, 229J, 230J, 231J, 232J, 233J, 234.1, 235J, 236J, 237J, 238J, 239J, 240J, 241J, 242J, 243J, 244J, 245J, 246J; and 247J-1047J; 1L, 2L, 3L, 4L, 5L, 6L, 7L, 8L, 9L, 10L, 11L, 12L, 13L, 14L, 15L, 16L, 17L, 18L, 19L, 20L, 21L, 22L, 23L, 24L, 25L, 26L, 27L, 28L, 29L, 30L, 31L, 32L, 33L, 34L, 35L, 36L, 37L, 38L, 39L, 40L, 41L, 42L, 43L, 44L, 45L, 46L, 47L, 48L, 49L, 50L, 51L, 52L, 53L, 54L, 55L, 56L, 57L, 58L, 59L, 60L, 61L, 62L, 63L, 64L, 65L, 66L, 67L, 68L, 69L, 70L, 71L, 72L, 73L, 74L, 75L, 76L, 77L, 78L, 79L, 80L, 81L, 82L, 83L, 84L, 85L, 86L, 87L, 88L, 89L, 90L, 91L, 92L, 93L, 94L, 95L, 96L, 97L, 98L, 99L, 100L, 101L, 102L, 103L, 104L, 105L, 106L, 107L, 108L, 109L, 110L, 111L, 112L, 113L, 114L, 115L, 116L, 117L, 118L, 119L, 120L, 121L, 122L, 123L, 124L, 125L, 126L, 127L, 128L, 129L, 130L, 131L, 132L, 133L, 134L, 135L, 136L, 137L, 138L, 139L, 140L, 141L, 142L, 143L, 144L, 145L, 146L, 147L, 148L, 149L, 150L, 151L, 152L, 153L, 154L, 155L, 156L, 157L, 158L, 159L, 160L, 161L, 162L, 163L, 164L, 165L, 166L, 167L, 168L, 169L, 170L, 171L, 172L, 173L, 174L, 175L, 176L, 177L, 178L, 179L, 180L, 181L, 182L, 183L, 184L, 185L, 186L, 187L, 188L, 189L, 190L, 191L, 192L, 193L, 194L, 195L, 196L, 197L, 198L, 199L, 200L, 201L, 202L, 203L, 204L, 205L, 206L, 207L, 208L, 209L, 210L, 211L, 212L, 213L, 214L, 215L, 216L, 217L, 218L, 219L, 220L, 221L, 222L, 223L, 224L, 225L, 226L, 227L, 228L, 229L, 230L, 231L, 232L, 233L, 234L, 235L, 236L, 237L, 238L, 239L, 240L, 241L, 242L, 243L, 244L, 245L, 246L; and 247L-1047L; 1V, 2V, 3V, 4V, 5V, 6V, 7V, 8V, 9V, 10V, 11V, 12V, 13V, 14V, 15V, 16V, 17V, 18V, 19V, 20V, 21V, 22V, 23V, 24V, 25V, 26V, 27V, 28V, 29V, 30V, 31V, 32V, 33V, 34V, 35V, 36V, 37V, 38V, 39V, 40V, 41V, 42V, 43V, 44V, 45V, 46V, 47V, 48V, 49V, 50V, 51V, 52V, 53V, 54V, 55V, 56V, 57V, 58V, 59V, 60V, 61V, 62V, 63V, 64V, 65V, 66V, 67V, 68V, 69V, 70V, 71V, 72V, 73V, 74V, 75V, 76V, 77V, 78V, 79V, 80V, 81V, 82V, 83V, 84V, 85V, 86V, 87V, 88V, 89V, 90V, 91V, 92V, 93V, 94V, 95V, 96V, 97V, 98V, 99V, 100V, 101V, 102V, 103V, 104V, 105V, 106V, 107V, 108V, 109V, 110V, 111V, 112V, 113V, 114V, 115V, 116V, 117V, 118V, 119V, 120V, 121V, 122V, 123V, 124V, 125V, 126V, 127V, 128V, 129V, 130V, 131V, 132V, 133V, 134V, 135V, 136V, 137V, 138V, 139V, 140V, 141V, 142V, 143V, 144V, 145V, 146V, 147V, 148V, 149V, 150V, 151V, 152V, 153V, 154V, 155V, 156V, 157V, 158V, 159V, 160V, 161V, 162V, 163V, 164V, 165V, 166V, 167V, 168V, 169V, 170V, 171V, 172V, 173V, 174V, 175V, 176V, 177V, 178V, 179V, 180V, 181V, 182V, 183V, 184V, 185V, 186V, 187V, 188V, 189V, 190V, 191V, 192V, 193V, 194V, 195V, 196V, 197V, 198V, 199V, 200V, 201V, 202V, 203V, 204V, 205V, 206V, 207V, 208V, 209V, 210V, 211V, 212V, 213V, 214V, 215V, 216V, 217V, 218V, 219V, 220V, 221V, 222V, 223V, 224V, 225V, 226V, 227V, 228V, 229V, 230V, 231V, 232V, 233V, 234V, 235V, 236V, 237V, 238V, 239V, 240V, 241V, 242V, 243V, 244V, 245V, 246V; and 247V-1047V; 1KK, 2KK, 3KK, 4KK, 5KK, 6KK, 7KK, 8KK, 9KK, 10KK, 11KK, 12KK, 13KK, 14KK, 15KK, 16KK, 17KK, 18KK, 19KK, 20KK, 21KK, 22KK, 23KK, 24KK, 25KK, 26KK, 27KK, 28KK, 29KK, 30KK, 31KK, 32KK, 33KK, 34KK, 35KK, 36KK, 37KK, 38KK, 39KK, 40KK, 41KK, 42KK, 43KK, 44KK, 45KK, 46KK, 47KK, 48KK, 49KK, 50KK, 51KK, 52KK, 53KK, 54KK, 55KK, 56KK, 57KK, 58KK, 59KK, 60KK, 61KK, 62KK, 63KK, 64KK, 65KK, 66KK, 67KK, 68KK, 69KK, 70KK, 71KK, 72KK, 73KK, 74KK, 75KK, 76KK, 77KK, 78KK, 79KK, 80KK, 81KK, 82KK, 83KK, 84KK, 85KK, 86KK, 87KK, 88KK, 89KK, 90KK, 91KK, 92KK, 93KK, 94KK, 95KK, 96KK, 97KK, 98KK, 99KK, 100KK, 101KK, 102KK, 103KK, 104KK, 105KK, 106KK, 107KK, 108KK, 109KK, 110KK, 111KK, 112KK, 113KK, 114KK, 115KK, 116KK, 117KK, 118KK, 119KK, 120KK, 121KK, 122KK, 123KK, 124KK, 125KK, 126KK, 127KK, 128KK, 129KK, 130KK, 131KK, 132KK, 133KK, 134KK, 135KK, 136KK, 137KK, 138KK, 139KK, 140KK, 141KK, 142KK, 143KK, 144KK, 145KK, 146KK, 147KK, 148KK, 149KK, 150KK, 151KK, 152KK, 153KK, 154KK, 155KK, 156KK, 157KK, 158KK, 159KK, 160KK, 161KK, 162KK, 163KK, 164KK, 165KK, 166KK, 167KK, 168KK, 169KK, 170KK, 171KK, 172KK, 173KK, 174KK, 175KK, 176KK, 177KK, 178KK, 179KK, 180KK, 181KK, 182KK, 183KK, 184KK, 185KK, 186KK, 187KK, 188KK, 189KK, 190KK, 191KK, 192KK, 193KK, 194KK, 195KK, 196KK, 197KK, 198KK, 199KK, 200KK, 201KK, 202KK, 203KK, 204KK, 205KK, 206KK, 207KK, 208KK, 209KK, 210KK, 211KK, 212KK, 213KK, 214KK, 215KK, 216KK, 217KK, 218KK, 219KK, 220KK, 221KK, 222KK, 223KK, 224KK, 225KK, 226KK, 227KK, 228KK, 229KK, 230KK, 231KK, 232KK, 233KK, 234KK, 235KK, 236KK, 237KK, 238KK, 239KK, 240KK, 241KK, 242KK, 243KK, 244KK, 245KK, 246KK; and 247KK-1047KK; 1LL, 2LL, 3LL, 4LL, 5LL, 6LL, 7LL, 8LL, 9LL, 10LL, 11LL, 12LL, 13LL, 14LL, 15LL, 16LL, 17LL, 18LL, 19LL, 20LL, 21LL, 22LL, 23LL, 24LL, 25LL, 26LL, 27LL, 28LL, 29LL, 30LL, 31LL, 32LL, 33LL, 34LL, 35LL, 36LL, 37LL, 38LL, 39LL, 40LL, 41LL, 42LL, 43LL, 44LL, 45LL, 46LL, 47LL, 48LL, 49LL, 50LL, 51LL, 52LL, 53LL, 54LL, 55LL, 56LL, 57LL, 58LL, 59LL, 60LL, 61LL, 62LL, 63LL, 64LL, 65LL, 66LL, 67LL, 68LL, 69LL, TOLL, 71LL, 72LL, 73LL, 74LL, 75LL, 76LL, 77LL, 78LL, 79LL, 80LL, 81LL, 82LL, 83LL, 84LL, 85LL, 86LL, 87LL, 88LL, 89LL, 90LL, 91LL, 92LL, 93LL, 94LL, 95LL, 96LL, 97LL, 98LL, 99LL, 100LL, 101LL, 102LL, 103LL, 104LL, 105LL, 106LL, 107LL, 108LL, 109LL, 110LL, 111LL, 112LL, 113LL, 114LL, 115LL, 116LL, 117LL, 118LL, 119LL, 120LL, 121LL, 122LL, 123LL, 124LL, 125LL, 126LL, 127LL, 128LL, 129LL, 130LL, 131LL, 132LL, 133LL, 134LL, 135LL, 136LL, 137LL, 138LL, 139LL, 140LL, 141LL, 142LL, 143LL, 144LL, 145LL, 146LL, 147LL, 148LL, 149LL, 150LL, 151LL, 152LL, 153LL, 154LL, 155LL, 156LL, 157LL, 158LL, 159LL, 160LL, 161LL, 162LL, 163LL, 164LL, 165LL, 166LL, 167LL, 168LL, 169LL, 170LL, 171LL, 172LL, 173LL, 174LL, 175LL, 176LL, 177LL, 178LL, 179LL, 180LL, 181LL, 182LL, 183LL, 184LL, 185LL, 186LL, 187LL, 188LL, 189LL, 190LL, 191LL, 192LL, 193LL, 194LL, 195LL, 196LL, 197LL, 198LL, 199LL, 200LL, 201LL, 202LL, 203LL, 204LL, 205LL, 206LL, 207LL, 208LL, 209LL, 210LL, 211LL, 212LL, 213LL, 214LL, 215LL, 216LL, 217LL, 218LL, 219LL, 220LL, 221LL, 222LL, 223LL, 224LL, 225LL, 226LL, 227LL, 228LL, 229LL, 230LL, 231LL, 232LL, 233LL, 234LL, 235LL, 236LL, 237LL, 238LL, 239LL, 240LL, 241LL, 242LL, 243LL, 244LL, 245LL, 246LL; and 247LL-1047LL; 1XX, 2XX, 3XX, 4XX, 5XX, 6XX, 7XX, 8XX, 9XX, 10XX, 11XX, 12XX, 13XX, 14XX, 15XX, 16XX, 17XX, 18XX, 19XX, 20XX, 21XX, 22XX, 23XX, 24XX, 25XX, 26XX, 27XX, 28XX, 29XX, 30XX, 31XX, 32XX, 33XX, 34XX, 35XX, 36XX, 37XX, 38XX, 39XX, 40XX, 41XX, 42XX, 43XX, 44XX, 45XX, 46XX, 47XX, 48XX, 49XX, 50XX, 51XX, 52XX, 53XX, 54XX, 55XX, 56XX, 57XX, 58XX, 59XX, 60XX, 61XX, 62XX, 63XX, 64XX, 65XX, 66XX, 67XX, 68XX, 69XX, 70XX, 71XX, 72XX, 73XX, 74XX, 75XX, 76XX, 77XX, 78XX, 79XX, 80XX, 81XX, 82XX, 83XX, 84XX, 85XX, 86XX, 87XX, 88XX, 89XX, 90XX, 91XX, 92XX, 93XX, 94XX, 95XX, 96XX, 97XX, 98XX, 99XX, 100XX, 101XX, 102XX, 103XX, 104XX, 105XX, 106XX, 107XX, 108XX, 109XX, 110XX, 111XX, 112XX, 113XX, 114XX, 115XX, 116XX, 117XX, 118XX, 119XX, 120XX, 121XX, 122XX, 123XX, 124XX, 125XX, 126XX, 127XX, 128XX, 129XX, 130XX, 131XX, 132XX, 133XX, 134XX, 135XX, 136XX, 137XX, 138XX, 139XX, 140XX, 141XX, 142XX, 143XX, 144XX, 145XX, 146XX, 147XX, 148XX, 149XX, 150XX, 151XX, 152XX, 153XX, 154XX, 155XX, 156XX, 157XX, 158XX, 159XX, 160XX, 161XX, 162XX, 163XX, 164XX, 165XX, 166XX, 167XX, 168XX, 169XX, 170XX, 171XX, 172XX, 173XX, 174XX, 175XX, 176XX, 177XX, 178XX, 179XX, 180XX, 181XX, 182XX, 183XX, 184XX, 185XX, 186XX, 187XX, 188XX, 189XX, 190XX, 191XX, 192XX, 193XX, 194XX, 195XX, 196XX, 197XX, 198XX, 199XX, 200XX, 201XX, 202XX, 203XX, 204XX, 205XX, 206XX, 207XX, 208XX, 209XX, 210XX, 211XX, 212XX, 213XX, 214XX, 215XX, 216XX, 217XX, 218XX, 219XX, 220XX, 221XX, 222XX, 223XX, 224XX, 225XX, 226XX, 227XX, 228XX, 229XX, 230XX, 231XX, 232XX, 233XX, 234XX, 235XX, 236XX, 237XX, 238XX, 239XX, 240XX, 241XX, 242XX, 243XX, 244XX, 245XX, 246XX; and 247XX-1047XX.

**As** indicated, these peptides can be formulated for administration to the respiratory tract. Therapeutically effective amounts (also referred to herein as doses) that lead to effective treatments can be initially estimated based on results from in vitro assays and/or animal model studies. Such information can be used to more accurately determine useful doses in subjects of interest.

**The** actual dose amount administered to a particular subject can be determined by a physician, veterinarian, or researcher taking into account parameters such as physical, physiological and psychological factors including target, body weight, type of condition, stage or severity of condition, previous or concurrent therapeutic interventions, idiopathy of the subject, etc. Exemplary doses can include, for example, 0.1 µg/kg - 1000 mg/kg.

### Exemplary Embodiments.

1. A method of targeting and activating B-1a lymphocytes in the pleural cavity to treat a medical condition including an inflammatory or autoimmune component in a subject in need thereof including:
   administering a therapeutically effective amount of a formulation including a fibril or a fibril-forming peptide of SEQ ID NO: 1053 to the respiratory tract of the subject in need thereof,
   thereby targeting and activating B-1a lymphocytes in the pleural cavity of the subject and treating the medical condition.
2. A method of embodiment 1 wherein the medical condition including an inflammatory or autoimmune component is multiple sclerosis, rheumatoid arthritis, Huntington's disease, Parkinson's disease, amyotrophic lateral sclerosis, traumatic brain injury, cancer, ischemic reperfusion injury, arthritis asthma, or chronic obstructive pulmonary disease (COPD).
3. A method of targeting and activating B-1a lymphocytes and/or macrophages to suppress the immune system and treat a medical condition with an inflammatory or autoimmune component in a subject in need thereof including:
   administering a therapeutically effective amount of a formulation including a fibril or a fibril-forming peptide to the subject in need thereof,
   thereby suppressing the immune system and treating the medical condition in the subject.
4. A method of embodiment 3 wherein the medical condition is multiple sclerosis, rheumatoid arthritis, Huntington's disease, Parkinson's disease, amyotrophic lateral sclerosis, traumatic brain injury, cancer, ischemic reperfusion injury, arthritis asthma, or chronic obstructive pulmonary disease (COPD).
5. A method of embodiment 3 or 4 wherein the administering is to the respiratory tract.
6. A method of embodiment 1 or 5 wherein the administering to the respiratory tract is to the alveolar region of the lung.
7. A method of any of embodiments 3-6 wherein the fibril or fibril-forming peptide is SEQ ID NO: 1053.
8. A method of any of embodiments 3-6 wherein the fibril or fibril-forming peptide is a hexapeptide that oligomerizes with a Rosetta energy at or below -23 kcal/mol.
9. A method of any of embodiments 3-8 wherein the fibril or fibril-forming peptide includes one or more of SEQ ID NOs. 1-1062.
10. A method of any of embodiments 3-8 wherein the fibril or fibril-forming peptide includes one or more of SEQ ID NO: 5; SEQ ID NO: 1048; SEQ ID NO: 1049; SEQ ID NO: 123; SEQ ID NO: 1050; SEQ ID NO: 1051; SEQ ID NO: 791; SEQ ID NO: 1052; SEQ ID NO: 1053; SEQ ID NO: 1054; SEQ ID NO: 1055; SEQ ID NO: 1056; SEQ ID NO: 1057; SEQ ID NO: 220; and SEQ ID NO: 1058.
11. A method of any of the preceding embodiments wherein the formulation is a conductive formulation.
12. A method of any of the preceding embodiments wherein the formulation further includes a mucoactive or mucolytic agent.
13. A method of any of the preceding embodiments wherein the formulation is a dry powder formulation.
14. A method of any of the preceding embodiments wherein the formulation further includes a conductive agent.
15. A method of any of the preceding embodiments wherein said targeting and activating of B-1a lymphocytes and/or macrophages occurs in the pleural cavity.
16. A method of any of the preceding embodiments wherein the methods suppress the immune system.
17. A method of embodiment 16 wherein the suppressing the immune system includes suppression of one or more proinflammatory cytokines.
18. A method of embodiment 17 wherein the one or more proinflammatory cytokines are TNFα, IL-6, and/or IFNγ.
19. A conductive formulation including a fibril or fibril-forming peptide and a conductive agent.
20. A conductive formulation of embodiment 19 wherein the conductive agent is a hypertonic saline solution.
21. A conductive formulation of embodiment 19 or 20 further including a mucoactive or mucolytic agent.
22. A conductive formulation of embodiment 19, 20 or 21 wherein the fibril or fibril-forming peptide is a hexapeptide that oligomerizes with a Rosetta energy at or below -23 kcal/mol.
23. A conductive formulation of embodiment 19, 20 or 21 wherein the fibril of fibril forming peptide is SEQ ID NO: 1053.
24. A conductive formulation of embodiment 19, 20 or 21 wherein the fibril or fibril-forming peptide includes a peptide of one or more of SEQ ID NOs. 1-1062.
25. A conductive formulation of embodiment 19, 20 or 21 wherein the fibril or fibril-forming peptide includes a peptide of one or more of SEQ ID NOs. SEQ ID NO: 5; SEQ ID NO: 1048; SEQ ID NO: 1049; SEQ ID NO: 123; SEQ ID NO: 1050; SEQ ID NO: 1051; SEQ ID NO: 791; SEQ ID NO: 1052; SEQ ID NO: 1053; SEQ ID NO: 1054; SEQ ID NO: 1055; SEQ ID NO: 1056; SEQ ID NO: 1057; SEQ ID NO: 220; and SEQ ID NO: 1058.

Example 1. Amyloid fibrils, composed of hexapeptides, when injected into the peritoneum of mice, stimulate an immune suppressive response of sufficient magnitude to reduce the paralytic signs of experimental autoimmune encephalomyelitis (EAE). Analysis of the differential gene expression pattern in PBMCs revealed the amyloidogenic peptides, e.g. Tau 623-628 (SEQ ID NO: 123), induced a type 1 interferon (IFN) response. Plasmacytoid dendritic cells were the source of type 1 IFN, which was induced by NETosis arising from neutrophil endocytosis of the amyloid fibrils. Production of the type 1 IFN was therapeutic in Th1 induced EAE, but exacerbated the paralytic signs of Th17 induced disease. However, not all amyloidogenic peptides induced equivalent amounts of type 1 IFN. The induction of type 1 IFN appeared to correlate with the amount of fibril formation as measured by thioflavin T. A set of peptides with a polar fibril interface, e.g. Amylin 28-33 (SEQ ID NO: 1053), did not form measurable amounts of fibrils in physiological buffers, induced minimal amounts of type 1 IFN, but nevertheless were therapeutic, reducing IFNγ, TNFα, and IL-6 production by PBMCs and thus providing evidence of a second immune suppressive pathway. Further proof of the importance of this second immune suppressive pathway was the ability of amyloidogenic peptides to be therapeutic in IFNα/βR -/- animals with EAE.

To better define this second immune suppressive pathway, the induced effects of the amyloid fibrils at the site of injection in the peritoneum were investigated. The peritoneal cavity contains a variety of specialized cells including two types of resident macrophages (MΦs), large peritoneal MΦs (LPM) (CD11b^{hi}F4/80^{hi}MHC-II-) and small peritoneal MΦs (SPM) (CD11b⁺F4/80^{lo}MHC-II^{hi}), B-1a lymphocytes (CD19^{hi}CD5⁺CD23), and more common components of blood, including the B-2 lymphocytes (CD19⁺CD5-CD23⁺), T lymphocytes, mast cells, neutrophils, eosinophils, and NK cells. The LPMs (F4/80^{hi}) are more prevalent than the SPMs (MHC-II), representing 90% of peritoneal MΦs. The B-1a and MΦs (LPM+SPM) each include 30% of the total peritoneal cells. Several groups have established that B-1a lymphocytes are distinguishable from the more plentiful B-2 lymphocytes and are enriched in body cavities. The chemokines and integrins, which are responsible for B-1a localization to the peritoneal cavity and their exodus when activated, have also been defined. The B-1a cell population is notable for its constitutive expression of IL-10, a well- established immune suppressive cytokine. IL-10 producing B cells, B10 cells, were initially shown by Janeway and colleagues to be necessary for the recovery from the signs of EAE and were subsequently demonstrated to be immune suppressive in animal models of multiple sclerosis, inflammatory bowel disease, collagen induced arthritis, lupus, stroke, insulin resistance, and allergic airway disease. The B10 cells in many of these studies were isolated from the spleen and not the peritoneal cavity.

Maximal immune suppression by B10 cells is observed after the cells are activated through TLR, CD40, or IL-21 ligation, all of which induce both an increase in IL-10 production and an egress of the cells from the peritoneum into secondary lymph organs. Reduction of symptoms in each of the inflammatory, autoimmune diseases correlated with reduction of TNFα, IL-6, and IFNγ, a pattern similar to that seen with the administration of the amyloidogenic peptides.

Methods. Induction of active EAE in mice by immunization with MOG and adjuvant. EAE was induced in female wild type C57BI/6 mice or µMT and IL-10 deficient mice on C57BI/6 background (Jackson Laboratories) by procedures previously described. Briefly, EAE was induced at 9 weeks of age by subcutaneous immunization in the flank with an emulsion containing 200µg myelin oligodendrocyte glycoprotein35-55 (MOG₃₅₋₅₅; MEVGWYRSPFSRVVHLYRNGK (SEQ ID NO: 1065)) in saline and an equal volume of complete Freund's adjuvant containing 4µg/ml mycobacterium tuberculosis H37RA (Disco Laboratories). All mice were given 400ng of pertussis toxin (List Biological) intraperitoneal (i.p.) at 0 and 48 h post-immunization. The signs of neurological impairment were scored as follows: 0, no clinical disease; 1, tail weakness; 2, hindlimb weakness; 3, complete hindlimb paralysis; 4, hindlimb paralysis and some forelimb weakness; 5, moribund or dead. When animals exhibited an average of level one to two for clinical signs they were injected in the peritoneum with 10µg of Tau 623-628, Amylin 28-33 peptide (SEQ ID NO: 1053), or PBS daily. For intranasal inoculation, 10µg of Amylin 28-33 in 10 µl PBS was gradually released into the nostrils of anesthetized mice. All animal protocols were approved by institutional IACUC.

Adoptive transfer of B-1a lymphocytes. B-1a lymphocytes were purified by cell sorting of peritoneal cells from wild type C57BI/6 mice. Ten days following induction of active EAE in µMT mice, 3.5x10⁵ B-1a lymphocytes were transferred into the peritoneal cavity. Mice were treated with 10µg Amylin 28-33 or PBS daily for 14 days. Control µMT mice with EAE were treated with 10 µg Amylin 28-33 without transfer of B-1a lymphocytes. Mice were examined daily for clinical signs of EAE and were scored on a five point scale described above.

Microscopy. C57BI/6 female mice were injected with FITC-Tau 623-628, and peritoneal cells were isolated after 10 minutes, washed, stained with rat antimouse CD19 (PE), F4/80 (Alexa Fluor 647), and DAPI, washed, and plated on polylysine coated microscope slides. Cells were visualized using a Leica TCS SP8 white light laser confocal microscope.

Bioluminescence experiments. B-1a lymphocytes and peritoneal MΦs were purified by cell sorting of peritoneal cells isolated from luciferase transgenic mice ((B6.FVB-Ptprca Tg(CAG-luc,-GFP)L2G85Chco Thy1a/J), which express the CAT-luc-eGFP, L2G85 transgene). In the initial experiment 2 x 10⁵ and 1 x 10⁵ B-1a lymphocytes were injected in the peritoneum of C57BL/6 female albino mice (B6(Cg)-Tyr^{c-2J}/J), followed by injection of 10µg of Tau 623-628 to activate the lymphocytes, and 0.3mg/g body weight of luciferin substrate (D luciferin firefly L-8220 Biosynth) to initiate the bioluminescence imaging. The location of the luciferase expressing cells was measured by imaging every 5 minutes for 75 minutes.

To confirm the diminution of luminescence was due to the trafficking of the luc⁺ cells, and not the degradation of the luciferin, two C57BL/6 albino mice were injected with 10⁶ luc⁺ peritoneal MΦs, a third with 2x10⁵ B-1a lymphocytes, and fourth mouse serving as a BLI control was injected with luciferin only. The mice were subsequently injected with 10µg of Tau 623-628 and 0.3mg/gram of luciferin. Bioluminescence was measured immediately after the injection of luciferin and 5 minutes later. After thirty minutes the mice were reinjected with luciferin and the resultant luminescence measured. A similar injection and measurement was done after 60 minutes. The multiple injections of luciferin kept the drug level close to saturation during all measurements, so that any changes were due to cell movement. Mice were imaged using an IVIS100 charge-coupled device (CCD) imaging system (Xenogen, Alameda, CA). Imaging data were analyzed and quantified with Living Image software 4.4 (Xenogen).

Peptide synthesis and preparation of FITC-Tau. Peptides were synthesized using solid phase techniques and commercially available Fmoc amino acids, resins, and reagents (PE Biosystems, Foster City CA, and Bache, Torrance, CA) on an Applied Biosystems 433A peptide synthesizer as previously described. Wender et al. (2000) Proc. Natl. Acad. Sci. U S A 97(24):13003-13008. Purity of the peptides was shown to be greater than 90% using a PE Biosystems 700E HPLC and a reversephase column (Alltech Altima). The molecular weight of the peptides was confirmed using matrix assisted laser desorption mass spectrometry. To prevent excess amounts of fluorophore in the fibrils, Tau 623-628 was mixed at a ratio of 10:1 with an analog with FITC attached to the amino terminus of Tau 623-628 with an amino caproic acid linker. The resulting fibril mixture is referred to in the text as FITC-Tau.

RNA isolation, chip hybridization, and qPCR. Total RNA was extracted from FACS purified B-1a lymphocytes and peritoneal MΦs pooled from 3-6 C57BL/6 female mice injected with 10µg of LPS, Tau 623-628, Amylin 28-33, or PBS using Trizol reagent and the Qiagen RNeasy micro kit. First strand cDNA was synthesized with 30-50ng of total RNA using Superscript III first strand synthesis supermix for qRT-PCR. QPCR assays were performed using the 7900HT Fast Real Time PCR System (Applied Biosystems), and the Taqman Gene Expression Arrays (Applied Biosystems) using commercially available primers (ABI). All assays were performed according to manufacturer's instructions. The comparative Ct method for relative quantification (ΔΔCt) was used to compare gene expression. Housekeeping gene expression was used to normalize expression using the following equation: Normalized expression = 2^{[Ct (house-keeping gene) - Ct (gene)]}.

Gene expression changes associated with treatment with LPS, Amylin 28-33, and Tau 623-628, were quantified using a microarray (SurePrint G3 Mouse; Agilent Technologies). RNA quality was shown to be suitable for microarray experiments (2100 Bioanalyzer, Agilent Technologies, Inc.). Analysis and quantitation of the data were done using GeneSpring and Ingenuity software.

Flow cytometry. Peritoneal cavity cells were obtained by flushing the peritoneal cavity with 10ml of cold PBS containing 0.1% bovine serum albumin and 5mM EDTA. Single-cell suspensions were stained with the following fluorochome conjugates: CD5 (PE Cy5, PE, or APC), CD19 (PE Cy5.5, Pacific Blue, or APC), CD11b (Pacific Blue or PE), F4/80 (APC), CD21 (APC), CD23 (PE), Gr-1 (PECy7), B220 (APC-Cy7), CD80/86 (biotin-Qdot605-streptavidin), CD4 (FITC), CD3 (PerCP-Cy5.5), and IgM (Alexa Fluor 700). Sorting of cells used a FACS-Aria or a Fortessa (BD) equipped with four lasers and optics for 22-paramenter analysis. Analysis was done using FlowJo.

TLR binding assays. Commercially available HEK293 cells transfected with murine TLR4, MD-2, CD14, or TLR2 and an inducible secreted embryonic alkaline phosphatase (InVivoGen) were plated in a 96 well plate. The SEAP reporter gene in the cells is under the control of an IL-12 p40 minimal promoter fused to five NF-κB and AP-1-binding sites. Stimulation with either a TLR4 or TLR 2 ligand activates NF-κB and AP-1, which induces the production of SEAP. Levels of the secreted alkaline phosphatase measured in the cell culture medium, is proportional to the stimulation of the TLR pathway. Background levels are measured using HEK-Blue Null cells, which are transfected with the alkaline phosphatase, but not the TLR receptor. The TLR4 transfected cells were grown to confluence and 2, 1, 0.2µg of LPS, or 10µl of a set of amyloidogenic peptides were added to each well in duplicate in HEK-Blue detection medium (InVivoGen). In the case of the TLR2 transfected cells, PAM2CSK4 was the positive control, and only 2µg of LPS were assayed. The plates were incubated for 12 hours at 37° C, and the resulting blue color measured by reading the absorption at 650nm.

Results. Amyloid fibrils are endocytosed by peritoneal B cells and MΦs. Amyloid fibrils are endocytosed by MΦs, dendritic cells, microglia, and neutrophils. To determine whether peritoneal cells bind the amyloid fibrils, fluorescently labeled Tau 623-628 was mixed with unlabeled peptide at a 1:10 ratio, and the resultant fibrils injected in the peritoneum of healthy, wild type C57BL/6 mice. After twenty minutes the peritoneal cells were collected by lavage, stained with anti-CD19 and anti-F4/80, and layered on poly-lysine coated slides. Confocal microscopy was performed and revealed the presence of intracellular fluorescent fibrils, suggesting that the fibrils were bound and were endocytosed by both B cells (CD19⁺) and MΦs (F4/80⁺) (FIGs. 1A and B). Viewing multiple fields revealed that the majority of the fluorescent fibrils were bound by F4/80⁺ cells with a smaller percentage binding CD19⁺ B cells.

Analysis of peritoneal cells isolated from mice injected with the fluorescent amyloid fibrils by flow cytometry confirmed and extended the microscopic study. The composition of the various cell types in the peritoneum can be delineated using 10-color, 12-parameter hi-dimensional analysis (FIGs. 2A and 2B). When the peritoneal MΦs (both SPM and LPM), mast cells, T, B-2, and B-1 lymphocytes are delineated with antibodies against CD11b, CD5, and CD19, a more complex pattern of uptake and trafficking is apparent (FIG. 1C). Within 10 minutes of the FITC-Tau injection more than 70% of the B-1 and B-2 lymphocytes and LPM are FITC positive. T lymphocytes and mast cells are minimally stained, demonstrating specific binding or uptake by B cells and MΦs. Five hours after injection of the amyloid fibrils an interesting pattern emerges. The majority of the CD11b high population is significantly reduced from 45% to 3% of total peritoneal cells (FIG. 2C). Most of the B-1a population has disappeared, with the remaining cells being FITC-Tau negative (FIG. 2D). The majority of T lymphocytes and mast cells remained unstained with the fibrils. Collectively, the flow cytometry studies revealed that B cells and MΦs bind the fibrils, and that the relative number of B-1a lymphocytes (CD19^{hi}CD5⁺) and the LPMs (CD11b^{hi}) was dramatically reduced 5 hours after injection of the amyloid.

Whether fibrils were selectively toxic to the B-1a and LPMs was evaluated. This was ruled unlikely when no cell death was observed when the peritoneal cells were cultured in vitro with the fluorescent amyloid (FIG. 3). Rather than killing the cells, a testable hypothesis was that the fibrils induced the exodus of the two cell populations from the peritoneum.

µMT and IL-10 knockout mice do not respond to therapy. To determine the importance of B lymphocytes in the mode of action of the amyloid fibrils, EAE was induced in µMT mice, which due to a mutation in the transmembrane region of IgM lack expression of all subtypes of B cells. The paralytic signs of the disease were induced in these animals, consistent with induction of the disease by T lymphocytes, but neither Tau 623-628, nor Amylin 28-33 was therapeutic when compared to the effect seen in wild type mice (FIGs. 4A-4C). The therapeutic activity of the amyloidogenic peptides appeared to require the presence of B cells, most likely B-1a lymphocytes based on the composition of the peritoneum and the microscopy.

B-1a lymphocytes are characterized by the constitutive expression of relatively large amounts of IL-10. To establish whether this cytokine was central to therapeutic effects of the peptides and to correlate the activity with this B cell subtype, 10µg Amylin 28-33 was used to treat EAE induced in IL-10 knockout animals (FIGs. 4D and 4E). Again, the peptide was ineffective in this animal model, establishing that both IL-10 and B lymphocytes are central to the therapeutic activity of the amyloidogenic peptides.

Adoptive transfer of B-1a lymphocytes restored therapeutic efficacy of amyloidogenic peptides in µMT animals. The characteristics of peritoneal B-1a lymphocytes clearly correlate with the apparent requirements for therapeutic function. Thus replacement of this population in µMT mice should restore the immunosuppressive activity of the amyloid fibrils. Peritoneal cells were isolated from C57BL/6 mice, and B-1a lymphocytes (CD19^{hi}CD5⁺) were purified by cell sorting. EAE was induced in µMT mice and, on day 10 after induction, prior to the appearance of clinical signs, the mice were injected in the peritoneal cavity with purified B-1a lymphocytes (3.5x10⁵ cells). The mice were divided into two groups and treated daily with Amylin 28-33 (10 µg) or buffer alone (FIG. 4F). µMT mice induced with EAE that did not receive B-1a lymphocytes but were treated with Amylin 28-33 were used for comparison. Interestingly, only mice that received the transfer of B-1a lymphocytes treated daily with the amyloidogenic peptide exhibited reduced paralytic signs of EAE. Adoptive transfer of untreated B-1a lymphocytes was as ineffective as buffer control, establishing not only the importance of B-1a lymphocytes, but that the cell population needs to be activated by the fibrils to be effective. This corroborates studies that regulatory B cells are more potent suppressors of autoimmunity than their non-activated counterparts.

Amyloidogenic peptides induce exodus of B-1a lymphocytes and LPM from the peritoneal cavity. Real time measurement of bioluminescence demonstrated that the amyloidogenic peptides induce an exodus of both B-1a lymphocytes and LPM from the peritoneum. B-1a lymphocytes (CD19^{hi}CD5⁺CD23⁻) and LPMs (CD11bhiF4/80hi) were sorted from peritoneal cells isolated from luciferase transgenic mice, C57BL/6 L2G85 (H-2d) (B6.FVB-Ptprca Tg(CAG-luc,-GFP)L2G85Chco Thy1a/J), that express the CAT-luc-eGFP, L2G85 transgene. In the initial experiment 1x10⁵ and 2x10⁵ B-1a lymphocytes were injected into the peritoneum of C57BL/6 female albino mice, followed by injection of 10µg of Tau 623-628 to activate the lymphocytes, and 300µg/g body weight of the substrate luciferin, to initiate the enzyme-substrate reaction for the bioluminescence imaging. The location of the luciferase expressing cells was monitored by imaging the mice every 5 minutes for 75 minutes (FIG. 5, top left). The diffuse distribution of the luminescence, corresponding to the peritoneal cavity, seen at early times, was reduced in intensity over time, with focal regions of intensity appearing to localize in inguinal lymph nodes beginning at 35 minutes (FIG. 5, top left). Measuring the total number of photons per second in the abdominal region revealed a rapid, dose dependent, reduction of light emitted over the 75 minutes of the experiment (FIG. 5, top right). Even though the size of the signal was proportional to the number of cells injected, the rate of reduction, or slope of the curve, was equivalent in the two animals. Such a result is consistent with the amyloid fibrils triggering egress of the B-1a lymphocytes from the peritoneum.

The experimental design of the initial experiment did not allow the reduction of the signal to be assigned unequivocally to the migration of the lymphocytes because the concentration of the luciferin also diminishes during the 75 minutes of examination. To better assign the basis of the reduction of luminescence to the migration of the luc⁺ cells, a second experiment was performed with four recipient mice. Two were injected with 1x10⁶ luc⁺ MΦs (LPMs), a third with 2x10⁵ B-1a lymphocytes, and fourth mouse serving as a control was injected only with luciferin. In this experiment, the mice were subsequently injected with 10µg of Tau 623-628 and luciferin. Bioluminescence was measured immediately after the injection of luciferin and 5 minutes later. After thirty minutes the mice were reinjected with luciferin and the resultant luminescence measured. A similar injection and measurement was done after 60 minutes. The multiple injections of luciferin kept the drug level close to saturation during all measurements, so that any changes were due to migration and trafficking. Using 1x10⁶ LPMs produced a vivid signal at all time points, whose details were similar to that observed in the initial experiment; increased distribution of light with time, along with a concentration in an area over inguinal lymph nodes (FIG. 5, bottom left). In the mouse receiving the B-1a lymphocytes, a less intense signal was observed, but nevertheless evidence of exodus of the luc⁺ cells from the peritoneum was apparent. When photons per second over the full body of each mouse were measured and plotted versus time, there was a proportional increase in signal as a function of time. In the case of the area corresponding to the inguinal lymph nodes, there was close to a 10-fold increase in luminescence from the measurement at 5 to 60 minutes (FIG. 5, bottom right). Collectively the imaging experiments establish that the amyloidogenic peptides induce a migration of both B-1a lymphocytes and LPMs from the peritoneum to the inguinal lymph nodes.

**To** further examine how amyloid fibrils induce an exodus of B-1a lymphocytes and LPMs a series of experiments was performed using IL-10 reporter mice in which IL-10 and GFP are connected via an internal ribosome entry site (IRES) to create a bicistronic message marking IL-10 secreting cells with fluorescence (Bouabe (2012) Scand. J. Immunol. 75(6):553-567). Because LPS induces B-1a cell migration from the peritoneum to the spleen, the effects of the amyloid fibrils were compared to those effects observed with the TLR4 ligand (Balabanian et al. (2003) J. Immunol. 170(6):3392-3400; Ghosn et al. (2011) Proc. Natl. Acad. Sci. U S A 108(7):2879-2884). To allow for maximal changes in IL-10 transcription, and because the resulting GFP is relatively long-lived, the experiment was designed to confirm exodus, and to identify possible sites of migration. Because previous experiments established that 80% of the B-1a and LPMs exited the peritoneum at 5 hours, time points for analysis were chosen at 30 minutes and at 24 hours after injection of the fibrils. Three groups of three IL-10 reporter C57BL/6 female mice were injected with 10µg LPS, 10µg Amylin 28-33, or buffer alone and after 5 or 24 hours the peritoneal cells were lavaged, the spleen and inguinal and axillary lymph nodes were dissected with the lymph nodes being pooled, and single cell suspensions were prepared and delineated using 10-color, 12-parameter hi-dimensional analysis. Similar cells and tissues also were taken from three wild type mice as additional controls. Injection of LPS not only increased the relative numbers of IL-10-secreting B-1a lymphocytes (from 25% to 40% in 24h), but it also induced higher levels of IL-10 as evidenced by higher levels of GFP median fluorescence intensity (MFI). In contrast, injection of Amylin 28-33 did not increase IL-10 secretion, however there was a decrease in the number of IL-10⁺ B-1a lymphocytes and LPMs in the peritoneum after 24 hours that was not observed with LPS.

In the spleen, LPS induced an increase in both the number of B-1a lymphocytes and the amount of IL-10 expressed per cell 24 hours after injection. Amylin fibrils did not induce an increase of the B-1a lymphocytes in spleen, but rather an apparent reduction in both numbers and IL-10 expression. The opposite pattern was observed in the pooled lymph nodes. LPS resulted in a slight increase of B-1a lymphocytes in the pooled lymph nodes. Injection of Amylin 28-33 increased the percentage of B-1a lymphocytes in the lymph node, with an increase in the amount of IL-10 expression. In IL-10 reporter mice with EAE, an additional pattern was observed. No significant increase in B-1a lymphocytes were detected in the brain or spinal cord, consistent with the hypothesis that the B-1a and MΦ populations migrate to the secondary lymph organs, and not to the primary sites of inflammation.

The experiments using the IL-10 reporter mice revealed that both LPS and the fibrils activated B-1a lymphocytes, SPM, and LPM, inducing IL-10 gene expression, and subsequent exodus from the peritoneum. However the magnitude of the increase and the details of the directions of migration differ. As previously reported, LPS activates the B-1a lymphocytes resulting in greater production of IL-10 and migration to the spleen, whereas the fibrils predominantly induced migration to the lymph nodes. Interestingly only B-1a lymphocytes expressing CD80/86 secrete IL-10, and IL-10 secreting B-1a lymphocytes were found in lymph nodes of normal animals with the relative number increased with injection of amylin fibrils. The flow cytometry experiments establish that the amyloidogenic peptides activate both B-1a lymphocytes and LPMs, resulting in both cell types trafficking to the draining lymph nodes.

Differential gene expression in B-1a lymphocytes and MΦs induced by amyloid fibrils. The migration of B-1a lymphocytes and LPMs after the injection of amyloid fibrils was consistent with the activation of both cell types. However, the receptor(s) for the fibrils has not been defined for either the B-1a lymphocytes or the peritoneal MΦs. Unlike migration induced by LPS stimulation, the fibrils composed of the peptides do not bind to TLR2 MD2/TLR4. A set of over fifteen different amyloidogenic peptides was screened for binding to commercially available HEK cells transfected with murine TLR2 or MD2/CD14/TLR4. The transfected cells contained a secretable form of alkaline phosphatase under the control of an NFkB promoter. None of the amyloid fibrils composed of the varying peptides was positive in this assay.

To confirm and increase the understanding of how the fibrils are activating the peritoneal cells, differential gene induction in purified B-1a and LPMs was analyzed. Making such measurements was complicated by the fact that LPS and the fibrils induce a rapid migration of the relevant cells from the peritoneal cavity, and consequently a high percentage would not be isolated by lavage an hour after injection. To minimize the population bias, and yet allow sufficient time for the fibrils to induce gene expression, cells were isolated between 30-40 minutes after injection of LPS or the amyloidogenic peptides. Consequently, the analysis is limited to gene expression in the 30-40 minutes after stimulation. Peritoneal cells from groups of three C57BL/6 female mice were isolated after injection with either LPS, fibrils composed of Amylin 28-33 or Tau 623-628, or buffer control. B-1a lymphocytes (CD19^{hi}CD5⁺CD23⁻) and LPMs (CD11b^{hi} MΦs) from the four groups of three mice were sorted into Trizol, RNA extracted, and gene expression measured using a murine Agilent whole genome expression microchip. Differential gene expression of the B-1a and LPMs was calculated by subtracting the gene expression data from cells isolated from mice injected with buffer from expression data from mice injected with LPS or the amyloid fibrils (FIG. 6A). All microarray data are available at the Gene Expression Omnibus (GEO) database (GEO series accession number: GSE73026).

The pattern of gene expression induced by LPS is well characterized in both cell types binding to CD14/TLR4 (Kawai & Akira (2010) Nat. Immunol. 11(5):373-384; Rossol, et al. (2011) Critical reviews in Immunol. 31(5):379-446) resulting in the induction of a wide spectrum of proinflammatory mediators such as IL-6, TNFα, type 1 IFN, Serpins, IL-1α and β, chemokines CXCL10, CXCL3, MYD88, and over 50 genes known to be induced by ReIA/p65 NFkB (Bode et al. (2012) Cellular Signalling 24(6):1185-1194). In peritoneal MΦs amyloidogenic peptides stimulated a distinctly different set of genes from those induced by LPS. To portray the variation, a heatmap of the correlation of a set of 730 annotated genes induced by LPS in MΦs demonstrates the gene expression signature induced by the two amyloidogenic peptides is similar and distinct from the pattern generated by injection with LPS (FIG. 6A). The majority of genes preferentially induced by the fibrils corresponded to MΦ stimulation, cytokine production, oxidative phosphorylation, and mitochondrial dysfunction pathways. The oxidative phosphorylation pathways were induced, demonstrated by the expression of a large number of mitochondrial genes composing the five complexes involved in mitochondrial electron transport and ATP production, characteristic of amyloid fibril interactions with mitochondria (DuBoff B, Feany M, & Gotz J (2013) Trends in Neurosciences 36(6):325-335).

In the case of B-1a lymphocytes, a less vivid difference between LPS and the peptides is seen, but the patterns induced by the two types of fibrils are distinguishable from that of LPS. In contrast, with MΦs, LPS and the amyloidogenic peptides both induced a pattern of expression characteristic of B cell activation. The amyloidogenic peptides stimulated CDC42 small effector protein) Calcium release (stim 1, orai 1 and 3), BcR (CD79, Syk, Lyn, PI3K, Akt, m-Tor, Bc12A1d, c-src, PTEN, and Vav-1) and CD40 signaling (Traf 2, 4, and 5), all of which induce NFκB activation. Even though the LPS and amyloidogenic peptides induced a large number of similar genes involved in lymphocyte activation, a clear distinction could be observed, with the amyloidogenic peptides inducing a set of immunosuppressive proteins such as BTLA, IRF4, and Siglec G.

To confirm the gene expression data from the chip, a set of genes was analyzed by qPCR (FIGs. 6B-6D). In these experiments RNA was isolated from B-1a, large and small MΦs using identical methods and similar times after injection of the stimulants as was done for the microarray. Consistent with the pathway analysis of the chip data, IL-6, TNF, IL-1β, and IFNβ1 were significantly induced by LPS in the peritoneal MΦs, and minimally by the peptide fibrils. Interestingly, the SPMs (CD11b⁺F4/80^{lo/-} MΦs) uniformly expressed greater amount of the inflammatory genes, particularly IL-1β, than the LPMs (CD11b^{hi}F4/80^{hi} MΦs). In the peritoneum, SPMs compose less than 10% of the MΦ population, are not the dominant cell type that endocytose the fibrils, nor do they represent the major depleted population of cells after injection of LPS or the fibrils. In contrast, the fibrils induced a set of genes associated with immune regulation, BTLA, Siglec G, and IRF4 in B-1a lymphocytes, and CD274 in LPMs. LPS induced some, but not all of these genes. The third set of genes analyzed were those known to be associated with cell activation. CD40, CD80, CD86, and semaphorin 4D were induced by both LPS and the fibrils in B-1a lymphocytes and both types of MΦs. CD83 was induced by both stimuli principally on the MΦs, while CD79a and Raftlin were induced on the B-1a lymphocytes.

The pattern of gene expression indicated that both types of amyloid fibrils activated the B-1a lymphocytes and both populations of the peritoneal MΦs (SPM and LPM). IL-10 gene expression was increased in both B-1a and LPMs, two of the cell types shown to traffic to lymph nodes. The induction of BTLA and Siglec G in the B-1a lymphocytes would increase their immune regulatory phenotype. The expression of IL-10 in the LPMs is consistent with the conversion of these cells to a M2 phenotype, also believed to suppress inflammatory responses.

Delivery to the respiratory tract retains the therapeutic efficacy of the amyloidogenic peptides. Peritoneal injection is not a practical route of drug administration for activation of B-1a lymphocytes in humans. However, B-1a lymphocytes also are plentiful in the pleural cavity of both mice and humans (Yenson V & Baumgarth N (2014) Methods Mol. Biol. 1190:17-34). To examine whether this alternative route of administration is both practical and sufficient for treatment, 10 µg Amylin 28-33 was administered daily intranasally to groups of 10 C57BL/6 mice with EAE. The paralytic signs of the disease were reduced in a fashion equivalent to that seen when the amyloidogenic peptide is injected i.p. (FIG. 7A). In addition, splenocytes from peptide treated mice exhibited a reduction in secretion of proinflammatory cytokines, IL-6, IFNγ, IL-2, and IL-17, in response to MOG₃₅₋₅₅ challenge in vitro, compared to control (FIG. 7B), a pattern identical to when Amylin 28-33 was injected (Kurnellas et al. (2013) Sci. Transl. Med. 5(179): 179ra142).

**The** success of delivery to the respiratory tract is consistent with a mode of action in which the B-1a lymphocytes play a central role, but also establish a potential route of administration that can be used in clinical trials in human patients.

Discussion. Amyloid fibrils composed of amyloidogenic peptides exhibit wide spectrum of biological activities, the sum of which results in an immune suppressive response of sufficient magnitude to be therapeutic in a robust model of multiple sclerosis. As molecular chaperones they bind a spectrum of proinflammatory mediators in plasma. In blood they are endocytosed by neutrophils, which induce the production of nets, which in turn induces plasmacytoid dendritic cells to secrete type 1 IFN. Here it is shown that fibrils bind and activate both B-1a lymphocytes and a subset of peritoneal MΦs known as large peritoneal MΦs (LPMs) (Ghosn EE, et al. (2010) Proc. Nat.l. Acad. Sci U S A 107(6):2568-2573), which are induced to increase IL-10 transcription and migrate out of the peritoneum to secondary lymph organs. The exodus results in the selective delivery of IL-10 to immunological sites shared with inflammatory T lymphocytes and their complementary antigen presenting cells. IL-10 effectively inhibits both inflammatory cell populations, with reduction of the production of proinflammatory cytokines, IL-6, TNFα, and IFNγ. This reduction of cytokines was the hallmark of the immune suppression induced in EAE by the amyloidogenic peptides. The peritoneal cells do not appear to migrate to the sites of inflammation in the CNS, and consequently do not need to cross the blood-brain barrier. Knockout mice were central to establishing the mechanism. The inability of the peptide amyloid to reduce inflammation in B cell-deficient µMT mice with EAE highlighted the importance of B cells. Flow cytometry and fluorescent microscopy were used to establish that in the peritoneum the relevant target in the B cell population was B-1a lymphocytes, a secretor of IL-10. Additional support for the role of IL-10 secreting B-1a lymphocytes was the failure of IL-10-/- mice with EAE to respond the amyloid therapy. Further support for the role of IL-10-secreting B-1a lymphocytes in the mechanism of action came from classic adoptive transfer experiments. The adoptive transfer of purified B-1a lymphocytes into µMT mice converted the B cell deficient mice from nonresponders to responders to the amyloidogenic peptides. An important point was that the transfer of the B-1a lymphocytes alone did not reduce the paralytic signs of EAE. The signs were reduced only after injection of the fibrils, which were shown to activate the transferred population.

Once activated, both the B-1a lymphocytes and the LPMs leave the peritoneum, but their trafficking patterns are less clear. Previous studies established that LPS activation of B-1a lymphocytes resulted in trafficking from the peritoneum to the spleen. Tedder and colleagues have shown activation of a splenic population of regulatory B cells that migrate to draining lymph nodes. Using real-time measurement of the trafficking of adoptively transferred luminescent B-1a lymphocytes and LPMs, revealed that activation with the amyloid fibrils resulted in migration to inguinal lymph nodes. Flow cytometric studies using IL-10 reporter mice supported both the timing and the location of the trafficking.

In the case of an intraperitoneal injection, the activation of the peritoneal cells would be expected to precede any biological activity stimulated by the fibrils in serum, and consequently should contribute to a greater percentage of the response. The proposed mode of action is consistent with the relatively long pharmacokinetics and pharmacodynamics of the amyloidogenic peptides. The fibrils themselves will have an expected half-life measured in minutes. However, the fibrils activate a set of peritoneal cells, which are the therapeutic agents and migrate to the secondary lymph organs, where they secrete immune suppressive IL-10. The cells do not appear to traffic to the sites of inflammation in the spine and the brain in mice with EAE and do not cross the blood-brain barrier, which is consistent with the documented scarcity of B lymphocytes in EAE lesions. The fate of the activated B-1a lymphocytes and the LPMs reflects the pharmacodynamics of the therapy, and not the fate of the amyloidogenic peptides. Similarly in the pharmacokinetics of the response, the one or two day delay in the reduction of the paralytic signs after the injection of the fibrils reflects the time necessary for the activation and migration of the peritoneal cells, combined with the immune suppression of a sufficiently large percentage of the inflammatory T lymphocytes. Reciprocally, cessation of therapy results in a 24 to 72 hour delay in the return of the paralytic signs, which is consistent with the half-life of the immune suppression induced by the IL-10 producing cells, and not the half-life of the fibrils. In many respects the therapeutic effects of the fibrils resemble pharmacokinetics of adoptive cell therapy rather than a classical small molecule therapeutic. The proposed mechanism of the migration of the immune suppressive cells to secondary lymph organs, where they suppress both circulating inflammatory antigen presenting cells and T lymphocytes, is consistent with published studies on B regulatory cells (Baumgarth N, Waffarn EE, & Nguyen TT (2015) Annals of the New York Academy of Sciences; Bouaziz JD, Yanaba K, & Tedder TF (2008) Immunological Reviews 224:201-214). The mechanism also argues that this therapeutic approach might be beneficial in a number of systemic inflammatory indications.

The fibrils induce a concomitant inflammatory response, most evidently in the SPMs, with induction of IL-1β, TNFα, and IL-6. Why this response does not dominate the immune suppressive effects can best be explained by the large excess of LPMs and B-1a lymphocytes and their greater propensity to rapidly traffic out of the peritoneum to the secondary lymphoid tissues.

The effective therapy with respiratory tract administration of the fibrils bodes well for translation to human therapy, particularly in light of the predominance of B-1a lymphocytes in the pleural cavity.

The amyloidogenic peptides disclosed herein are the first therapeutic that targets regulatory B cells. The extensive list of indications in which this population of cells limits inflammation is supportive of the potential for the strategy of using the amyloid fibrils in a spectrum of conditions with inflammatory or autoimmune components.

As will be understood by one of ordinary skill in the art, each embodiment disclosed herein can comprise, consist essentially of or consist of its particular stated element, step, ingredient or component. Thus, the terms "include" or "including" should be interpreted to recite: "comprise, consist of, or consist essentially of." The transition term "comprise" or "comprises" means includes, but is not limited to, and allows for the inclusion of unspecified elements, steps, ingredients, or components, even in major amounts. The transitional phrase "consisting of' excludes any element, step, ingredient or component not specified. The transition phrase "consisting essentially of" limits the scope of the embodiment to the specified elements, steps, ingredients or components and to those that do not materially affect the embodiment. A material effect would cause a statistically-significant reduction in effectiveness in the EAE animal model of MS.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Furthermore, numerous references have been made to patents, printed publications, journal articles and other written text throughout this specification (referenced materials herein). Each of the referenced materials are individually incorporated herein by reference in their entirety for their referenced teaching.

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

The particulars shown herein are by way of example and for purposes of illustrative discussion of the preferred embodiments of the present invention only and are presented in the cause of providing what is believed to be the most useful and readily understood description of the principles and conceptual aspects of various embodiments of the invention. In this regard, no attempt is made to show structural details of the invention in more detail than is necessary for the fundamental understanding of the invention, the description taken with the drawings and/or examples making apparent to those skilled in the art how the several forms of the invention may be embodied in practice.

Definitions and explanations used in the present disclosure are meant and intended to be controlling in any future construction unless clearly and unambiguously modified in the following examples or when application of the meaning renders any construction meaningless or essentially meaningless. In cases where the construction of the term would render it meaningless or essentially meaningless, the definition should be taken from Webster's Dictionary, 3rd Edition or a dictionary known to those of ordinary skill in the art, such as the Oxford Dictionary of Biochemistry and Molecular Biology (Ed. Anthony Smith, Oxford University Press, Oxford, 2004).

### CLAUSES

1. A method of treating multiple sclerosis in a subject in need thereof comprising:
   administering a therapeutically effective amount of a formulation comprising a fibril-forming peptide of SEQ ID NO: 1053 to the respiratory tract of the subject,
   thereby treating multiple sclerosis in the subject in need thereof.
2. A method of activating B-1a lymphocytes to treat an inflammatory or autoimmune condition in a subject in need thereof comprising:
   administering a therapeutically effective amount of a formulation comprising a fibril or a fibril-forming peptide to the subject in need thereof,
   thereby activating B-1a lymphocytes and treating the inflammatory or autoimmune condition in the subject in need thereof.
3. A method of clause 2 wherein the inflammatory or autoimmune condition comprises multiple sclerosis, rheumatoid arthritis, Huntington's disease, Parkinson's disease, amyotrophic lateral sclerosis, traumatic brain injury, cancer, ischemic reperfusion injury, arthritis asthma, or chronic obstructive pulmonary disease (COPD).
4. A method of clause 2 wherein the administering is to the respiratory tract.
5. A method of clause 4 wherein the administering to the respiratory tract is to the alveolar region of the lung.
6. A method of clause 2 wherein the fibril or fibril-forming peptide is SEQ ID NO: 1053.
7. A method of clause 2 wherein the fibril-forming peptide is a hexapeptide that oligomerizes with a Rosetta energy at or below -23 kcal/mol.
8. A method of clause 2 wherein the fibril or fibril-forming peptide comprises one or more of SEQ ID NOs. 1-1062.
9. A method of clause 2 wherein the fibril or fibril-forming peptide comprises one or more of SEQ ID NO: 5; SEQ ID NO: 1048; SEQ ID NO: 1049; SEQ ID NO: 123; SEQ ID NO: 1050; SEQ ID NO: 1051; SEQ ID NO: 791; SEQ ID NO: 1052; SEQ ID NO: 1053; SEQ ID NO: 1054; SEQ ID NO: 1055; SEQ ID NO: 1056; SEQ ID NO: 1057; SEQ ID NO: 220; and SEQ ID NO: 1058.
10. A method of clause 1 or 2 wherein the formulation is a conductive formulation.
11. A method of clause 1 or 2 wherein the formulation further comprises a mucoactive or mucolytic agent.
12. A method of clause 1 or 2 wherein the formulation is a dry powder formulation.
13. A method of clause 1 or 2 wherein the formulation further comprises a conductive agent.
14. A method of clause 2 wherein said activating of B-1a lymphocytes occurs in the pleural cavity.
15. A conductive formulation comprising a fibril or fibril-forming peptide and a conductive agent.
16. A conductive formulation of clause 15 wherein the conductive agent is a hypertonic saline solution.
17. A conductive formulation of clause 15 further comprising a mucoactive or mucolytic agent.
18. A conductive formulation of clause 15 wherein the fibril-forming peptide is a hexapeptide that oligomerizes with a Rosetta energy at or below -23 kcal/mol.
19. A conductive formulation of clause 15 wherein the fibril of fibril forming peptide comprises SEQ ID NO: 1053.
20. A conductive formulation of clause 15 wherein the fibril or fibril-forming peptide comprises one or more peptides selected from SEQ ID NOs. 1-1062.
21. A conductive formulation of clause 15 wherein the fibril or fibril-forming peptide comprises one or more peptides selected from SEQ ID NOs. SEQ ID NO: 5; SEQ ID NO: 1048; SEQ ID NO: 1049; SEQ ID NO: 123; SEQ ID NO: 1050; SEQ ID NO: 1051; SEQ ID NO: 791; SEQ ID NO: 1052; SEQ ID NO: 1053; SEQ ID NO: 1054; SEQ ID NO: 1055; SEQ ID NO: 1056; SEQ ID NO: 1057; SEQ ID NO: 220; and SEQ ID NO: 1058.

## Claims

1. A formulation comprising a fibril or a fibril-forming peptide for use in a method of activating B-1a lymphocytes to treat an inflammatory or autoimmune condition in a subject in need thereof, wherein the inflammatory or autoimmune condition comprises multiple sclerosis, rheumatoid arthritis, arthritis, asthma, chronic obstructive pulmonary disease (COPD), or allergic airway disease, the method comprising:
administering a therapeutically effective amount of the formulation to the respiratory tract of the subject in need thereof, thereby activating B-1a lymphocytes and treating the inflammatory or autoimmune condition in the subject in need thereof, wherein the fibril-forming peptide is a hexapeptide that oligomerizes with a Rosetta energy at or below -23 kcal/mol.

2. The formulation for use according to claim 1, wherein the administering to the respiratory tract is to the alveolar region of the lung.

3. The formulation for use according to claim 1, wherein the fibril or fibril-forming peptide comprises one or more of SEQ ID NOs. 1062, 1-1052 or 1054-1061.

4. The formulation for use according to claim 1, wherein the fibril or fibril-forming peptide comprises one or more of SEQ ID NO: 1062; SEQ ID NO: 5; SEQ ID NO: 1048; SEQ ID NO: 1049; SEQ ID NO: 123; SEQ ID NO: 1050; SEQ ID NO: 1051; SEQ ID NO: 791; SEQ ID NO: 1052; SEQ ID NO: 1054; SEQ ID NO: 1055; SEQ ID NO: 1056; SEQ ID NO: 1057; SEQ ID NO: 220; and SEQ ID NO: 1058.

5. The formulation for use according to claim 1, wherein the formulation is a conductive formulation.

6. The formulation for use according to claim 1, wherein the formulation further comprises a mucoactive or mucolytic agent.

7. The formulation for use according to claim 1, wherein the formulation is a dry powder formulation.

8. The formulation for use according to claim 1, wherein the formulation further comprises a conductive agent.

9. The formulation for use according to claim 1, wherein said activating of B-1a lymphocytes occurs in the pleural cavity.

10. A conductive formulation comprising a fibril or fibril-forming peptide and a conductive agent, wherein the fibril-forming peptide is a hexapeptide that oligomerizes with a Rosetta energy at or below -23 kcal/mol.

11. The conductive formulation according to claim 10, wherein the conductive agent is a hypertonic saline solution.

12. The conductive formulation according to claim 10, further comprising a mucoactive or mucolytic agent.

13. The conductive formulation according to claim 10, wherein the fibril or fibril-forming peptide comprises one or more peptides selected from SEQ ID NOs. 1062, 1-1052 or 1054-1061.

14. The conductive formulation according to claim 10, wherein the fibril or fibril-forming peptide comprises one or more peptides selected from SEQ ID NOs. SEQ ID NO: 1062; SEQ ID NO: 5; SEQ ID NO: 1048; SEQ ID NO: 1049; SEQ ID NO: 123; SEQ ID NO: 1050; SEQ ID NO: 1051; SEQ ID NO: 791; SEQ ID NO: 1052; SEQ ID NO: 1054; SEQ ID NO: 1055; SEQ ID NO: 1056; SEQ ID NO: 1057; SEQ ID NO: 220; and SEQ ID NO: 1058.


SEQUENCE LISTING

<110>  THE BOARD OF TRUSTEES OF THE LELAND STANFORD JUNIOR UNIVERSITY

<120>  B-1A LYMPHOCYTE AND/OR MACROPHAGE TARGETING AND ACTIVATION TO TREAT
MEDICAL CONDITIONS WITH INFLAMMATORY OR AUTOIMMUNE COMPONENTS

<130>  P59332EP-D1-PCT

<150>  US 62/256,814
<151>  2015-11-18

<160>  1065

<170>  PatentIn version 3.5

<210>  1
<211>  6
<212>  PRT
<213>  Artificial Sequence

<220>
<223>  Synthetic peptide

<400>  1

Ser Val Asn Val Asp Leu
1               5


<210>  2
<211>  6
<212>  PRT
<213>  Artificial Sequence

<220>
<223>  Synthetic peptide

<400>  2

Ser Leu Asn Val Asp Val
1               5


<210>  3
<211>  6
<212>  PRT
<213>  Artificial Sequence

<220>
<223>  Synthetic peptide

<400>  3

Ser Val Asp Val Asn Leu
1               5


<210>  4
<211>  6
<212>  PRT
<213>  Artificial Sequence

<220>
<223>  Synthetic peptide

<400>  4

Asp Leu Ser Val Val Leu
1               5


<210>  5
<211>  6
<212>  PRT
<213>  Artificial Sequence

<220>
<223>  Synthetic peptide

<400>  5

Ser Val Asn Leu Asp Val
1               5


<210>  6
<211>  6
<212>  PRT
<213>  Artificial Sequence

<220>
<223>  Synthetic peptide

<400>  6

Ser Val Val Asn Asp Val
1               5


<210>  7
<211>  6
<212>  PRT
<213>  Artificial Sequence

<220>
<223>  Synthetic peptide

<400>  7

Asp Val Ser Leu Val Asn
1               5


<210>  8
<211>  6
<212>  PRT
<213>  Artificial Sequence

<220>
<223>  Synthetic peptide

<400>  8

Asp Val Ser Val Leu Asn
1               5

<210>  9
<211>  6
<212>  PRT
<213>  Artificial Sequence

<220>
<223>  Synthetic peptide

<400>  9

Ser Asp Leu Val Asn Val
1               5


<210>  10
<211>  6
<212>  PRT
<213>  Artificial Sequence

<220>
<223>  Synthetic peptide

<400>  10

Ser Leu Asn Val Val Ser
1               5


<210>  11
<211>  6
<212>  PRT
<213>  Artificial Sequence

<220>
<223>  Synthetic peptide

<400>  11

Leu Asn Val Asp Ser Val
1               5


<210>  12
<211>  6
<212>  PRT
<213>  Artificial Sequence

<220>
<223>  Synthetic peptide

<400>  12

Asn Asp Leu Ser Val Val
1               5


<210>  13
<211>  6
<212>  PRT
<213>  Artificial Sequence

<220>
<223>  Synthetic peptide

<400>  13

Val Asp Asn Leu Val Ser
1               5



<210>  14
<211>  6
<212>  PRT
<213>  Artificial Sequence

<220>
<223>  Synthetic peptide

<400>  14

Val Asn Asp Val Ser Leu
1               5



<210>  15
<211>  6
<212>  PRT
<213>  Artificial Sequence

<220>
<223>  Synthetic peptide

<400>  15

Val Ser Asp Asn Val Leu
1               5



<210>  16
<211>  6
<212>  PRT
<213>  Artificial Sequence

<220>
<223>  Synthetic peptide

<400>  16

Leu Asn Asp Val Val Ser
1               5



<210>  17
<211>  6
<212>  PRT
<213>  Artificial Sequence

<220>
<223>  Synthetic peptide

<400>  17

Leu Ser Val Asp Val Asn
1               5

<210>  18
<211>  6
<212>  PRT
<213>  Artificial Sequence

<220>
<223>  Synthetic peptide

<400>  18

Asn Ser Val Asp Leu Val
1               5


<210>  19
<211>  6
<212>  PRT
<213>  Artificial Sequence

<220>
<223>  Synthetic peptide

<400>  19

Val Asp Val Leu Asn Ser
1               5


<210>  20
<211>  6
<212>  PRT
<213>  Artificial Sequence

<220>
<223>  Synthetic peptide

<400>  20

Val Asn Asp Ser Val Leu
1               5


<210>  21
<211>  6
<212>  PRT
<213>  Artificial Sequence

<220>
<223>  Synthetic peptide

<400>  21

Val Asn Val Ser Leu Asp
1               5


<210>  22
<211>  6
<212>  PRT
<213>  Artificial Sequence

<220>
<223>  Synthetic peptide

<400>  22

Leu Asp Val Asn Ser Val
1               5


<210>  23
<211>  6
<212>  PRT
<213>  Artificial Sequence

<220>
<223>  Synthetic peptide

<400>  23

Leu Asn Val Val Asp Ser
1               5


<210>  24
<211>  6
<212>  PRT
<213>  Artificial Sequence

<220>
<223>  Synthetic peptide

<400>  24

Leu Ser Asp Val Val Asn
1               5


<210>  25
<211>  6
<212>  PRT
<213>  Artificial Sequence

<220>
<223>  Synthetic peptide

<400>  25

Leu Val Val Asn Asp Ser
1               5


<210>  26
<211>  6
<212>  PRT
<213>  Artificial Sequence

<220>
<223>  Synthetic peptide

<400>  26

Asn Ser Leu Asp Val Val
1               5

<210>  27
<211>  6
<212>  PRT
<213>  Artificial Sequence

<220>
<223>  Synthetic peptide

<400>  27

Val Leu Val Asp Asn Ser
1               5


<210>  28
<211>  6
<212>  PRT
<213>  Artificial Sequence

<220>
<223>  Synthetic peptide

<400>  28

Val Asn Leu Ser Asp Val
1               5


<210>  29
<211>  6
<212>  PRT
<213>  Artificial Sequence

<220>
<223>  Synthetic peptide

<400>  29

Val Asn Val Asp Leu Ser
1               5


<210>  30
<211>  6
<212>  PRT
<213>  Artificial Sequence

<220>
<223>  Synthetic peptide

<400>  30

Asp Asn Val Ser Val Asp
1               5


<210>  31
<211>  6
<212>  PRT
<213>  Artificial Sequence

<220>
<223>  Synthetic peptide

<400>  31

Leu Ser Asp Asn Val Val
1               5

<210>  32
<211>  6
<212>  PRT
<213>  Artificial Sequence

<220>
<223>  Synthetic peptide

<400>  32

Leu Ser Val Val Asp Asn
1               5

<210>  33
<211>  6
<212>  PRT
<213>  Artificial Sequence

<220>
<223>  Synthetic peptide

<400>  33

Val Leu Asp Val Ser Asn
1               5

<210>  34
<211>  6
<212>  PRT
<213>  Artificial Sequence

<220>
<223>  Synthetic peptide

<400>  34

Val Asn Asp Leu Val Ser
1               5

<210>  35
<211>  6
<212>  PRT
<213>  Artificial Sequence

<220>
<223>  Synthetic peptide

<400>  35

Val Asn Val Ser Asp Leu
1               5

<210>  36
<211>  6
<212>  PRT
<213>  Artificial Sequence

<220>
<223>  Synthetic peptide

<400>  36

Val Val Leu Ser Asp Asn
1               5


<210>  37
<211>  6
<212>  PRT
<213>  Artificial Sequence

<220>
<223>  Synthetic peptide

<400>  37

Leu Val Ser Val Asn Leu
1               5


<210>  38
<211>  6
<212>  PRT
<213>  Artificial Sequence

<220>
<223>  Synthetic peptide

<400>  38

Leu Val Asn Val Ser Val
1               5


<210>  39
<211>  6
<212>  PRT
<213>  Artificial Sequence

<220>
<223>  Synthetic peptide

<400>  39

Ser Leu Asn Val Ser Val
1               5


<210>  40
<211>  6
<212>  PRT
<213>  Artificial Sequence

<220>
<223>  Synthetic peptide

<400>  40

Leu Val Ser Val Asn Ser
1               5


<210>  41
<211>  6
<212>  PRT
<213>  Artificial Sequence

<220>
<223>  Synthetic peptide

<400>  41

Ser Val Asp Val Asn Val
1               5


<210>  42
<211>  6
<212>  PRT
<213>  Artificial Sequence

<220>
<223>  Synthetic peptide

<400>  42

Leu Val Val Ser Val Leu
1               5


<210>  43
<211>  6
<212>  PRT
<213>  Artificial Sequence

<220>
<223>  Synthetic peptide

<400>  43

Val Asn Leu Val Val Ser
1               5


<210>  44
<211>  6
<212>  PRT
<213>  Artificial Sequence

<220>
<223>  Synthetic peptide

<400>  44

Ser Asn Leu Val Ser Val
1               5

<210>  45
<211>  6
<212>  PRT
<213>  Artificial Sequence

<220>
<223>  Synthetic peptide

<400>  45

Ser Val Asn Val Leu Ser
1               5


<210>  46
<211>  6
<212>  PRT
<213>  Artificial Sequence

<220>
<223>  Synthetic peptide

<400>  46

Val Leu Val Ser Val Leu
1               5


<210>  47
<211>  6
<212>  PRT
<213>  Artificial Sequence

<220>
<223>  Synthetic peptide

<400>  47

Leu Val Asn Val Ser Leu
1               5


<210>  48
<211>  6
<212>  PRT
<213>  Artificial Sequence

<220>
<223>  Synthetic peptide

<400>  48

Ser Val Asn Val Asp Ser
1               5


<210>  49
<211>  6
<212>  PRT
<213>  Artificial Sequence

<220>
<223>  Synthetic peptide

<400>  49

Val Leu Ser Val Asn Val
1               5


<210>  50
<211>  6
<212>  PRT
<213>  Artificial Sequence

<220>
<223>  Synthetic peptide

<400>  50

Asn Leu Val Val Ser Val
1               5


<210>  51
<211>  6
<212>  PRT
<213>  Artificial Sequence

<220>
<223>  Synthetic peptide

<400>  51

Ser Val Val Leu Asn Val
1               5


<210>  52
<211>  6
<212>  PRT
<213>  Artificial Sequence

<220>
<223>  Synthetic peptide

<400>  52

Leu Ser Val Val Asn Leu
1               5


<210>  53
<211>  6
<212>  PRT
<213>  Artificial Sequence

<220>
<223>  Synthetic peptide

<400>  53

Ser Asn Leu Val Val Ser
1               5

<210>  54
<211>  6
<212>  PRT
<213>  Artificial Sequence

<220>
<223>  Synthetic peptide

<400>  54

Ser Val Val Leu Asp Val
1               5


<210>  55
<211>  6
<212>  PRT
<213>  Artificial Sequence

<220>
<223>  Synthetic peptide

<400>  55

Leu Val Ser Leu Asn Val
1               5


<210>  56
<211>  6
<212>  PRT
<213>  Artificial Sequence

<220>
<223>  Synthetic peptide

<400>  56

Val Ser Leu Asn Val Val
1               5


<210>  57
<211>  6
<212>  PRT
<213>  Artificial Sequence

<220>
<223>  Synthetic peptide

<400>  57

Val Lys Val Gln Ile Tyr
1               5


<210>  58
<211>  6
<212>  PRT
<213>  Artificial Sequence

<220>
<223>  Synthetic peptide

<400>  58

Gln Val Val Ile Tyr Lys
1               5

<210>  59
<211>  6
<212>  PRT
<213>  Artificial Sequence

<220>
<223>  Synthetic peptide

<400>  59

Lys Val Ile Gln Val Tyr
1               5

<210>  60
<211>  6
<212>  PRT
<213>  Artificial Sequence

<220>
<223>  Synthetic peptide

<400>  60

Val Tyr Val Lys Ile Tyr
1               5

<210>  61
<211>  6
<212>  PRT
<213>  Artificial Sequence

<220>
<223>  Synthetic peptide

<400>  61

Gln Ile Val Val Tyr Lys
1               5

<210>  62
<211>  6
<212>  PRT
<213>  Artificial Sequence

<220>
<223>  Synthetic peptide

<400>  62

Gln Val Ile Lys Val Tyr
1               5

<210>  63
<211>  6
<212>  PRT
<213>  Artificial Sequence

<220>
<223>  Synthetic peptide

<400>  63

Val Gln Val Lys Ile Tyr
1               5


<210>  64
<211>  6
<212>  PRT
<213>  Artificial Sequence

<220>
<223>  Synthetic peptide

<400>  64

Gln Val Val Lys Ile Tyr
1               5


<210>  65
<211>  6
<212>  PRT
<213>  Artificial Sequence

<220>
<223>  Synthetic peptide

<400>  65

Gln Val Ile Val Tyr Lys
1               5


<210>  66
<211>  6
<212>  PRT
<213>  Artificial Sequence

<220>
<223>  Synthetic peptide

<400>  66

Gln Ile Val Lys Val Tyr
1               5


<210>  67
<211>  6
<212>  PRT
<213>  Artificial Sequence

<220>
<223>  Synthetic peptide

<400>  67

Gln Lys Ile Val Val Tyr
1               5


<210>  68
<211>  6
<212>  PRT
<213>  Artificial Sequence

<220>
<223>  Synthetic peptide

<400>  68

Gln Lys Val Val Tyr Ile
1               5


<210>  69
<211>  6
<212>  PRT
<213>  Artificial Sequence

<220>
<223>  Synthetic peptide

<400>  69

Lys Val Gln Val Tyr Ile
1               5


<210>  70
<211>  6
<212>  PRT
<213>  Artificial Sequence

<220>
<223>  Synthetic peptide

<400>  70

Gln Val Val Lys Tyr Ile
1               5


<210>  71
<211>  6
<212>  PRT
<213>  Artificial Sequence

<220>
<223>  Synthetic peptide

<400>  71

Lys Val Gln Ile Tyr Val
1               5

<210>  72
<211>  6
<212>  PRT
<213>  Artificial Sequence

<220>
<223>  Synthetic peptide

<400>  72

Val Lys Ile Gln Val Tyr
1               5


<210>  73
<211>  6
<212>  PRT
<213>  Artificial Sequence

<220>
<223>  Synthetic peptide

<400>  73

Val Ile Gln Lys Val Tyr
1               5


<210>  74
<211>  6
<212>  PRT
<213>  Artificial Sequence

<220>
<223>  Synthetic peptide

<400>  74

Lys Val Val Ile Tyr Lys
1               5


<210>  75
<211>  6
<212>  PRT
<213>  Artificial Sequence

<220>
<223>  Synthetic peptide

<400>  75

Gln Ile Val Lys Tyr Val
1               5


<210>  76
<211>  6
<212>  PRT
<213>  Artificial Sequence

<220>
<223>  Synthetic peptide

<400>  76

Gln Lys Val Ile Tyr Val
1               5


<210>  77
<211>  6
<212>  PRT
<213>  Artificial Sequence

<220>
<223>  Synthetic peptide

<400>  77

Lys Gln Val Val Ile Tyr
1               5


<210>  78
<211>  6
<212>  PRT
<213>  Artificial Sequence

<220>
<223>  Synthetic peptide

<400>  78

Lys Ile Gln Val Tyr Val
1               5


<210>  79
<211>  6
<212>  PRT
<213>  Artificial Sequence

<220>
<223>  Synthetic peptide

<400>  79

Lys Val Tyr Val Gln Ile
1               5


<210>  80
<211>  6
<212>  PRT
<213>  Artificial Sequence

<220>
<223>  Synthetic peptide

<400>  80

Val Val Gln Ile Tyr Lys
1               5

<210>  81
<211>  6
<212>  PRT
<213>  Artificial Sequence

<220>
<223>  Synthetic peptide

<400>  81

Lys Gln Val Ile Val Tyr
1               5

<210>  82
<211>  6
<212>  PRT
<213>  Artificial Sequence

<220>
<223>  Synthetic peptide

<400>  82

Val Gln Ile Lys Val Tyr
1               5

<210>  83
<211>  6
<212>  PRT
<213>  Artificial Sequence

<220>
<223>  Synthetic peptide

<400>  83

Gln Lys Ile Val Tyr Val
1               5

<210>  84
<211>  6
<212>  PRT
<213>  Artificial Sequence

<220>
<223>  Synthetic peptide

<400>  84

Val Ile Gln Val Tyr Lys
1               5

<210>  85
<211>  6
<212>  PRT
<213>  Artificial Sequence

<220>
<223>  Synthetic peptide

<400>  85

Lys Val Val Ile Gln Tyr
1               5



<210>  86
<211>  6
<212>  PRT
<213>  Artificial Sequence

<220>
<223>  Synthetic peptide

<400>  86

Lys Val Gln Val Ile Tyr
1               5



<210>  87
<211>  6
<212>  PRT
<213>  Artificial Sequence

<220>
<223>  Synthetic peptide

<400>  87

Gln Tyr Val Val Ile Lys
1               5



<210>  88
<211>  6
<212>  PRT
<213>  Artificial Sequence

<220>
<223>  Synthetic peptide

<400>  88

Tyr Val Val Ile Gln Lys
1               5



<210>  89
<211>  6
<212>  PRT
<213>  Artificial Sequence

<220>
<223>  Synthetic peptide

<400>  89

Lys Ile Val Val Gln Tyr
1               5

<210>  90
<211>  6
<212>  PRT
<213>  Artificial Sequence

<220>
<223>  Synthetic peptide

<400>  90

Tyr Gln Val Ile Val Lys
1               5


<210>  91
<211>  6
<212>  PRT
<213>  Artificial Sequence

<220>
<223>  Synthetic peptide

<400>  91

Lys Ile Tyr Val Gln Val
1               5


<210>  92
<211>  6
<212>  PRT
<213>  Artificial Sequence

<220>
<223>  Synthetic peptide

<400>  92

Lys Val Val Ile Tyr Gln
1               5


<210>  93
<211>  6
<212>  PRT
<213>  Artificial Sequence

<220>
<223>  Synthetic peptide

<400>  93

Lys Tyr Val Val Gln Ile
1               5


<210>  94
<211>  6
<212>  PRT
<213>  Artificial Sequence

<220>
<223>  Synthetic peptide

<400>  94

Tyr Gln Ile Val Val Lys
1               5


<210>  95
<211>  6
<212>  PRT
<213>  Artificial Sequence

<220>
<223>  Synthetic peptide

<400>  95

Tyr Val Val Ile Gln Tyr
1               5


<210>  96
<211>  6
<212>  PRT
<213>  Artificial Sequence

<220>
<223>  Synthetic peptide

<400>  96

Lys Gln Val Ile Tyr Val
1               5


<210>  97
<211>  6
<212>  PRT
<213>  Artificial Sequence

<220>
<223>  Synthetic peptide

<400>  97

Lys Tyr Val Gln Val Ile
1               5


<210>  98
<211>  6
<212>  PRT
<213>  Artificial Sequence

<220>
<223>  Synthetic peptide

<400>  98

Lys Tyr Val Val Ile Gln
1               5

<210>  99
<211>  6
<212>  PRT
<213>  Artificial Sequence

<220>
<223>  Synthetic peptide

<400>  99

Gln Lys Val Val Ile Tyr
1               5


<210>  100
<211>  6
<212>  PRT
<213>  Artificial Sequence

<220>
<223>  Synthetic peptide

<400>  100

Gln Tyr Val Ile Val Lys
1               5


<210>  101
<211>  6
<212>  PRT
<213>  Artificial Sequence

<220>
<223>  Synthetic peptide

<400>  101

Ile Val Gln Lys Val Tyr
1               5


<210>  102
<211>  6
<212>  PRT
<213>  Artificial Sequence

<220>
<223>  Synthetic peptide

<400>  102

Lys Tyr Ile Val Val Gln
1               5


<210>  103
<211>  6
<212>  PRT
<213>  Artificial Sequence

<220>
<223>  Synthetic peptide

<400>  103

Gln Val Ile Lys Tyr Val
1               5


<210>  104
<211>  6
<212>  PRT
<213>  Artificial Sequence

<220>
<223>  Synthetic peptide

<400>  104

Tyr Ile Val Val Gln Lys
1               5


<210>  105
<211>  6
<212>  PRT
<213>  Artificial Sequence

<220>
<223>  Synthetic peptide

<400>  105

Tyr Gln Val Val Ile Lys
1               5


<210>  106
<211>  6
<212>  PRT
<213>  Artificial Sequence

<220>
<223>  Synthetic peptide

<400>  106

Ile Lys Val Gln Val Tyr
1               5


<210>  107
<211>  6
<212>  PRT
<213>  Artificial Sequence

<220>
<223>  Synthetic peptide

<400>  107

Lys Val Val Gln Tyr Ile
1               5

<210>  108
<211>  6
<212>  PRT
<213>  Artificial Sequence

<220>
<223>  Synthetic peptide

<400>  108

Lys Tyr Val Gln Ile Val
1               5


<210>  109
<211>  6
<212>  PRT
<213>  Artificial Sequence

<220>
<223>  Synthetic peptide

<400>  109

Val Lys Gln Val Tyr Ile
1               5


<210>  110
<211>  6
<212>  PRT
<213>  Artificial Sequence

<220>
<223>  Synthetic peptide

<400>  110

Val Gln Val Ile Tyr Lys
1               5


<210>  111
<211>  6
<212>  PRT
<213>  Artificial Sequence

<220>
<223>  Synthetic peptide

<400>  111

Val Val Ile Gln Lys Tyr
1               5


<210>  112
<211>  6
<212>  PRT
<213>  Artificial Sequence

<220>
<223>  Synthetic peptide

<400>  112

Ile Gln Val Val Tyr Lys
1               5


<210>  113
<211>  6
<212>  PRT
<213>  Artificial Sequence

<220>
<223>  Synthetic peptide

<400>  113

Lys Ile Val Val Tyr Gln
1               5


<210>  114
<211>  6
<212>  PRT
<213>  Artificial Sequence

<220>
<223>  Synthetic peptide

<400>  114

Lys Tyr Gln Val Ile Val
1               5


<210>  115
<211>  6
<212>  PRT
<213>  Artificial Sequence

<220>
<223>  Synthetic peptide

<400>  115

Lys Tyr Val Ile Val Gln
1               5


<210>  116
<211>  6
<212>  PRT
<213>  Artificial Sequence

<220>
<223>  Synthetic peptide

<400>  116

Val Val Tyr Ile Gln Lys
1               5

<210>  117
<211>  6
<212>  PRT
<213>  Artificial Sequence

<220>
<223>  Synthetic peptide

<400>  117

Ile Val Gln Val Tyr Lys
1               5

<210>  118
<211>  6
<212>  PRT
<213>  Artificial Sequence

<220>
<223>  Synthetic peptide

<400>  118

Lys Gln Ile Val Tyr Val
1               5

<210>  119
<211>  6
<212>  PRT
<213>  Artificial Sequence

<220>
<223>  Synthetic peptide

<400>  119

Lys Tyr Val Ile Gln Val
1               5

<210>  120
<211>  6
<212>  PRT
<213>  Artificial Sequence

<220>
<223>  Synthetic peptide

<400>  120

Gln Lys Val Ile Val Tyr
1               5

<210>  121
<211>  6
<212>  PRT
<213>  Artificial Sequence

<220>
<223>  Synthetic peptide

<400>  121

Gln Tyr Ile Lys Val Val
1               5

<210>  122
<211>  6
<212>  PRT
<213>  Artificial Sequence

<220>
<223>  Synthetic peptide

<400>  122

Gln Tyr Ile Val Lys Val
1               5

<210>  123
<211>  6
<212>  PRT
<213>  Artificial Sequence

<220>
<223>  Synthetic peptide

<400>  123

Val Gln Ile Val Tyr Lys
1               5

<210>  124
<211>  6
<212>  PRT
<213>  Artificial Sequence

<220>
<223>  Synthetic peptide

<400>  124

Val Val Gln Lys Tyr Ile
1               5

<210>  125
<211>  6
<212>  PRT
<213>  Artificial Sequence

<220>
<223>  Synthetic peptide

<400>  125

Val Tyr Gln Ile Val Lys
1               5

<210>  126
<211>  6
<212>  PRT
<213>  Artificial Sequence

<220>
<223>  Synthetic peptide

<400>  126

Tyr Val Gln Val Ile Lys
1               5


<210>  127
<211>  6
<212>  PRT
<213>  Artificial Sequence

<220>
<223>  Synthetic peptide

<400>  127

Gln Ile Val Tyr Val Lys
1               5


<210>  128
<211>  6
<212>  PRT
<213>  Artificial Sequence

<220>
<223>  Synthetic peptide

<400>  128

Ile Lys Val Tyr Gln Val
1               5


<210>  129
<211>  6
<212>  PRT
<213>  Artificial Sequence

<220>
<223>  Synthetic peptide

<400>  129

Lys Val Val Tyr Ile Gln
1               5


<210>  130
<211>  6
<212>  PRT
<213>  Artificial Sequence

<220>
<223>  Synthetic peptide

<400>  130

Val Gln Lys Tyr Ile Val
1               5


<210>  131
<211>  6
<212>  PRT
<213>  Artificial Sequence

<220>
<223>  Synthetic peptide

<400>  131

Val Gln Lys Tyr Val Ile
1               5


<210>  132
<211>  6
<212>  PRT
<213>  Artificial Sequence

<220>
<223>  Synthetic peptide

<400>  132

Lys Val Val Gln Ile Tyr
1               5


<210>  133
<211>  6
<212>  PRT
<213>  Artificial Sequence

<220>
<223>  Synthetic peptide

<400>  133

Tyr Val Ile Lys Ile Tyr
1               5


<210>  134
<211>  6
<212>  PRT
<213>  Artificial Sequence

<220>
<223>  Synthetic peptide

<400>  134

Gln Val Ile Val Tyr Val
1               5

<210>  135
<211>  6
<212>  PRT
<213>  Artificial Sequence

<220>
<223>  Synthetic peptide

<400>  135

Val Val Ile Lys Val Tyr
1               5


<210>  136
<211>  6
<212>  PRT
<213>  Artificial Sequence

<220>
<223>  Synthetic peptide

<400>  136

Lys Val Val Ile Tyr Ile
1               5


<210>  137
<211>  6
<212>  PRT
<213>  Artificial Sequence

<220>
<223>  Synthetic peptide

<400>  137

Val Tyr Val Val Ile Lys
1               5


<210>  138
<211>  6
<212>  PRT
<213>  Artificial Sequence

<220>
<223>  Synthetic peptide

<400>  138

Tyr Ile Val Gln Val Tyr
1               5


<210>  139
<211>  6
<212>  PRT
<213>  Artificial Sequence

<220>
<223>  Synthetic peptide

<400>  139

Tyr Val Ile Gln Val Tyr
1               5


<210>  140
<211>  6
<212>  PRT
<213>  Artificial Sequence

<220>
<223>  Synthetic peptide

<400>  140

Tyr Val Ile Val Val Tyr
1               5


<210>  141
<211>  6
<212>  PRT
<213>  Artificial Sequence

<220>
<223>  Synthetic peptide

<400>  141

Lys Val Ile Val Tyr Ile
1               5


<210>  142
<211>  6
<212>  PRT
<213>  Artificial Sequence

<220>
<223>  Synthetic peptide

<400>  142

Tyr Val Ile Lys Val Tyr
1               5


<210>  143
<211>  6
<212>  PRT
<213>  Artificial Sequence

<220>
<223>  Synthetic peptide

<400>  143

Val Gln Val Ile Val Lys
1               5

<210>  144
<211>  6
<212>  PRT
<213>  Artificial Sequence

<220>
<223>  Synthetic peptide

<400>  144

Tyr Val Val Lys Ile Tyr
1               5


<210>  145
<211>  6
<212>  PRT
<213>  Artificial Sequence

<220>
<223>  Synthetic peptide

<400>  145

Tyr Val Val Gln Val Ile
1               5


<210>  146
<211>  6
<212>  PRT
<213>  Artificial Sequence

<220>
<223>  Synthetic peptide

<400>  146

Gln Ile Val Val Tyr Gln
1               5


<210>  147
<211>  6
<212>  PRT
<213>  Artificial Sequence

<220>
<223>  Synthetic peptide

<400>  147

Val Val Gln Ile Val Lys
1               5


<210>  148
<211>  6
<212>  PRT
<213>  Artificial Sequence

<220>
<223>  Synthetic peptide

<400>  148

Val Gln Ile Val Val Lys
1               5


<210>  149
<211>  6
<212>  PRT
<213>  Artificial Sequence

<220>
<223>  Synthetic peptide

<400>  149

Val Ile Val Val Tyr Lys
1               5


<210>  150
<211>  6
<212>  PRT
<213>  Artificial Sequence

<220>
<223>  Synthetic peptide

<400>  150

Ile Val Gln Val Tyr Ile
1               5


<210>  151
<211>  6
<212>  PRT
<213>  Artificial Sequence

<220>
<223>  Synthetic peptide

<400>  151

Tyr Val Val Ile Gln Val
1               5


<210>  152
<211>  6
<212>  PRT
<213>  Artificial Sequence

<220>
<223>  Synthetic peptide

<400>  152

Val Tyr Gln Val Val Ile
1               5

<210>  153
<211>  6
<212>  PRT
<213>  Artificial Sequence

<220>
<223>  Synthetic peptide

<400>  153

Lys Tyr Ile Gln Val Tyr
1               5


<210>  154
<211>  6
<212>  PRT
<213>  Artificial Sequence

<220>
<223>  Synthetic peptide

<400>  154

Lys Tyr Val Val Ile Tyr
1               5


<210>  155
<211>  6
<212>  PRT
<213>  Artificial Sequence

<220>
<223>  Synthetic peptide

<400>  155

Lys Tyr Val Gln Ile Tyr
1               5


<210>  156
<211>  6
<212>  PRT
<213>  Artificial Sequence

<220>
<223>  Synthetic peptide

<400>  156

Val Val Gln Val Ile Lys
1               5


<210>  157
<211>  6
<212>  PRT
<213>  Artificial Sequence

<220>
<223>  Synthetic peptide

<400>  157

Gln Lys Ile Val Val Lys
1               5


<210>  158
<211>  6
<212>  PRT
<213>  Artificial Sequence

<220>
<223>  Synthetic peptide

<400>  158

Lys Tyr Val Ile Val Tyr
1               5


<210>  159
<211>  6
<212>  PRT
<213>  Artificial Sequence

<220>
<223>  Synthetic peptide

<400>  159

Lys Gln Val Val Ile Lys
1               5


<210>  160
<211>  6
<212>  PRT
<213>  Artificial Sequence

<220>
<223>  Synthetic peptide

<400>  160

Tyr Val Gln Ile Tyr Val
1               5


<210>  161
<211>  6
<212>  PRT
<213>  Artificial Sequence

<220>
<223>  Synthetic peptide

<400>  161

Tyr Lys Val Gln Val Tyr
1               5

<210>  162
<211>  6
<212>  PRT
<213>  Artificial Sequence

<220>
<223>  Synthetic peptide

<400>  162

Ile Lys Val Gln Ile Tyr
1               5


<210>  163
<211>  6
<212>  PRT
<213>  Artificial Sequence

<220>
<223>  Synthetic peptide

<400>  163

Lys Ile Val Val Gln Lys
1               5


<210>  164
<211>  6
<212>  PRT
<213>  Artificial Sequence

<220>
<223>  Synthetic peptide

<400>  164

Val Ile Lys Val Val Ile
1               5


<210>  165
<211>  6
<212>  PRT
<213>  Artificial Sequence

<220>
<223>  Synthetic peptide

<400>  165

Val Tyr Ile Lys Val Val
1               5


<210>  166
<211>  6
<212>  PRT
<213>  Artificial Sequence

<220>
<223>  Synthetic peptide

<400>  166

Val Tyr Ile Val Gln Val
1               5

<210>  167
<211>  6
<212>  PRT
<213>  Artificial Sequence

<220>
<223>  Synthetic peptide

<400>  167

Ile Val Tyr Val Gln Ile
1               5

<210>  168
<211>  6
<212>  PRT
<213>  Artificial Sequence

<220>
<223>  Synthetic peptide

<400>  168

Lys Val Gln Ile Tyr Lys
1               5

<210>  169
<211>  6
<212>  PRT
<213>  Artificial Sequence

<220>
<223>  Synthetic peptide

<400>  169

Ile Tyr Val Ile Val Tyr
1               5

<210>  170
<211>  6
<212>  PRT
<213>  Artificial Sequence

<220>
<223>  Synthetic peptide

<400>  170

Val Ile Tyr Val Ile Val
1               5

<210>  171
<211>  6
<212>  PRT
<213>  Artificial Sequence

<220>
<223>  Synthetic peptide

<400>  171

Lys Tyr Gln Val Tyr Ile
1               5


<210>  172
<211>  6
<212>  PRT
<213>  Artificial Sequence

<220>
<223>  Synthetic peptide

<400>  172

Val Ile Gln Lys Val Val
1               5


<210>  173
<211>  6
<212>  PRT
<213>  Artificial Sequence

<220>
<223>  Synthetic peptide

<400>  173

Val Lys Ile Val Tyr Val
1               5


<210>  174
<211>  6
<212>  PRT
<213>  Artificial Sequence

<220>
<223>  Synthetic peptide

<400>  174

Val Lys Gln Ile Val Val
1               5


<210>  175
<211>  6
<212>  PRT
<213>  Artificial Sequence

<220>
<223>  Synthetic peptide

<400>  175

Tyr Ile Val Lys Gln Tyr
1               5


<210>  176
<211>  6
<212>  PRT
<213>  Artificial Sequence

<220>
<223>  Synthetic peptide

<400>  176

Tyr Ile Val Val Gln Tyr
1               5


<210>  177
<211>  6
<212>  PRT
<213>  Artificial Sequence

<220>
<223>  Synthetic peptide

<400>  177

Tyr Lys Ile Gln Val Tyr
1               5


<210>  178
<211>  6
<212>  PRT
<213>  Artificial Sequence

<220>
<223>  Synthetic peptide

<400>  178

Tyr Gln Val Val Ile Tyr
1               5


<210>  179
<211>  6
<212>  PRT
<213>  Artificial Sequence

<220>
<223>  Synthetic peptide

<400>  179

Tyr Val Val Lys Gln Tyr
1               5

<210>  180
<211>  6
<212>  PRT
<213>  Artificial Sequence

<220>
<223>  Synthetic peptide

<400>  180

Val Ile Val Lys Val Gln
1               5


<210>  181
<211>  6
<212>  PRT
<213>  Artificial Sequence

<220>
<223>  Synthetic peptide

<400>  181

Gln Lys Val Val Ile Lys
1               5


<210>  182
<211>  6
<212>  PRT
<213>  Artificial Sequence

<220>
<223>  Synthetic peptide

<400>  182

Gly Met Val Val Val Gly
1               5


<210>  183
<211>  6
<212>  PRT
<213>  Artificial Sequence

<220>
<223>  Synthetic peptide

<400>  183

Gly Val Val Val Met Gly
1               5


<210>  184
<211>  6
<212>  PRT
<213>  Artificial Sequence

<220>
<223>  Synthetic peptide

<400>  184

Gly Val Val Met Val Gly
1               5


<210>  185
<211>  6
<212>  PRT
<213>  Artificial Sequence

<220>
<223>  Synthetic peptide

<400>  185

Gly Gly Val Val Val Met
1               5


<210>  186
<211>  6
<212>  PRT
<213>  Artificial Sequence

<220>
<223>  Synthetic peptide

<400>  186

Gly Val Val Gly Val Met
1               5


<210>  187
<211>  6
<212>  PRT
<213>  Artificial Sequence

<220>
<223>  Synthetic peptide

<400>  187

Gly Val Val Val Gly Met
1               5


<210>  188
<211>  6
<212>  PRT
<213>  Artificial Sequence

<220>
<223>  Synthetic peptide

<400>  188

Gly Val Met Val Val Gly
1               5

<210>  189
<211>  6
<212>  PRT
<213>  Artificial Sequence

<220>
<223>  Synthetic peptide

<400>  189

Gly Gly Val Val Met Val
1               5


<210>  190
<211>  6
<212>  PRT
<213>  Artificial Sequence

<220>
<223>  Synthetic peptide

<400>  190

Gly Gly Val Met Val Val
1               5


<210>  191
<211>  6
<212>  PRT
<213>  Artificial Sequence

<220>
<223>  Synthetic peptide

<400>  191

Gly Met Val Val Gly Val
1               5


<210>  192
<211>  6
<212>  PRT
<213>  Artificial Sequence

<220>
<223>  Synthetic peptide

<400>  192

Gly Val Val Met Gly Val
1               5


<210>  193
<211>  6
<212>  PRT
<213>  Artificial Sequence

<220>
<223>  Synthetic peptide

<400>  193

Gly Met Val Gly Val Val
1               5



<210>  194
<211>  6
<212>  PRT
<213>  Artificial Sequence

<220>
<223>  Synthetic peptide

<400>  194

Gly Val Met Val Gly Val
1               5



<210>  195
<211>  6
<212>  PRT
<213>  Artificial Sequence

<220>
<223>  Synthetic peptide

<400>  195

Gly Val Val Gly Met Val
1               5



<210>  196
<211>  6
<212>  PRT
<213>  Artificial Sequence

<220>
<223>  Synthetic peptide

<400>  196

Gly Gly Met Val Val Val
1               5



<210>  197
<211>  6
<212>  PRT
<213>  Artificial Sequence

<220>
<223>  Synthetic peptide

<400>  197

Gly Val Gly Val Met Val
1               5

<210>  198
<211>  6
<212>  PRT
<213>  Artificial Sequence

<220>
<223>  Synthetic peptide

<400>  198

Gly Val Gly Val Val Met
1               5


<210>  199
<211>  6
<212>  PRT
<213>  Artificial Sequence

<220>
<223>  Synthetic peptide

<400>  199

Met Val Val Val Gly Met
1               5


<210>  200
<211>  6
<212>  PRT
<213>  Artificial Sequence

<220>
<223>  Synthetic peptide

<400>  200

Gly Met Gly Val Val Val
1               5


<210>  201
<211>  6
<212>  PRT
<213>  Artificial Sequence

<220>
<223>  Synthetic peptide

<400>  201

Met Val Val Val Gly Gly
1               5


<210>  202
<211>  6
<212>  PRT
<213>  Artificial Sequence

<220>
<223>  Synthetic peptide

<400>  202

Gly Val Gly Met Val Val
1               5



<210>  203
<211>  6
<212>  PRT
<213>  Artificial Sequence

<220>
<223>  Synthetic peptide

<400>  203

Gly Val Val Gly Gly Val
1               5



<210>  204
<211>  6
<212>  PRT
<213>  Artificial Sequence

<220>
<223>  Synthetic peptide

<400>  204

Met Val Gly Val Val Gly
1               5



<210>  205
<211>  6
<212>  PRT
<213>  Artificial Sequence

<220>
<223>  Synthetic peptide

<400>  205

Val Val Met Val Gly Gly
1               5



<210>  206
<211>  6
<212>  PRT
<213>  Artificial Sequence

<220>
<223>  Synthetic peptide

<400>  206

Met Gly Val Val Val Gly
1               5

<210>  207
<211>  6
<212>  PRT
<213>  Artificial Sequence

<220>
<223>  Synthetic peptide

<400>  207

Met Val Gly Val Gly Val
1               5


<210>  208
<211>  6
<212>  PRT
<213>  Artificial Sequence

<220>
<223>  Synthetic peptide

<400>  208

Val Gly Val Met Val Gly
1               5


<210>  209
<211>  6
<212>  PRT
<213>  Artificial Sequence

<220>
<223>  Synthetic peptide

<400>  209

Met Val Val Gly Val Gly
1               5


<210>  210
<211>  6
<212>  PRT
<213>  Artificial Sequence

<220>
<223>  Synthetic peptide

<400>  210

Val Val Gly Val Met Gly
1               5


<210>  211
<211>  6
<212>  PRT
<213>  Artificial Sequence

<220>
<223>  Synthetic peptide

<400>  211

Val Met Gly Val Val Gly
1               5

<210>  212
<211>  6
<212>  PRT
<213>  Artificial Sequence

<220>
<223>  Synthetic peptide

<400>  212

Val Gly Val Gly Val Met
1               5

<210>  213
<211>  6
<212>  PRT
<213>  Artificial Sequence

<220>
<223>  Synthetic peptide

<400>  213

Val Met Val Gly Val Gly
1               5

<210>  214
<211>  6
<212>  PRT
<213>  Artificial Sequence

<220>
<223>  Synthetic peptide

<400>  214

Met Gly Gly Val Val Val
1               5

<210>  215
<211>  6
<212>  PRT
<213>  Artificial Sequence

<220>
<223>  Synthetic peptide

<400>  215

Val Gly Met Val Val Gly
1               5

<210>  216
<211>  6
<212>  PRT
<213>  Artificial Sequence

<220>
<223>  Synthetic peptide

<400>  216

Val Val Gly Val Gly Met
1               5


<210>  217
<211>  6
<212>  PRT
<213>  Artificial Sequence

<220>
<223>  Synthetic peptide

<400>  217

Val Gly Gly Val Val Met
1               5


<210>  218
<211>  6
<212>  PRT
<213>  Artificial Sequence

<220>
<223>  Synthetic peptide

<400>  218

Val Val Gly Met Val Gly
1               5


<210>  219
<211>  6
<212>  PRT
<213>  Artificial Sequence

<220>
<223>  Synthetic peptide

<400>  219

Val Gly Val Val Met Gly
1               5


<210>  220
<211>  6
<212>  PRT
<213>  Artificial Sequence

<220>
<223>  Synthetic peptide

<400>  220

Met Val Gly Gly Val Val
1               5


<210>  221
<211>  6
<212>  PRT
<213>  Artificial Sequence

<220>
<223>  Synthetic peptide

<400>  221

Val Gly Met Val Gly Val
1               5


<210>  222
<211>  6
<212>  PRT
<213>  Artificial Sequence

<220>
<223>  Synthetic peptide

<400>  222

Val Met Val Val Gly Gly
1               5


<210>  223
<211>  6
<212>  PRT
<213>  Artificial Sequence

<220>
<223>  Synthetic peptide

<400>  223

Val Val Gly Gly Val Met
1               5


<210>  224
<211>  6
<212>  PRT
<213>  Artificial Sequence

<220>
<223>  Synthetic peptide

<400>  224

Val Gly Gly Val Met Val
1               5

<210>  225
<211>  6
<212>  PRT
<213>  Artificial Sequence

<220>
<223>  Synthetic peptide

<400>  225

Val Val Val Gly Met Gly
1               5


<210>  226
<211>  6
<212>  PRT
<213>  Artificial Sequence

<220>
<223>  Synthetic peptide

<400>  226

Val Val Gly Met Gly Val
1               5


<210>  227
<211>  6
<212>  PRT
<213>  Artificial Sequence

<220>
<223>  Synthetic peptide

<400>  227

Met Gly Val Gly Val Val
1               5


<210>  228
<211>  6
<212>  PRT
<213>  Artificial Sequence

<220>
<223>  Synthetic peptide

<400>  228

Val Gly Val Val Gly Met
1               5


<210>  229
<211>  6
<212>  PRT
<213>  Artificial Sequence

<220>
<223>  Synthetic peptide

<400>  229

Val Gly Gly Met Val Val
1               5

<210>  230
<211>  6
<212>  PRT
<213>  Artificial Sequence

<220>
<223>  Synthetic peptide

<400>  230

Val Val Val Met Gly Gly
1               5

<210>  231
<211>  6
<212>  PRT
<213>  Artificial Sequence

<220>
<223>  Synthetic peptide

<400>  231

Val Val Met Gly Gly Val
1               5

<210>  232
<211>  6
<212>  PRT
<213>  Artificial Sequence

<220>
<223>  Synthetic peptide

<400>  232

Val Val Val Gly Gly Met
1               5

<210>  233
<211>  6
<212>  PRT
<213>  Artificial Sequence

<220>
<223>  Synthetic peptide

<400>  233

Val Val Gly Gly Met Val
1               5

<210>  234
<211>  6
<212>  PRT
<213>  Artificial Sequence

<220>
<223>  Synthetic peptide

<400>  234

Val Gly Met Gly Val Val
1               5


<210>  235
<211>  6
<212>  PRT
<213>  Artificial Sequence

<220>
<223>  Synthetic peptide

<400>  235

Val Met Gly Gly Val Val
1               5


<210>  236
<211>  6
<212>  PRT
<213>  Artificial Sequence

<220>
<223>  Synthetic peptide

<400>  236

Val Gly Gly Val Gly Val
1               5


<210>  237
<211>  6
<212>  PRT
<213>  Artificial Sequence

<220>
<223>  Synthetic peptide

<400>  237

Val Gly Val Met Gly Val
1               5


<210>  238
<211>  6
<212>  PRT
<213>  Artificial Sequence

<220>
<223>  Synthetic peptide

<400>  238

Val Gly Val Gly Met Val
1               5


<210>  239
<211>  6
<212>  PRT
<213>  Artificial Sequence

<220>
<223>  Synthetic peptide

<400>  239

Val Met Val Gly Gly Val
1               5


<210>  240
<211>  6
<212>  PRT
<213>  Artificial Sequence

<220>
<223>  Synthetic peptide

<400>  240

Ala Asn Ser Thr Ser Val
1               5


<210>  241
<211>  6
<212>  PRT
<213>  Artificial Sequence

<220>
<223>  Synthetic peptide

<400>  241

Ala Asn Ser Val Ser Gly
1               5


<210>  242
<211>  6
<212>  PRT
<213>  Artificial Sequence

<220>
<223>  Synthetic peptide

<400>  242

Ala Asn Ser Val Ser Ser
1               5

<210>  243
<211>  6
<212>  PRT
<213>  Artificial Sequence

<220>
<223>  Synthetic peptide

<400>  243

Ala Gln Asn Ser Asn Val
1               5


<210>  244
<211>  6
<212>  PRT
<213>  Artificial Sequence

<220>
<223>  Synthetic peptide

<400>  244

Ala Gln Asn Val Asn Ser
1               5


<210>  245
<211>  6
<212>  PRT
<213>  Artificial Sequence

<220>
<223>  Synthetic peptide

<400>  245

Ala Gln Asn Val Thr Ser
1               5


<210>  246
<211>  6
<212>  PRT
<213>  Artificial Sequence

<220>
<223>  Synthetic peptide

<400>  246

Ala Gln Ser Gln Ser Val
1               5


<210>  247
<211>  6
<212>  PRT
<213>  Artificial Sequence

<220>
<223>  Synthetic peptide

<400>  247

Ala Gln Ser Ser Ser Val
1               5


<210>  248
<211>  6
<212>  PRT
<213>  Artificial Sequence

<220>
<223>  Synthetic peptide

<400>  248

Ala Gln Ser Thr Ser Val
1               5


<210>  249
<211>  6
<212>  PRT
<213>  Artificial Sequence

<220>
<223>  Synthetic peptide

<400>  249

Ala Gln Ser Val Asn Ser
1               5


<210>  250
<211>  6
<212>  PRT
<213>  Artificial Sequence

<220>
<223>  Synthetic peptide

<400>  250

Ala Gln Ser Val Gln Ser
1               5


<210>  251
<211>  6
<212>  PRT
<213>  Artificial Sequence

<220>
<223>  Synthetic peptide

<400>  251

Ala Gln Ser Val Ser Gln
1               5

<210>  252
<211>  6
<212>  PRT
<213>  Artificial Sequence

<220>
<223>  Synthetic peptide

<400>  252

Ala Gln Ser Val Ser Ser
1               5


<210>  253
<211>  6
<212>  PRT
<213>  Artificial Sequence

<220>
<223>  Synthetic peptide

<400>  253

Ala Gln Ser Val Ser Thr
1               5


<210>  254
<211>  6
<212>  PRT
<213>  Artificial Sequence

<220>
<223>  Synthetic peptide

<400>  254

Ala Ser Asn Asn Asn Val
1               5


<210>  255
<211>  6
<212>  PRT
<213>  Artificial Sequence

<220>
<223>  Synthetic peptide

<400>  255

Ala Ser Asn Gln Asn Gln
1               5


<210>  256
<211>  6
<212>  PRT
<213>  Artificial Sequence

<220>
<223>  Synthetic peptide

<400>  256

Ala Ser Asn Gln Asn Val
1               5


<210>  257
<211>  6
<212>  PRT
<213>  Artificial Sequence

<220>
<223>  Synthetic peptide

<400>  257

Ala Ser Asn Gln Thr Gln
1               5


<210>  258
<211>  6
<212>  PRT
<213>  Artificial Sequence

<220>
<223>  Synthetic peptide

<400>  258

Ala Ser Asn Ser Asn Val
1               5


<210>  259
<211>  6
<212>  PRT
<213>  Artificial Sequence

<220>
<223>  Synthetic peptide

<400>  259

Ala Ser Asn Ser Thr Val
1               5


<210>  260
<211>  6
<212>  PRT
<213>  Artificial Sequence

<220>
<223>  Synthetic peptide

<400>  260

Ala Ser Asn Thr Asn Ser
1               5

<210>  261
<211>  6
<212>  PRT
<213>  Artificial Sequence

<220>
<223>  Synthetic peptide

<400>  261

Ala Ser Asn Thr Asn Val
1               5


<210>  262
<211>  6
<212>  PRT
<213>  Artificial Sequence

<220>
<223>  Synthetic peptide

<400>  262

Ala Ser Asn Thr Ser Val
1               5


<210>  263
<211>  6
<212>  PRT
<213>  Artificial Sequence

<220>
<223>  Synthetic peptide

<400>  263

Ala Ser Asn Val Asn Gly
1               5


<210>  264
<211>  6
<212>  PRT
<213>  Artificial Sequence

<220>
<223>  Synthetic peptide

<400>  264

Ala Ser Asn Val Asn Gln
1               5


<210>  265
<211>  6
<212>  PRT
<213>  Artificial Sequence

<220>
<223>  Synthetic peptide

<400>  265

Ala Ser Asn Val Asn Ser
1               5


<210>  266
<211>  6
<212>  PRT
<213>  Artificial Sequence

<220>
<223>  Synthetic peptide

<400>  266

Ala Ser Asn Val Asn Thr
1               5


<210>  267
<211>  6
<212>  PRT
<213>  Artificial Sequence

<220>
<223>  Synthetic peptide

<400>  267

Ala Ser Asn Val Thr Gly
1               5


<210>  268
<211>  6
<212>  PRT
<213>  Artificial Sequence

<220>
<223>  Synthetic peptide

<400>  268

Ala Ser Asn Val Thr Thr
1               5


<210>  269
<211>  6
<212>  PRT
<213>  Artificial Sequence

<220>
<223>  Synthetic peptide

<400>  269

Ala Ser Ser Asn Ser Val
1               5

<210>  270
<211>  6
<212>  PRT
<213>  Artificial Sequence

<220>
<223>  Synthetic peptide

<400>  270

Ala Ser Ser Val Ser Gly
1               5


<210>  271
<211>  6
<212>  PRT
<213>  Artificial Sequence

<220>
<223>  Synthetic peptide

<400>  271

Ala Ser Ser Val Ser Asn
1               5


<210>  272
<211>  6
<212>  PRT
<213>  Artificial Sequence

<220>
<223>  Synthetic peptide

<400>  272

Ala Thr Asn Val Asn Ser
1               5


<210>  273
<211>  6
<212>  PRT
<213>  Artificial Sequence

<220>
<223>  Synthetic peptide

<400>  273

Ala Thr Asn Val Thr Ser
1               5


<210>  274
<211>  6
<212>  PRT
<213>  Artificial Sequence

<220>
<223>  Synthetic peptide

<400>  274

Ala Thr Ser Gln Ser Gln
1               5


<210>  275
<211>  6
<212>  PRT
<213>  Artificial Sequence

<220>
<223>  Synthetic peptide

<400>  275

Ala Thr Ser Gln Ser Val
1               5


<210>  276
<211>  6
<212>  PRT
<213>  Artificial Sequence

<220>
<223>  Synthetic peptide

<400>  276

Ala Thr Ser Thr Ser Gly
1               5


<210>  277
<211>  6
<212>  PRT
<213>  Artificial Sequence

<220>
<223>  Synthetic peptide

<400>  277

Ala Thr Ser Thr Ser Val
1               5


<210>  278
<211>  6
<212>  PRT
<213>  Artificial Sequence

<220>
<223>  Synthetic peptide

<400>  278

Ala Thr Ser Val Ser Gly
1               5

<210>  279
<211>  6
<212>  PRT
<213>  Artificial Sequence

<220>
<223>  Synthetic peptide

<400>  279

Ala Thr Ser Val Ser Ser
1               5


<210>  280
<211>  6
<212>  PRT
<213>  Artificial Sequence

<220>
<223>  Synthetic peptide

<400>  280

Ala Val Asn Gln Asn Ser
1               5


<210>  281
<211>  6
<212>  PRT
<213>  Artificial Sequence

<220>
<223>  Synthetic peptide

<400>  281

Ala Val Asn Gln Ser Gln
1               5


<210>  282
<211>  6
<212>  PRT
<213>  Artificial Sequence

<220>
<223>  Synthetic peptide

<400>  282

Ala Val Asn Ser Asn Gly
1               5


<210>  283
<211>  6
<212>  PRT
<213>  Artificial Sequence

<220>
<223>  Synthetic peptide

<400>  283

Ala Val Asn Ser Asn Ser
1               5


<210>  284
<211>  6
<212>  PRT
<213>  Artificial Sequence

<220>
<223>  Synthetic peptide

<400>  284

Ala Val Asn Ser Asn Thr
1               5


<210>  285
<211>  6
<212>  PRT
<213>  Artificial Sequence

<220>
<223>  Synthetic peptide

<400>  285

Ala Val Asn Thr Asn Ser
1               5


<210>  286
<211>  6
<212>  PRT
<213>  Artificial Sequence

<220>
<223>  Synthetic peptide

<400>  286

Ala Val Ser Asn Ser Gly
1               5


<210>  287
<211>  6
<212>  PRT
<213>  Artificial Sequence

<220>
<223>  Synthetic peptide

<400>  287

Ala Val Ser Asn Ser Ser
1               5

<210>  288
<211>  6
<212>  PRT
<213>  Artificial Sequence

<220>
<223>  Synthetic peptide

<400>  288

Ala Val Ser Gln Asn Gln
1               5


<210>  289
<211>  6
<212>  PRT
<213>  Artificial Sequence

<220>
<223>  Synthetic peptide

<400>  289

Ala Val Ser Gln Ser Gly
1               5


<210>  290
<211>  6
<212>  PRT
<213>  Artificial Sequence

<220>
<223>  Synthetic peptide

<400>  290

Ala Val Ser Gln Ser Gln
1               5


<210>  291
<211>  6
<212>  PRT
<213>  Artificial Sequence

<220>
<223>  Synthetic peptide

<400>  291

Ala Val Ser Gln Thr Gln
1               5


<210>  292
<211>  6
<212>  PRT
<213>  Artificial Sequence

<220>
<223>  Synthetic peptide

<400>  292

Ala Val Ser Ser Asn Gln
1               5


<210>  293
<211>  6
<212>  PRT
<213>  Artificial Sequence

<220>
<223>  Synthetic peptide

<400>  293

Ala Val Ser Ser Asn Ser
1               5


<210>  294
<211>  6
<212>  PRT
<213>  Artificial Sequence

<220>
<223>  Synthetic peptide

<400>  294

Ala Val Ser Ser Ser Gln
1               5


<210>  295
<211>  6
<212>  PRT
<213>  Artificial Sequence

<220>
<223>  Synthetic peptide

<400>  295

Ala Val Ser Thr Ser Gly
1               5


<210>  296
<211>  6
<212>  PRT
<213>  Artificial Sequence

<220>
<223>  Synthetic peptide

<400>  296

Gly Ala Asn Thr Val Ser
1               5

<210>  297
<211>  6
<212>  PRT
<213>  Artificial Sequence

<220>
<223>  Synthetic peptide

<400>  297

Gly Ala Gln Thr Ser Ser
1               5


<210>  298
<211>  6
<212>  PRT
<213>  Artificial Sequence

<220>
<223>  Synthetic peptide

<400>  298

Gly Ala Ser Asn Gln Ser
1               5


<210>  299
<211>  6
<212>  PRT
<213>  Artificial Sequence

<220>
<223>  Synthetic peptide

<400>  299

Gly Ala Ser Gln Gln Ser
1               5


<210>  300
<211>  6
<212>  PRT
<213>  Artificial Sequence

<220>
<223>  Synthetic peptide

<400>  300

Gly Ala Ser Ser Gln Gln
1               5


<210>  301
<211>  6
<212>  PRT
<213>  Artificial Sequence

<220>
<223>  Synthetic peptide

<400>  301

Gly Gly Gln Val Thr Ser
1               5


<210>  302
<211>  6
<212>  PRT
<213>  Artificial Sequence

<220>
<223>  Synthetic peptide

<400>  302

Gly Gly Ser Asn Gln Val
1               5


<210>  303
<211>  6
<212>  PRT
<213>  Artificial Sequence

<220>
<223>  Synthetic peptide

<400>  303

Gly Asn Asn Val Gln Ser
1               5


<210>  304
<211>  6
<212>  PRT
<213>  Artificial Sequence

<220>
<223>  Synthetic peptide

<400>  304

Gly Asn Gln Val Thr Ser
1               5


<210>  305
<211>  6
<212>  PRT
<213>  Artificial Sequence

<220>
<223>  Synthetic peptide

<400>  305

Gly Asn Ser Asn Gln Val
1               5

<210>  306
<211>  6
<212>  PRT
<213>  Artificial Sequence

<220>
<223>  Synthetic peptide

<400>  306

Gly Asn Ser Gln Gln Gln
1               5


<210>  307
<211>  6
<212>  PRT
<213>  Artificial Sequence

<220>
<223>  Synthetic peptide

<400>  307

Gly Asn Ser Gln Gln Ser
1               5


<210>  308
<211>  6
<212>  PRT
<213>  Artificial Sequence

<220>
<223>  Synthetic peptide

<400>  308

Gly Asn Ser Gln Gln Val
1               5


<210>  309
<211>  6
<212>  PRT
<213>  Artificial Sequence

<220>
<223>  Synthetic peptide

<400>  309

Gly Asn Ser Ser Thr Val
1               5


<210>  310
<211>  6
<212>  PRT
<213>  Artificial Sequence

<220>
<223>  Synthetic peptide

<400>  310

Gly Asn Ser Thr Gln Ser
1               5


<210>  311
<211>  6
<212>  PRT
<213>  Artificial Sequence

<220>
<223>  Synthetic peptide

<400>  311

Gly Asn Ser Thr Gln Val
1               5


<210>  312
<211>  6
<212>  PRT
<213>  Artificial Sequence

<220>
<223>  Synthetic peptide

<400>  312

Gly Asn Ser Thr Val Ser
1               5


<210>  313
<211>  6
<212>  PRT
<213>  Artificial Sequence

<220>
<223>  Synthetic peptide

<400>  313

Gly Asn Ser Val Gln Ser
1               5


<210>  314
<211>  6
<212>  PRT
<213>  Artificial Sequence

<220>
<223>  Synthetic peptide

<400>  314

Gly Asn Ser Val Ser Ser
1               5

<210>  315
<211>  6
<212>  PRT
<213>  Artificial Sequence

<220>
<223>  Synthetic peptide

<400>  315

Gly Asn Ser Val Ser Thr
1               5


<210>  316
<211>  6
<212>  PRT
<213>  Artificial Sequence

<220>
<223>  Synthetic peptide

<400>  316

Gly Asn Ser Val Thr Ser
1               5


<210>  317
<211>  6
<212>  PRT
<213>  Artificial Sequence

<220>
<223>  Synthetic peptide

<400>  317

Gly Gln Asn Thr Val Ser
1               5


<210>  318
<211>  6
<212>  PRT
<213>  Artificial Sequence

<220>
<223>  Synthetic peptide

<400>  318

Gly Gln Asn Val Ala Ser
1               5


<210>  319
<211>  6
<212>  PRT
<213>  Artificial Sequence

<220>
<223>  Synthetic peptide

<400>  319

Gly Gln Asn Val Gln Ser
1               5


<210>  320
<211>  6
<212>  PRT
<213>  Artificial Sequence

<220>
<223>  Synthetic peptide

<400>  320

Gly Gln Asn Val Ser Ser
1               5


<210>  321
<211>  6
<212>  PRT
<213>  Artificial Sequence

<220>
<223>  Synthetic peptide

<400>  321

Gly Gln Asn Val Thr Ser
1               5


<210>  322
<211>  6
<212>  PRT
<213>  Artificial Sequence

<220>
<223>  Synthetic peptide

<400>  322

Gly Gln Gln Gln Ser Gln
1               5


<210>  323
<211>  6
<212>  PRT
<213>  Artificial Sequence

<220>
<223>  Synthetic peptide

<400>  323

Gly Gln Gln Thr Ser Ser
1               5

<210>  324
<211>  6
<212>  PRT
<213>  Artificial Sequence

<220>
<223>  Synthetic peptide

<400>  324

Gly Gln Gln Thr Ser Val
1               5


<210>  325
<211>  6
<212>  PRT
<213>  Artificial Sequence

<220>
<223>  Synthetic peptide

<400>  325

Gly Gln Gln Val Ala Ser
1               5


<210>  326
<211>  6
<212>  PRT
<213>  Artificial Sequence

<220>
<223>  Synthetic peptide

<400>  326

Gly Gln Gln Val Asn Ser
1               5


<210>  327
<211>  6
<212>  PRT
<213>  Artificial Sequence

<220>
<223>  Synthetic peptide

<400>  327

Gly Gln Gln Val Gln Ser
1               5


<210>  328
<211>  6
<212>  PRT
<213>  Artificial Sequence

<220>
<223>  Synthetic peptide

<400>  328

Gly Gln Gln Val Ser Gly
1               5


<210>  329
<211>  6
<212>  PRT
<213>  Artificial Sequence

<220>
<223>  Synthetic peptide

<400>  329

Gly Gln Gln Val Ser Gln
1               5


<210>  330
<211>  6
<212>  PRT
<213>  Artificial Sequence

<220>
<223>  Synthetic peptide

<400>  330

Gly Gln Gln Val Ser Ser
1               5


<210>  331
<211>  6
<212>  PRT
<213>  Artificial Sequence

<220>
<223>  Synthetic peptide

<400>  331

Gly Gln Gln Val Ser Thr
1               5


<210>  332
<211>  6
<212>  PRT
<213>  Artificial Sequence

<220>
<223>  Synthetic peptide

<400>  332

Gly Gln Gln Val Thr Ser
1               5

<210>  333
<211>  6
<212>  PRT
<213>  Artificial Sequence

<220>
<223>  Synthetic peptide

<400>  333

Gly Gln Ser Gly Gln Val
1               5


<210>  334
<211>  6
<212>  PRT
<213>  Artificial Sequence

<220>
<223>  Synthetic peptide

<400>  334

Gly Gln Ser Asn Gln Ala
1               5


<210>  335
<211>  6
<212>  PRT
<213>  Artificial Sequence

<220>
<223>  Synthetic peptide

<400>  335

Gly Gln Ser Asn Gln Ser
1               5


<210>  336
<211>  6
<212>  PRT
<213>  Artificial Sequence

<220>
<223>  Synthetic peptide

<400>  336

Gly Gln Ser Asn Gln Val
1               5


<210>  337
<211>  6
<212>  PRT
<213>  Artificial Sequence

<220>
<223>  Synthetic peptide

<400>  337

Gly Gln Ser Gln Ala Gln
1               5


<210>  338
<211>  6
<212>  PRT
<213>  Artificial Sequence

<220>
<223>  Synthetic peptide

<400>  338

Gly Gln Ser Gln Gln Gln
1               5


<210>  339
<211>  6
<212>  PRT
<213>  Artificial Sequence

<220>
<223>  Synthetic peptide

<400>  339

Gly Gln Ser Gln Gln Ser
1               5


<210>  340
<211>  6
<212>  PRT
<213>  Artificial Sequence

<220>
<223>  Synthetic peptide

<400>  340

Gly Gln Ser Gln Ser Gln
1               5


<210>  341
<211>  6
<212>  PRT
<213>  Artificial Sequence

<220>
<223>  Synthetic peptide

<400>  341

Gly Gln Ser Ser Gln Gln
1               5

<210>  342
<211>  6
<212>  PRT
<213>  Artificial Sequence

<220>
<223>  Synthetic peptide

<400>  342

Gly Gln Ser Ser Gln Ser
1               5


<210>  343
<211>  6
<212>  PRT
<213>  Artificial Sequence

<220>
<223>  Synthetic peptide

<400>  343

Gly Gln Ser Ser Gln Val
1               5


<210>  344
<211>  6
<212>  PRT
<213>  Artificial Sequence

<220>
<223>  Synthetic peptide

<400>  344

Gly Gln Ser Thr Gln Ser
1               5


<210>  345
<211>  6
<212>  PRT
<213>  Artificial Sequence

<220>
<223>  Synthetic peptide

<400>  345

Gly Gln Ser Thr Gln Val
1               5


<210>  346
<211>  6
<212>  PRT
<213>  Artificial Sequence

<220>
<223>  Synthetic peptide

<400>  346

Gly Gln Ser Val Ala Gly
1               5


<210>  347
<211>  6
<212>  PRT
<213>  Artificial Sequence

<220>
<223>  Synthetic peptide

<400>  347

Gly Gln Ser Val Ala Gln
1               5


<210>  348
<211>  6
<212>  PRT
<213>  Artificial Sequence

<220>
<223>  Synthetic peptide

<400>  348

Gly Gln Ser Val Ala Ser
1               5


<210>  349
<211>  6
<212>  PRT
<213>  Artificial Sequence

<220>
<223>  Synthetic peptide

<400>  349

Gly Gln Ser Val Gln Asn
1               5


<210>  350
<211>  6
<212>  PRT
<213>  Artificial Sequence

<220>
<223>  Synthetic peptide

<400>  350

Gly Gln Ser Val Gln Gln
1               5

<210>  351
<211>  6
<212>  PRT
<213>  Artificial Sequence

<220>
<223>  Synthetic peptide

<400>  351

Gly Gln Ser Val Gln Ser
1               5


<210>  352
<211>  6
<212>  PRT
<213>  Artificial Sequence

<220>
<223>  Synthetic peptide

<400>  352

Gly Gln Ser Val Ser Gly
1               5


<210>  353
<211>  6
<212>  PRT
<213>  Artificial Sequence

<220>
<223>  Synthetic peptide

<400>  353

Gly Gln Ser Val Ser Asn
1               5


<210>  354
<211>  6
<212>  PRT
<213>  Artificial Sequence

<220>
<223>  Synthetic peptide

<400>  354

Gly Gln Ser Val Ser Gln
1               5


<210>  355
<211>  6
<212>  PRT
<213>  Artificial Sequence

<220>
<223>  Synthetic peptide

<400>  355

Gly Gln Ser Val Ser Ser
1               5


<210>  356
<211>  6
<212>  PRT
<213>  Artificial Sequence

<220>
<223>  Synthetic peptide

<400>  356

Gly Gln Ser Val Ser Thr
1               5


<210>  357
<211>  6
<212>  PRT
<213>  Artificial Sequence

<220>
<223>  Synthetic peptide

<400>  357

Gly Gln Ser Val Thr Ser
1               5


<210>  358
<211>  6
<212>  PRT
<213>  Artificial Sequence

<220>
<223>  Synthetic peptide

<400>  358

Gly Ser Asn Gln Gln Val
1               5


<210>  359
<211>  6
<212>  PRT
<213>  Artificial Sequence

<220>
<223>  Synthetic peptide

<400>  359

Gly Ser Asn Gln Val Gln
1               5

<210>  360
<211>  6
<212>  PRT
<213>  Artificial Sequence

<220>
<223>  Synthetic peptide

<400>  360

Gly Ser Asn Ser Thr Val
1               5


<210>  361
<211>  6
<212>  PRT
<213>  Artificial Sequence

<220>
<223>  Synthetic peptide

<400>  361

Gly Ser Asn Ser Val Gln
1               5


<210>  362
<211>  6
<212>  PRT
<213>  Artificial Sequence

<220>
<223>  Synthetic peptide

<400>  362

Gly Ser Asn Ser Val Thr
1               5


<210>  363
<211>  6
<212>  PRT
<213>  Artificial Sequence

<220>
<223>  Synthetic peptide

<400>  363

Gly Ser Asn Thr Ala Val
1               5


<210>  364
<211>  6
<212>  PRT
<213>  Artificial Sequence

<220>
<223>  Synthetic peptide

<400>  364

Gly Ser Asn Thr Gln Val
1               5


<210>  365
<211>  6
<212>  PRT
<213>  Artificial Sequence

<220>
<223>  Synthetic peptide

<400>  365

Gly Ser Asn Thr Val Ala
1               5


<210>  366
<211>  6
<212>  PRT
<213>  Artificial Sequence

<220>
<223>  Synthetic peptide

<400>  366

Gly Ser Asn Thr Val Ser
1               5


<210>  367
<211>  6
<212>  PRT
<213>  Artificial Sequence

<220>
<223>  Synthetic peptide

<400>  367

Gly Ser Asn Val Ala Ser
1               5


<210>  368
<211>  6
<212>  PRT
<213>  Artificial Sequence

<220>
<223>  Synthetic peptide

<400>  368

Gly Ser Asn Val Gln Gln
1               5

<210>  369
<211>  6
<212>  PRT
<213>  Artificial Sequence

<220>
<223>  Synthetic peptide

<400>  369

Gly Ser Asn Val Gln Ser
1               5


<210>  370
<211>  6
<212>  PRT
<213>  Artificial Sequence

<220>
<223>  Synthetic peptide

<400>  370

Gly Ser Asn Val Gln Thr
1               5


<210>  371
<211>  6
<212>  PRT
<213>  Artificial Sequence

<220>
<223>  Synthetic peptide

<400>  371

Gly Ser Asn Val Thr Ser
1               5


<210>  372
<211>  6
<212>  PRT
<213>  Artificial Sequence

<220>
<223>  Synthetic peptide

<400>  372

Gly Ser Gln Gln Ser Val
1               5


<210>  373
<211>  6
<212>  PRT
<213>  Artificial Sequence

<220>
<223>  Synthetic peptide

<400>  373

Gly Ser Gln Gln Thr Gln
1               5


<210>  374
<211>  6
<212>  PRT
<213>  Artificial Sequence

<220>
<223>  Synthetic peptide

<400>  374

Gly Ser Gln Gln Thr Val
1               5


<210>  375
<211>  6
<212>  PRT
<213>  Artificial Sequence

<220>
<223>  Synthetic peptide

<400>  375

Gly Ser Gln Thr Ser Ser
1               5


<210>  376
<211>  6
<212>  PRT
<213>  Artificial Sequence

<220>
<223>  Synthetic peptide

<400>  376

Gly Ser Gln Thr Ser Val
1               5


<210>  377
<211>  6
<212>  PRT
<213>  Artificial Sequence

<220>
<223>  Synthetic peptide

<400>  377

Gly Ser Gln Val Ala Ser
1               5

<210>  378
<211>  6
<212>  PRT
<213>  Artificial Sequence

<220>
<223>  Synthetic peptide

<400>  378

Gly Ser Gln Val Asn Ser
1               5


<210>  379
<211>  6
<212>  PRT
<213>  Artificial Sequence

<220>
<223>  Synthetic peptide

<400>  379

Gly Ser Gln Val Gln Ser
1               5


<210>  380
<211>  6
<212>  PRT
<213>  Artificial Sequence

<220>
<223>  Synthetic peptide

<400>  380

Gly Ser Gln Val Ser Ser
1               5


<210>  381
<211>  6
<212>  PRT
<213>  Artificial Sequence

<220>
<223>  Synthetic peptide

<400>  381

Gly Ser Gln Val Ser Thr
1               5


<210>  382
<211>  6
<212>  PRT
<213>  Artificial Sequence

<220>
<223>  Synthetic peptide

<400>  382

Gly Ser Gln Val Thr Gly
1               5


<210>  383
<211>  6
<212>  PRT
<213>  Artificial Sequence

<220>
<223>  Synthetic peptide

<400>  383

Gly Ser Gln Val Thr Ser
1               5


<210>  384
<211>  6
<212>  PRT
<213>  Artificial Sequence

<220>
<223>  Synthetic peptide

<400>  384

Gly Ser Ser Ala Gln Ser
1               5


<210>  385
<211>  6
<212>  PRT
<213>  Artificial Sequence

<220>
<223>  Synthetic peptide

<400>  385

Gly Ser Ser Gly Gln Val
1               5


<210>  386
<211>  6
<212>  PRT
<213>  Artificial Sequence

<220>
<223>  Synthetic peptide

<400>  386

Gly Ser Ser Asn Gln Ala
1               5

<210>  387
<211>  6
<212>  PRT
<213>  Artificial Sequence

<220>
<223>  Synthetic peptide

<400>  387

Gly Ser Ser Asn Gln Thr
1               5


<210>  388
<211>  6
<212>  PRT
<213>  Artificial Sequence

<220>
<223>  Synthetic peptide

<400>  388

Gly Ser Ser Asn Gln Val
1               5


<210>  389
<211>  6
<212>  PRT
<213>  Artificial Sequence

<220>
<223>  Synthetic peptide

<400>  389

Gly Ser Ser Asn Ser Val
1               5


<210>  390
<211>  6
<212>  PRT
<213>  Artificial Sequence

<220>
<223>  Synthetic peptide

<400>  390

Gly Ser Ser Asn Thr Val
1               5


<210>  391
<211>  6
<212>  PRT
<213>  Artificial Sequence

<220>
<223>  Synthetic peptide

<400>  391

Gly Ser Ser Asn Val Thr
1               5


<210>  392
<211>  6
<212>  PRT
<213>  Artificial Sequence

<220>
<223>  Synthetic peptide

<400>  392

Gly Ser Ser Gln Ala Gln
1               5


<210>  393
<211>  6
<212>  PRT
<213>  Artificial Sequence

<220>
<223>  Synthetic peptide

<400>  393

Gly Ser Ser Gln Gln Ala
1               5


<210>  394
<211>  6
<212>  PRT
<213>  Artificial Sequence

<220>
<223>  Synthetic peptide

<400>  394

Gly Ser Ser Gln Gln Val
1               5


<210>  395
<211>  6
<212>  PRT
<213>  Artificial Sequence

<220>
<223>  Synthetic peptide

<400>  395

Gly Ser Ser Gln Ser Gln
1               5

<210>  396
<211>  6
<212>  PRT
<213>  Artificial Sequence

<220>
<223>  Synthetic peptide

<400>  396

Gly Ser Ser Ser Gln Gln
1               5


<210>  397
<211>  6
<212>  PRT
<213>  Artificial Sequence

<220>
<223>  Synthetic peptide

<400>  397

Gly Ser Ser Ser Gln Ser
1               5


<210>  398
<211>  6
<212>  PRT
<213>  Artificial Sequence

<220>
<223>  Synthetic peptide

<400>  398

Gly Ser Ser Ser Gln Val
1               5


<210>  399
<211>  6
<212>  PRT
<213>  Artificial Sequence

<220>
<223>  Synthetic peptide

<400>  399

Gly Ser Ser Thr Gln Ala
1               5


<210>  400
<211>  6
<212>  PRT
<213>  Artificial Sequence

<220>
<223>  Synthetic peptide

<400>  400

Gly Ser Ser Thr Gln Gly
1               5


<210>  401
<211>  6
<212>  PRT
<213>  Artificial Sequence

<220>
<223>  Synthetic peptide

<400>  401

Gly Ser Ser Thr Gln Thr
1               5


<210>  402
<211>  6
<212>  PRT
<213>  Artificial Sequence

<220>
<223>  Synthetic peptide

<400>  402

Gly Ser Ser Thr Gln Val
1               5


<210>  403
<211>  6
<212>  PRT
<213>  Artificial Sequence

<220>
<223>  Synthetic peptide

<400>  403

Gly Ser Ser Thr Ser Val
1               5


<210>  404
<211>  6
<212>  PRT
<213>  Artificial Sequence

<220>
<223>  Synthetic peptide

<400>  404

Gly Ser Ser Thr Val Asn
1               5

<210>  405
<211>  6
<212>  PRT
<213>  Artificial Sequence

<220>
<223>  Synthetic peptide

<400>  405

Gly Ser Ser Val Asn Ser
1               5


<210>  406
<211>  6
<212>  PRT
<213>  Artificial Sequence

<220>
<223>  Synthetic peptide

<400>  406

Gly Ser Ser Val Gln Asn
1               5


<210>  407
<211>  6
<212>  PRT
<213>  Artificial Sequence

<220>
<223>  Synthetic peptide

<400>  407

Gly Ser Ser Val Gln Thr
1               5


<210>  408
<211>  6
<212>  PRT
<213>  Artificial Sequence

<220>
<223>  Synthetic peptide

<400>  408

Gly Ser Ser Val Ser Gly
1               5


<210>  409
<211>  6
<212>  PRT
<213>  Artificial Sequence

<220>
<223>  Synthetic peptide

<400>  409

Gly Ser Ser Val Ser Ser
1               5


<210>  410
<211>  6
<212>  PRT
<213>  Artificial Sequence

<220>
<223>  Synthetic peptide

<400>  410

Gly Ser Ser Val Ser Thr
1               5


<210>  411
<211>  6
<212>  PRT
<213>  Artificial Sequence

<220>
<223>  Synthetic peptide

<400>  411

Gly Ser Ser Val Thr Asn
1               5


<210>  412
<211>  6
<212>  PRT
<213>  Artificial Sequence

<220>
<223>  Synthetic peptide

<400>  412

Gly Thr Asn Ser Ser Val
1               5


<210>  413
<211>  6
<212>  PRT
<213>  Artificial Sequence

<220>
<223>  Synthetic peptide

<400>  413

Gly Thr Asn Ser Val Ser
1               5

<210>  414
<211>  6
<212>  PRT
<213>  Artificial Sequence

<220>
<223>  Synthetic peptide

<400>  414

Gly Thr Asn Thr Val Ser
1               5


<210>  415
<211>  6
<212>  PRT
<213>  Artificial Sequence

<220>
<223>  Synthetic peptide

<400>  415

Gly Thr Asn Val Gln Ser
1               5


<210>  416
<211>  6
<212>  PRT
<213>  Artificial Sequence

<220>
<223>  Synthetic peptide

<400>  416

Gly Thr Asn Val Ser Ser
1               5


<210>  417
<211>  6
<212>  PRT
<213>  Artificial Sequence

<220>
<223>  Synthetic peptide

<400>  417

Gly Thr Asn Val Thr Ser
1               5


<210>  418
<211>  6
<212>  PRT
<213>  Artificial Sequence

<220>
<223>  Synthetic peptide

<400>  418

Gly Thr Gln Gln Ser Gln
1               5


<210>  419
<211>  6
<212>  PRT
<213>  Artificial Sequence

<220>
<223>  Synthetic peptide

<400>  419

Gly Thr Gln Ser Thr Ser
1               5


<210>  420
<211>  6
<212>  PRT
<213>  Artificial Sequence

<220>
<223>  Synthetic peptide

<400>  420

Gly Thr Gln Ser Thr Val
1               5


<210>  421
<211>  6
<212>  PRT
<213>  Artificial Sequence

<220>
<223>  Synthetic peptide

<400>  421

Gly Thr Gln Thr Ser Ala
1               5


<210>  422
<211>  6
<212>  PRT
<213>  Artificial Sequence

<220>
<223>  Synthetic peptide

<400>  422

Gly Thr Gln Thr Ser Val
1               5

<210>  423
<211>  6
<212>  PRT
<213>  Artificial Sequence

<220>
<223>  Synthetic peptide

<400>  423

Gly Thr Gln Val Gln Ser
1               5


<210>  424
<211>  6
<212>  PRT
<213>  Artificial Sequence

<220>
<223>  Synthetic peptide

<400>  424

Gly Thr Gln Val Ser Gly
1               5


<210>  425
<211>  6
<212>  PRT
<213>  Artificial Sequence

<220>
<223>  Synthetic peptide

<400>  425

Gly Thr Gln Val Ser Asn
1               5


<210>  426
<211>  6
<212>  PRT
<213>  Artificial Sequence

<220>
<223>  Synthetic peptide

<400>  426

Gly Thr Gln Val Ser Ser
1               5


<210>  427
<211>  6
<212>  PRT
<213>  Artificial Sequence

<220>
<223>  Synthetic peptide

<400>  427

Gly Thr Gln Val Ser Thr
1               5


<210>  428
<211>  6
<212>  PRT
<213>  Artificial Sequence

<220>
<223>  Synthetic peptide

<400>  428

Gly Thr Ser Ala Gln Ser
1               5


<210>  429
<211>  6
<212>  PRT
<213>  Artificial Sequence

<220>
<223>  Synthetic peptide

<400>  429

Gly Thr Ser Gly Gln Val
1               5


<210>  430
<211>  6
<212>  PRT
<213>  Artificial Sequence

<220>
<223>  Synthetic peptide

<400>  430

Gly Thr Ser Asn Gln Ala
1               5


<210>  431
<211>  6
<212>  PRT
<213>  Artificial Sequence

<220>
<223>  Synthetic peptide

<400>  431

Gly Thr Ser Asn Gln Ser
1               5

<210>  432
<211>  6
<212>  PRT
<213>  Artificial Sequence

<220>
<223>  Synthetic peptide

<400>  432

Gly Thr Ser Asn Gln Thr
1               5


<210>  433
<211>  6
<212>  PRT
<213>  Artificial Sequence

<220>
<223>  Synthetic peptide

<400>  433

Gly Thr Ser Asn Gln Val
1               5


<210>  434
<211>  6
<212>  PRT
<213>  Artificial Sequence

<220>
<223>  Synthetic peptide

<400>  434

Gly Thr Ser Asn Ser Val
1               5


<210>  435
<211>  6
<212>  PRT
<213>  Artificial Sequence

<220>
<223>  Synthetic peptide

<400>  435

Gly Thr Ser Asn Val Ser
1               5


<210>  436
<211>  6
<212>  PRT
<213>  Artificial Sequence

<220>
<223>  Synthetic peptide

<400>  436

Gly Thr Ser Gln Gln Ala
1               5


<210>  437
<211>  6
<212>  PRT
<213>  Artificial Sequence

<220>
<223>  Synthetic peptide

<400>  437

Gly Thr Ser Gln Gln Ser
1               5


<210>  438
<211>  6
<212>  PRT
<213>  Artificial Sequence

<220>
<223>  Synthetic peptide

<400>  438

Gly Thr Ser Gln Gln Val
1               5


<210>  439
<211>  6
<212>  PRT
<213>  Artificial Sequence

<220>
<223>  Synthetic peptide

<400>  439

Gly Thr Ser Gln Ser Gln
1               5


<210>  440
<211>  6
<212>  PRT
<213>  Artificial Sequence

<220>
<223>  Synthetic peptide

<400>  440

Gly Thr Ser Gln Ser Val
1               5

<210>  441
<211>  6
<212>  PRT
<213>  Artificial Sequence

<220>
<223>  Synthetic peptide

<400>  441

Gly Thr Ser Ser Asn Val
1               5


<210>  442
<211>  6
<212>  PRT
<213>  Artificial Sequence

<220>
<223>  Synthetic peptide

<400>  442

Gly Thr Ser Thr Gln Ala
1               5


<210>  443
<211>  6
<212>  PRT
<213>  Artificial Sequence

<220>
<223>  Synthetic peptide

<400>  443

Gly Thr Ser Thr Gln Ser
1               5


<210>  444
<211>  6
<212>  PRT
<213>  Artificial Sequence

<220>
<223>  Synthetic peptide

<400>  444

Gly Thr Ser Thr Gln Thr
1               5


<210>  445
<211>  6
<212>  PRT
<213>  Artificial Sequence

<220>
<223>  Synthetic peptide

<400>  445

Gly Thr Ser Thr Gln Val
1               5


<210>  446
<211>  6
<212>  PRT
<213>  Artificial Sequence

<220>
<223>  Synthetic peptide

<400>  446

Gly Thr Ser Thr Ser Val
1               5


<210>  447
<211>  6
<212>  PRT
<213>  Artificial Sequence

<220>
<223>  Synthetic peptide

<400>  447

Gly Thr Ser Val Ala Gly
1               5


<210>  448
<211>  6
<212>  PRT
<213>  Artificial Sequence

<220>
<223>  Synthetic peptide

<400>  448

Gly Thr Ser Val Ala Ser
1               5


<210>  449
<211>  6
<212>  PRT
<213>  Artificial Sequence

<220>
<223>  Synthetic peptide

<400>  449

Gly Thr Ser Val Asn Ser
1               5

<210>  450
<211>  6
<212>  PRT
<213>  Artificial Sequence

<220>
<223>  Synthetic peptide

<400>  450

Gly Thr Ser Val Asn Thr
1               5


<210>  451
<211>  6
<212>  PRT
<213>  Artificial Sequence

<220>
<223>  Synthetic peptide

<400>  451

Gly Thr Ser Val Gln Gly
1               5


<210>  452
<211>  6
<212>  PRT
<213>  Artificial Sequence

<220>
<223>  Synthetic peptide

<400>  452

Gly Thr Ser Val Gln Asn
1               5


<210>  453
<211>  6
<212>  PRT
<213>  Artificial Sequence

<220>
<223>  Synthetic peptide

<400>  453

Gly Thr Ser Val Gln Gln
1               5


<210>  454
<211>  6
<212>  PRT
<213>  Artificial Sequence

<220>
<223>  Synthetic peptide

<400>  454

Gly Thr Ser Val Gln Ser
1               5

<210>  455
<211>  6
<212>  PRT
<213>  Artificial Sequence

<220>
<223>  Synthetic peptide

<400>  455

Gly Thr Ser Val Gln Thr
1               5

<210>  456
<211>  6
<212>  PRT
<213>  Artificial Sequence

<220>
<223>  Synthetic peptide

<400>  456

Gly Thr Ser Val Ser Gly
1               5

<210>  457
<211>  6
<212>  PRT
<213>  Artificial Sequence

<220>
<223>  Synthetic peptide

<400>  457

Gly Thr Ser Val Ser Asn
1               5

<210>  458
<211>  6
<212>  PRT
<213>  Artificial Sequence

<220>
<223>  Synthetic peptide

<400>  458

Gly Thr Ser Val Ser Ser
1               5

<210>  459
<211>  6
<212>  PRT
<213>  Artificial Sequence

<220>
<223>  Synthetic peptide

<400>  459

Gly Thr Ser Val Ser Thr
1               5


<210>  460
<211>  6
<212>  PRT
<213>  Artificial Sequence

<220>
<223>  Synthetic peptide

<400>  460

Gly Thr Ser Val Thr Ser
1               5


<210>  461
<211>  6
<212>  PRT
<213>  Artificial Sequence

<220>
<223>  Synthetic peptide

<400>  461

Gly Val Asn Ser Gln Ser
1               5


<210>  462
<211>  6
<212>  PRT
<213>  Artificial Sequence

<220>
<223>  Synthetic peptide

<400>  462

Gly Val Asn Ser Ser Thr
1               5


<210>  463
<211>  6
<212>  PRT
<213>  Artificial Sequence

<220>
<223>  Synthetic peptide

<400>  463

Gly Val Asn Ser Thr Ser
1               5


<210>  464
<211>  6
<212>  PRT
<213>  Artificial Sequence

<220>
<223>  Synthetic peptide

<400>  464

Gly Val Asn Thr Gln Ser
1               5


<210>  465
<211>  6
<212>  PRT
<213>  Artificial Sequence

<220>
<223>  Synthetic peptide

<400>  465

Gly Val Asn Thr Ser Ser
1               5


<210>  466
<211>  6
<212>  PRT
<213>  Artificial Sequence

<220>
<223>  Synthetic peptide

<400>  466

Gly Val Gln Asn Thr Ser
1               5


<210>  467
<211>  6
<212>  PRT
<213>  Artificial Sequence

<220>
<223>  Synthetic peptide

<400>  467

Gly Val Gln Gln Ser Gln
1               5

<210>  468
<211>  6
<212>  PRT
<213>  Artificial Sequence

<220>
<223>  Synthetic peptide

<400>  468

Gly Val Gln Ser Asn Gln
1               5


<210>  469
<211>  6
<212>  PRT
<213>  Artificial Sequence

<220>
<223>  Synthetic peptide

<400>  469

Gly Val Gln Ser Asn Ser
1               5


<210>  470
<211>  6
<212>  PRT
<213>  Artificial Sequence

<220>
<223>  Synthetic peptide

<400>  470

Gly Val Gln Ser Ser Gly
1               5


<210>  471
<211>  6
<212>  PRT
<213>  Artificial Sequence

<220>
<223>  Synthetic peptide

<400>  471

Gly Val Gln Ser Ser Gln
1               5


<210>  472
<211>  6
<212>  PRT
<213>  Artificial Sequence

<220>
<223>  Synthetic peptide

<400>  472

Gly Val Gln Ser Thr Ser
1               5


<210>  473
<211>  6
<212>  PRT
<213>  Artificial Sequence

<220>
<223>  Synthetic peptide

<400>  473

Gly Val Gln Ser Thr Thr
1               5


<210>  474
<211>  6
<212>  PRT
<213>  Artificial Sequence

<220>
<223>  Synthetic peptide

<400>  474

Gly Val Gln Thr Asn Ser
1               5


<210>  475
<211>  6
<212>  PRT
<213>  Artificial Sequence

<220>
<223>  Synthetic peptide

<400>  475

Gly Val Gln Thr Gln Ser
1               5


<210>  476
<211>  6
<212>  PRT
<213>  Artificial Sequence

<220>
<223>  Synthetic peptide

<400>  476

Gly Val Gln Thr Ser Gly
1               5

<210>  477
<211>  6
<212>  PRT
<213>  Artificial Sequence

<220>
<223>  Synthetic peptide

<400>  477

Gly Val Gln Thr Ser Ser
1               5


<210>  478
<211>  6
<212>  PRT
<213>  Artificial Sequence

<220>
<223>  Synthetic peptide

<400>  478

Gly Val Ser Gly Gln Gly
1               5


<210>  479
<211>  6
<212>  PRT
<213>  Artificial Sequence

<220>
<223>  Synthetic peptide

<400>  479

Gly Val Ser Gly Gln Thr
1               5


<210>  480
<211>  6
<212>  PRT
<213>  Artificial Sequence

<220>
<223>  Synthetic peptide

<400>  480

Gly Val Ser Asn Ala Ser
1               5


<210>  481
<211>  6
<212>  PRT
<213>  Artificial Sequence

<220>
<223>  Synthetic peptide

<400>  481

Gly Val Ser Asn Asn Val
1               5


<210>  482
<211>  6
<212>  PRT
<213>  Artificial Sequence

<220>
<223>  Synthetic peptide

<400>  482

Gly Val Ser Asn Gln Gly
1               5


<210>  483
<211>  6
<212>  PRT
<213>  Artificial Sequence

<220>
<223>  Synthetic peptide

<400>  483

Gly Val Ser Asn Gln Asn
1               5


<210>  484
<211>  6
<212>  PRT
<213>  Artificial Sequence

<220>
<223>  Synthetic peptide

<400>  484

Gly Val Ser Asn Gln Gln
1               5


<210>  485
<211>  6
<212>  PRT
<213>  Artificial Sequence

<220>
<223>  Synthetic peptide

<400>  485

Gly Val Ser Asn Gln Ser
1               5

<210>  486
<211>  6
<212>  PRT
<213>  Artificial Sequence

<220>
<223>  Synthetic peptide

<400>  486

Gly Val Ser Asn Gln Thr
1               5


<210>  487
<211>  6
<212>  PRT
<213>  Artificial Sequence

<220>
<223>  Synthetic peptide

<400>  487

Gly Val Ser Asn Ser Ser
1               5


<210>  488
<211>  6
<212>  PRT
<213>  Artificial Sequence

<220>
<223>  Synthetic peptide

<400>  488

Gly Val Ser Asn Ser Thr
1               5


<210>  489
<211>  6
<212>  PRT
<213>  Artificial Sequence

<220>
<223>  Synthetic peptide

<400>  489

Gly Val Ser Asn Thr Ser
1               5


<210>  490
<211>  6
<212>  PRT
<213>  Artificial Sequence

<220>
<223>  Synthetic peptide

<400>  490

Gly Val Ser Gln Ala Gln
1               5


<210>  491
<211>  6
<212>  PRT
<213>  Artificial Sequence

<220>
<223>  Synthetic peptide

<400>  491

Gly Val Ser Gln Asn Gln
1               5


<210>  492
<211>  6
<212>  PRT
<213>  Artificial Sequence

<220>
<223>  Synthetic peptide

<400>  492

Gly Val Ser Gln Asn Ser
1               5


<210>  493
<211>  6
<212>  PRT
<213>  Artificial Sequence

<220>
<223>  Synthetic peptide

<400>  493

Gly Val Ser Gln Gln Gly
1               5


<210>  494
<211>  6
<212>  PRT
<213>  Artificial Sequence

<220>
<223>  Synthetic peptide

<400>  494

Gly Val Ser Gln Gln Ser
1               5

<210>  495
<211>  6
<212>  PRT
<213>  Artificial Sequence

<220>
<223>  Synthetic peptide

<400>  495

Gly Val Ser Gln Gln Thr
1               5


<210>  496
<211>  6
<212>  PRT
<213>  Artificial Sequence

<220>
<223>  Synthetic peptide

<400>  496

Gly Val Ser Gln Ser Gly
1               5


<210>  497
<211>  6
<212>  PRT
<213>  Artificial Sequence

<220>
<223>  Synthetic peptide

<400>  497

Gly Val Ser Gln Ser Gln
1               5


<210>  498
<211>  6
<212>  PRT
<213>  Artificial Sequence

<220>
<223>  Synthetic peptide

<400>  498

Gly Val Ser Gln Ser Ser
1               5


<210>  499
<211>  6
<212>  PRT
<213>  Artificial Sequence

<220>
<223>  Synthetic peptide

<400>  499

Gly Val Ser Ser Ala Gln
1               5


<210>  500
<211>  6
<212>  PRT
<213>  Artificial Sequence

<220>
<223>  Synthetic peptide

<400>  500

Gly Val Ser Ser Ala Ser
1               5


<210>  501
<211>  6
<212>  PRT
<213>  Artificial Sequence

<220>
<223>  Synthetic peptide

<400>  501

Gly Val Ser Ser Gly Gln
1               5


<210>  502
<211>  6
<212>  PRT
<213>  Artificial Sequence

<220>
<223>  Synthetic peptide

<400>  502

Gly Val Ser Ser Asn Gly
1               5


<210>  503
<211>  6
<212>  PRT
<213>  Artificial Sequence

<220>
<223>  Synthetic peptide

<400>  503

Gly Val Ser Ser Asn Gln
1               5

<210>  504
<211>  6
<212>  PRT
<213>  Artificial Sequence

<220>
<223>  Synthetic peptide

<400>  504

Gly Val Ser Ser Asn Ser
1               5


<210>  505
<211>  6
<212>  PRT
<213>  Artificial Sequence

<220>
<223>  Synthetic peptide

<400>  505

Gly Val Ser Ser Asn Thr
1               5


<210>  506
<211>  6
<212>  PRT
<213>  Artificial Sequence

<220>
<223>  Synthetic peptide

<400>  506

Gly Val Ser Ser Gln Gly
1               5


<210>  507
<211>  6
<212>  PRT
<213>  Artificial Sequence

<220>
<223>  Synthetic peptide

<400>  507

Gly Val Ser Ser Gln Asn
1               5


<210>  508
<211>  6
<212>  PRT
<213>  Artificial Sequence

<220>
<223>  Synthetic peptide

<400>  508

Gly Val Ser Ser Gln Gln
1               5


<210>  509
<211>  6
<212>  PRT
<213>  Artificial Sequence

<220>
<223>  Synthetic peptide

<400>  509

Gly Val Ser Ser Ser Gly
1               5


<210>  510
<211>  6
<212>  PRT
<213>  Artificial Sequence

<220>
<223>  Synthetic peptide

<400>  510

Gly Val Ser Ser Ser Gln
1               5


<210>  511
<211>  6
<212>  PRT
<213>  Artificial Sequence

<220>
<223>  Synthetic peptide

<400>  511

Gly Val Ser Ser Ser Ser
1               5


<210>  512
<211>  6
<212>  PRT
<213>  Artificial Sequence

<220>
<223>  Synthetic peptide

<400>  512

Gly Val Ser Ser Thr Asn
1               5

<210>  513
<211>  6
<212>  PRT
<213>  Artificial Sequence

<220>
<223>  Synthetic peptide

<400>  513

Gly Val Ser Thr Ala Ser
1               5


<210>  514
<211>  6
<212>  PRT
<213>  Artificial Sequence

<220>
<223>  Synthetic peptide

<400>  514

Gly Val Ser Thr Asn Ser
1               5


<210>  515
<211>  6
<212>  PRT
<213>  Artificial Sequence

<220>
<223>  Synthetic peptide

<400>  515

Gly Val Ser Thr Gln Gly
1               5


<210>  516
<211>  6
<212>  PRT
<213>  Artificial Sequence

<220>
<223>  Synthetic peptide

<400>  516

Gly Val Ser Thr Gln Asn
1               5


<210>  517
<211>  6
<212>  PRT
<213>  Artificial Sequence

<220>
<223>  Synthetic peptide

<400>  517

Gly Val Ser Thr Gln Gln
1               5


<210>  518
<211>  6
<212>  PRT
<213>  Artificial Sequence

<220>
<223>  Synthetic peptide

<400>  518

Gly Val Ser Thr Gln Ser
1               5


<210>  519
<211>  6
<212>  PRT
<213>  Artificial Sequence

<220>
<223>  Synthetic peptide

<400>  519

Gly Val Ser Thr Gln Thr
1               5


<210>  520
<211>  6
<212>  PRT
<213>  Artificial Sequence

<220>
<223>  Synthetic peptide

<400>  520

Gly Val Ser Thr Ser Asn
1               5


<210>  521
<211>  6
<212>  PRT
<213>  Artificial Sequence

<220>
<223>  Synthetic peptide

<400>  521

Gly Val Ser Thr Ser Gln
1               5

<210>  522
<211>  6
<212>  PRT
<213>  Artificial Sequence

<220>
<223>  Synthetic peptide

<400>  522

Gly Val Ser Thr Ser Ser
1               5


<210>  523
<211>  6
<212>  PRT
<213>  Artificial Sequence

<220>
<223>  Synthetic peptide

<400>  523

Gly Val Ser Thr Ser Thr
1               5


<210>  524
<211>  6
<212>  PRT
<213>  Artificial Sequence

<220>
<223>  Synthetic peptide

<400>  524

Gly Val Thr Asn Ser Ser
1               5


<210>  525
<211>  6
<212>  PRT
<213>  Artificial Sequence

<220>
<223>  Synthetic peptide

<400>  525

Gly Val Thr Ser Asn Ser
1               5


<210>  526
<211>  6
<212>  PRT
<213>  Artificial Sequence

<220>
<223>  Synthetic peptide

<400>  526

Gly Val Thr Ser Ser Asn
1               5


<210>  527
<211>  6
<212>  PRT
<213>  Artificial Sequence

<220>
<223>  Synthetic peptide

<400>  527

Asn Gly Ser Thr Ser Val
1               5


<210>  528
<211>  6
<212>  PRT
<213>  Artificial Sequence

<220>
<223>  Synthetic peptide

<400>  528

Asn Gly Ser Val Thr Ser
1               5


<210>  529
<211>  6
<212>  PRT
<213>  Artificial Sequence

<220>
<223>  Synthetic peptide

<400>  529

Asn Asn Ser Val Ser Ser
1               5


<210>  530
<211>  6
<212>  PRT
<213>  Artificial Sequence

<220>
<223>  Synthetic peptide

<400>  530

Asn Gln Ser Gln Ser Gln
1               5

<210>  531
<211>  6
<212>  PRT
<213>  Artificial Sequence

<220>
<223>  Synthetic peptide

<400>  531

Asn Gln Ser Val Ser Asn
1               5


<210>  532
<211>  6
<212>  PRT
<213>  Artificial Sequence

<220>
<223>  Synthetic peptide

<400>  532

Asn Gln Ser Val Ser Gln
1               5


<210>  533
<211>  6
<212>  PRT
<213>  Artificial Sequence

<220>
<223>  Synthetic peptide

<400>  533

Asn Gln Ser Val Ser Ser
1               5


<210>  534
<211>  6
<212>  PRT
<213>  Artificial Sequence

<220>
<223>  Synthetic peptide

<400>  534

Asn Ser Ser Asn Ser Val
1               5


<210>  535
<211>  6
<212>  PRT
<213>  Artificial Sequence

<220>
<223>  Synthetic peptide

<400>  535

Asn Ser Ser Gln Ser Gln
1               5


<210>  536
<211>  6
<212>  PRT
<213>  Artificial Sequence

<220>
<223>  Synthetic peptide

<400>  536

Asn Ser Ser Thr Val Gly
1               5


<210>  537
<211>  6
<212>  PRT
<213>  Artificial Sequence

<220>
<223>  Synthetic peptide

<400>  537

Asn Ser Ser Val Ser Gly
1               5


<210>  538
<211>  6
<212>  PRT
<213>  Artificial Sequence

<220>
<223>  Synthetic peptide

<400>  538

Asn Ser Ser Val Ser Asn
1               5


<210>  539
<211>  6
<212>  PRT
<213>  Artificial Sequence

<220>
<223>  Synthetic peptide

<400>  539

Asn Ser Ser Val Ser Ser
1               5

<210>  540
<211>  6
<212>  PRT
<213>  Artificial Sequence

<220>
<223>  Synthetic peptide

<400>  540

Asn Ser Ser Val Thr Gly
1               5


<210>  541
<211>  6
<212>  PRT
<213>  Artificial Sequence

<220>
<223>  Synthetic peptide

<400>  541

Asn Thr Asn Val Asn Ser
1               5


<210>  542
<211>  6
<212>  PRT
<213>  Artificial Sequence

<220>
<223>  Synthetic peptide

<400>  542

Asn Thr Gln Val Ser Ser
1               5


<210>  543
<211>  6
<212>  PRT
<213>  Artificial Sequence

<220>
<223>  Synthetic peptide

<400>  543

Asn Thr Ser Gly Ser Val
1               5


<210>  544
<211>  6
<212>  PRT
<213>  Artificial Sequence

<220>
<223>  Synthetic peptide

<400>  544

Asn Thr Ser Gln Ser Gln
1               5


<210>  545
<211>  6
<212>  PRT
<213>  Artificial Sequence

<220>
<223>  Synthetic peptide

<400>  545

Asn Thr Ser Thr Ser Ser
1               5


<210>  546
<211>  6
<212>  PRT
<213>  Artificial Sequence

<220>
<223>  Synthetic peptide

<400>  546

Asn Thr Ser Thr Ser Val
1               5


<210>  547
<211>  6
<212>  PRT
<213>  Artificial Sequence

<220>
<223>  Synthetic peptide

<400>  547

Asn Thr Ser Val Ser Ser
1               5


<210>  548
<211>  6
<212>  PRT
<213>  Artificial Sequence

<220>
<223>  Synthetic peptide

<400>  548

Asn Val Ser Gly Ser Thr
1               5

<210>  549
<211>  6
<212>  PRT
<213>  Artificial Sequence

<220>
<223>  Synthetic peptide

<400>  549

Asn Val Ser Asn Ser Gly
1               5


<210>  550
<211>  6
<212>  PRT
<213>  Artificial Sequence

<220>
<223>  Synthetic peptide

<400>  550

Asn Val Ser Gln Ser Gly
1               5


<210>  551
<211>  6
<212>  PRT
<213>  Artificial Sequence

<220>
<223>  Synthetic peptide

<400>  551

Asn Val Ser Gln Ser Gln
1               5


<210>  552
<211>  6
<212>  PRT
<213>  Artificial Sequence

<220>
<223>  Synthetic peptide

<400>  552

Asn Val Ser Gln Ser Ser
1               5


<210>  553
<211>  6
<212>  PRT
<213>  Artificial Sequence

<220>
<223>  Synthetic peptide

<400>  553

Asn Val Ser Ser Ser Gly
1               5


<210>  554
<211>  6
<212>  PRT
<213>  Artificial Sequence

<220>
<223>  Synthetic peptide

<400>  554

Asn Val Ser Ser Ser Ser
1               5


<210>  555
<211>  6
<212>  PRT
<213>  Artificial Sequence

<220>
<223>  Synthetic peptide

<400>  555

Asn Val Ser Ser Thr Gly
1               5


<210>  556
<211>  6
<212>  PRT
<213>  Artificial Sequence

<220>
<223>  Synthetic peptide

<400>  556

Asn Val Ser Thr Ser Asn
1               5


<210>  557
<211>  6
<212>  PRT
<213>  Artificial Sequence

<220>
<223>  Synthetic peptide

<400>  557

Asn Val Ser Thr Ser Ser
1               5

<210>  558
<211>  6
<212>  PRT
<213>  Artificial Sequence

<220>
<223>  Synthetic peptide

<400>  558

Asn Val Ser Thr Ser Thr
1               5


<210>  559
<211>  6
<212>  PRT
<213>  Artificial Sequence

<220>
<223>  Synthetic peptide

<400>  559

Gln Asn Ser Thr Ser Val
1               5


<210>  560
<211>  6
<212>  PRT
<213>  Artificial Sequence

<220>
<223>  Synthetic peptide

<400>  560

Gln Asn Ser Val Ser Gly
1               5


<210>  561
<211>  6
<212>  PRT
<213>  Artificial Sequence

<220>
<223>  Synthetic peptide

<400>  561

Gln Gln Ser Gln Ser Gln
1               5


<210>  562
<211>  6
<212>  PRT
<213>  Artificial Sequence

<220>
<223>  Synthetic peptide

<400>  562

Gln Gln Ser Val Ala Gly
1               5


<210>  563
<211>  6
<212>  PRT
<213>  Artificial Sequence

<220>
<223>  Synthetic peptide

<400>  563

Gln Gln Ser Val Ala Gln
1               5


<210>  564
<211>  6
<212>  PRT
<213>  Artificial Sequence

<220>
<223>  Synthetic peptide

<400>  564

Gln Gln Ser Val Ala Ser
1               5


<210>  565
<211>  6
<212>  PRT
<213>  Artificial Sequence

<220>
<223>  Synthetic peptide

<400>  565

Gln Gln Ser Val Ala Thr
1               5


<210>  566
<211>  6
<212>  PRT
<213>  Artificial Sequence

<220>
<223>  Synthetic peptide

<400>  566

Gln Gln Ser Val Ser Gly
1               5

<210>  567
<211>  6
<212>  PRT
<213>  Artificial Sequence

<220>
<223>  Synthetic peptide

<400>  567

Gln Gln Ser Val Ser Asn
1               5


<210>  568
<211>  6
<212>  PRT
<213>  Artificial Sequence

<220>
<223>  Synthetic peptide

<400>  568

Gln Gln Ser Val Ser Ser
1               5


<210>  569
<211>  6
<212>  PRT
<213>  Artificial Sequence

<220>
<223>  Synthetic peptide

<400>  569

Gln Ser Asn Gln Val Gln
1               5


<210>  570
<211>  6
<212>  PRT
<213>  Artificial Sequence

<220>
<223>  Synthetic peptide

<400>  570

Gln Ser Asn Thr Ala Val
1               5


<210>  571
<211>  6
<212>  PRT
<213>  Artificial Sequence

<220>
<223>  Synthetic peptide

<400>  571

Gln Ser Asn Thr Asn Val
1               5


<210>  572
<211>  6
<212>  PRT
<213>  Artificial Sequence

<220>
<223>  Synthetic peptide

<400>  572

Gln Ser Ser Asn Ser Val
1               5


<210>  573
<211>  6
<212>  PRT
<213>  Artificial Sequence

<220>
<223>  Synthetic peptide

<400>  573

Gln Ser Ser Gln Ala Gln
1               5


<210>  574
<211>  6
<212>  PRT
<213>  Artificial Sequence

<220>
<223>  Synthetic peptide

<400>  574

Gln Ser Ser Gln Ser Gln
1               5


<210>  575
<211>  6
<212>  PRT
<213>  Artificial Sequence

<220>
<223>  Synthetic peptide

<400>  575

Gln Ser Ser Gln Ser Val
1               5

<210>  576
<211>  6
<212>  PRT
<213>  Artificial Sequence

<220>
<223>  Synthetic peptide

<400>  576

Gln Ser Ser Thr Ser Val
1               5


<210>  577
<211>  6
<212>  PRT
<213>  Artificial Sequence

<220>
<223>  Synthetic peptide

<400>  577

Gln Ser Ser Val Ser Gly
1               5


<210>  578
<211>  6
<212>  PRT
<213>  Artificial Sequence

<220>
<223>  Synthetic peptide

<400>  578

Gln Ser Ser Val Ser Asn
1               5


<210>  579
<211>  6
<212>  PRT
<213>  Artificial Sequence

<220>
<223>  Synthetic peptide

<400>  579

Gln Ser Ser Val Ser Gln
1               5


<210>  580
<211>  6
<212>  PRT
<213>  Artificial Sequence

<220>
<223>  Synthetic peptide

<400>  580

Gln Ser Ser Val Ser Ser
1               5


<210>  581
<211>  6
<212>  PRT
<213>  Artificial Sequence

<220>
<223>  Synthetic peptide

<400>  581

Gln Ser Ser Val Thr Gly
1               5


<210>  582
<211>  6
<212>  PRT
<213>  Artificial Sequence

<220>
<223>  Synthetic peptide

<400>  582

Gln Ser Ser Val Thr Ser
1               5


<210>  583
<211>  6
<212>  PRT
<213>  Artificial Sequence

<220>
<223>  Synthetic peptide

<400>  583

Gln Thr Ser Gln Ser Gln
1               5


<210>  584
<211>  6
<212>  PRT
<213>  Artificial Sequence

<220>
<223>  Synthetic peptide

<400>  584

Gln Thr Ser Ser Ser Gln
1               5

<210>  585
<211>  6
<212>  PRT
<213>  Artificial Sequence

<220>
<223>  Synthetic peptide

<400>  585

Gln Thr Ser Ser Ser Val
1               5


<210>  586
<211>  6
<212>  PRT
<213>  Artificial Sequence

<220>
<223>  Synthetic peptide

<400>  586

Gln Thr Ser Val Ala Gly
1               5


<210>  587
<211>  6
<212>  PRT
<213>  Artificial Sequence

<220>
<223>  Synthetic peptide

<400>  587

Gln Thr Ser Val Ala Ser
1               5


<210>  588
<211>  6
<212>  PRT
<213>  Artificial Sequence

<220>
<223>  Synthetic peptide

<400>  588

Gln Thr Ser Val Ser Gly
1               5


<210>  589
<211>  6
<212>  PRT
<213>  Artificial Sequence

<220>
<223>  Synthetic peptide

<400>  589

Gln Thr Ser Val Ser Asn
1               5



<210>  590
<211>  6
<212>  PRT
<213>  Artificial Sequence

<220>
<223>  Synthetic peptide

<400>  590

Gln Thr Ser Val Ser Ser
1               5



<210>  591
<211>  6
<212>  PRT
<213>  Artificial Sequence

<220>
<223>  Synthetic peptide

<400>  591

Gln Thr Ser Val Ser Thr
1               5



<210>  592
<211>  6
<212>  PRT
<213>  Artificial Sequence

<220>
<223>  Synthetic peptide

<400>  592

Gln Thr Ser Val Thr Ser
1               5



<210>  593
<211>  6
<212>  PRT
<213>  Artificial Sequence

<220>
<223>  Synthetic peptide

<400>  593

Gln Val Ser Asn Ser Gly
1               5

<210>  594
<211>  6
<212>  PRT
<213>  Artificial Sequence

<220>
<223>  Synthetic peptide

<400>  594

Gln Val Ser Asn Ser Ser
1               5


<210>  595
<211>  6
<212>  PRT
<213>  Artificial Sequence

<220>
<223>  Synthetic peptide

<400>  595

Gln Val Ser Asn Ser Thr
1               5


<210>  596
<211>  6
<212>  PRT
<213>  Artificial Sequence

<220>
<223>  Synthetic peptide

<400>  596

Gln Val Ser Gln Ala Gln
1               5


<210>  597
<211>  6
<212>  PRT
<213>  Artificial Sequence

<220>
<223>  Synthetic peptide

<400>  597

Gln Val Ser Gln Thr Gly
1               5


<210>  598
<211>  6
<212>  PRT
<213>  Artificial Sequence

<220>
<223>  Synthetic peptide

<400>  598

Gln Val Ser Gln Thr Gln
1               5


<210>  599
<211>  6
<212>  PRT
<213>  Artificial Sequence

<220>
<223>  Synthetic peptide

<400>  599

Gln Val Ser Ser Ala Gln
1               5


<210>  600
<211>  6
<212>  PRT
<213>  Artificial Sequence

<220>
<223>  Synthetic peptide

<400>  600

Gln Val Ser Ser Ser Gly
1               5


<210>  601
<211>  6
<212>  PRT
<213>  Artificial Sequence

<220>
<223>  Synthetic peptide

<400>  601

Gln Val Ser Ser Ser Gln
1               5


<210>  602
<211>  6
<212>  PRT
<213>  Artificial Sequence

<220>
<223>  Synthetic peptide

<400>  602

Gln Val Ser Ser Ser Ser
1               5

<210>  603
<211>  6
<212>  PRT
<213>  Artificial Sequence

<220>
<223>  Synthetic peptide

<400>  603

Gln Val Ser Ser Thr Gln
1               5


<210>  604
<211>  6
<212>  PRT
<213>  Artificial Sequence

<220>
<223>  Synthetic peptide

<400>  604

Gln Val Ser Thr Ser Gly
1               5


<210>  605
<211>  6
<212>  PRT
<213>  Artificial Sequence

<220>
<223>  Synthetic peptide

<400>  605

Gln Val Ser Thr Ser Asn
1               5


<210>  606
<211>  6
<212>  PRT
<213>  Artificial Sequence

<220>
<223>  Synthetic peptide

<400>  606

Gln Val Ser Thr Ser Ser
1               5


<210>  607
<211>  6
<212>  PRT
<213>  Artificial Sequence

<220>
<223>  Synthetic peptide

<400>  607

Gln Val Ser Thr Ser Thr
1               5


<210>  608
<211>  6
<212>  PRT
<213>  Artificial Sequence

<220>
<223>  Synthetic peptide

<400>  608

Ser Ala Gln Gln Ser Gln
1               5


<210>  609
<211>  6
<212>  PRT
<213>  Artificial Sequence

<220>
<223>  Synthetic peptide

<400>  609

Ser Ala Gln Gln Thr Gln
1               5


<210>  610
<211>  6
<212>  PRT
<213>  Artificial Sequence

<220>
<223>  Synthetic peptide

<400>  610

Ser Ala Ser Gln Gln Gln
1               5


<210>  611
<211>  6
<212>  PRT
<213>  Artificial Sequence

<220>
<223>  Synthetic peptide

<400>  611

Ser Ala Ser Gln Ser Gln
1               5

<210>  612
<211>  6
<212>  PRT
<213>  Artificial Sequence

<220>
<223>  Synthetic peptide

<400>  612

Ser Gly Asn Ser Thr Val
1               5


<210>  613
<211>  6
<212>  PRT
<213>  Artificial Sequence

<220>
<223>  Synthetic peptide

<400>  613

Ser Gly Asn Thr Ser Val
1               5


<210>  614
<211>  6
<212>  PRT
<213>  Artificial Sequence

<220>
<223>  Synthetic peptide

<400>  614

Ser Gly Asn Val Ser Thr
1               5


<210>  615
<211>  6
<212>  PRT
<213>  Artificial Sequence

<220>
<223>  Synthetic peptide

<400>  615

Ser Gly Asn Val Thr Ser
1               5


<210>  616
<211>  6
<212>  PRT
<213>  Artificial Sequence

<220>
<223>  Synthetic peptide

<400>  616

Ser Gly Gln Gln Thr Gln
1               5


<210>  617
<211>  6
<212>  PRT
<213>  Artificial Sequence

<220>
<223>  Synthetic peptide

<400>  617

Ser Gly Gln Gln Thr Val
1               5


<210>  618
<211>  6
<212>  PRT
<213>  Artificial Sequence

<220>
<223>  Synthetic peptide

<400>  618

Ser Gly Gln Thr Ser Val
1               5


<210>  619
<211>  6
<212>  PRT
<213>  Artificial Sequence

<220>
<223>  Synthetic peptide

<400>  619

Ser Gly Gln Val Ser Ser
1               5


<210>  620
<211>  6
<212>  PRT
<213>  Artificial Sequence

<220>
<223>  Synthetic peptide

<400>  620

Ser Gly Gln Val Thr Gly
1               5

<210>  621
<211>  6
<212>  PRT
<213>  Artificial Sequence

<220>
<223>  Synthetic peptide

<400>  621

Ser Gly Gln Val Thr Gln
1               5


<210>  622
<211>  6
<212>  PRT
<213>  Artificial Sequence

<220>
<223>  Synthetic peptide

<400>  622

Ser Gly Gln Val Thr Thr
1               5


<210>  623
<211>  6
<212>  PRT
<213>  Artificial Sequence

<220>
<223>  Synthetic peptide

<400>  623

Ser Gly Ser Asn Thr Val
1               5


<210>  624
<211>  6
<212>  PRT
<213>  Artificial Sequence

<220>
<223>  Synthetic peptide

<400>  624

Ser Gly Ser Thr Asn Val
1               5


<210>  625
<211>  6
<212>  PRT
<213>  Artificial Sequence

<220>
<223>  Synthetic peptide

<400>  625

Ser Gly Ser Thr Gln Val
1               5


<210>  626
<211>  6
<212>  PRT
<213>  Artificial Sequence

<220>
<223>  Synthetic peptide

<400>  626

Ser Gly Ser Val Ala Ser
1               5


<210>  627
<211>  6
<212>  PRT
<213>  Artificial Sequence

<220>
<223>  Synthetic peptide

<400>  627

Ser Gly Ser Val Asn Thr
1               5


<210>  628
<211>  6
<212>  PRT
<213>  Artificial Sequence

<220>
<223>  Synthetic peptide

<400>  628

Ser Gly Ser Val Gln Gly
1               5


<210>  629
<211>  6
<212>  PRT
<213>  Artificial Sequence

<220>
<223>  Synthetic peptide

<400>  629

Ser Gly Ser Val Gln Ser
1               5

<210>  630
<211>  6
<212>  PRT
<213>  Artificial Sequence

<220>
<223>  Synthetic peptide

<400>  630

Ser Asn Asn Gln Thr Val
1               5


<210>  631
<211>  6
<212>  PRT
<213>  Artificial Sequence

<220>
<223>  Synthetic peptide

<400>  631

Ser Asn Asn Thr Ala Val
1               5


<210>  632
<211>  6
<212>  PRT
<213>  Artificial Sequence

<220>
<223>  Synthetic peptide

<400>  632

Ser Asn Asn Thr Asn Val
1               5


<210>  633
<211>  6
<212>  PRT
<213>  Artificial Sequence

<220>
<223>  Synthetic peptide

<400>  633

Ser Asn Asn Thr Gln Val
1               5


<210>  634
<211>  6
<212>  PRT
<213>  Artificial Sequence

<220>
<223>  Synthetic peptide

<400>  634

Ser Asn Gln Gln Ser Gln
1               5


<210>  635
<211>  6
<212>  PRT
<213>  Artificial Sequence

<220>
<223>  Synthetic peptide

<400>  635

Ser Asn Gln Gln Ser Val
1               5


<210>  636
<211>  6
<212>  PRT
<213>  Artificial Sequence

<220>
<223>  Synthetic peptide

<400>  636

Ser Asn Gln Gln Thr Gln
1               5


<210>  637
<211>  6
<212>  PRT
<213>  Artificial Sequence

<220>
<223>  Synthetic peptide

<400>  637

Ser Asn Gln Gln Thr Val
1               5


<210>  638
<211>  6
<212>  PRT
<213>  Artificial Sequence

<220>
<223>  Synthetic peptide

<400>  638

Ser Asn Gln Ser Thr Val
1               5

<210>  639
<211>  6
<212>  PRT
<213>  Artificial Sequence

<220>
<223>  Synthetic peptide

<400>  639

Ser Asn Gln Thr Ser Val
1               5


<210>  640
<211>  6
<212>  PRT
<213>  Artificial Sequence

<220>
<223>  Synthetic peptide

<400>  640

Ser Asn Gln Val Ser Gly
1               5


<210>  641
<211>  6
<212>  PRT
<213>  Artificial Sequence

<220>
<223>  Synthetic peptide

<400>  641

Ser Asn Gln Val Ser Gln
1               5


<210>  642
<211>  6
<212>  PRT
<213>  Artificial Sequence

<220>
<223>  Synthetic peptide

<400>  642

Ser Asn Gln Val Ser Ser
1               5


<210>  643
<211>  6
<212>  PRT
<213>  Artificial Sequence

<220>
<223>  Synthetic peptide

<400>  643

Ser Asn Gln Val Ser Thr
1               5


<210>  644
<211>  6
<212>  PRT
<213>  Artificial Sequence

<220>
<223>  Synthetic peptide

<400>  644

Ser Asn Gln Val Thr Gly
1               5


<210>  645
<211>  6
<212>  PRT
<213>  Artificial Sequence

<220>
<223>  Synthetic peptide

<400>  645

Ser Asn Gln Val Thr Gln
1               5


<210>  646
<211>  6
<212>  PRT
<213>  Artificial Sequence

<220>
<223>  Synthetic peptide

<400>  646

Ser Asn Gln Val Thr Ser
1               5


<210>  647
<211>  6
<212>  PRT
<213>  Artificial Sequence

<220>
<223>  Synthetic peptide

<400>  647

Ser Asn Ser Gly Ala Val
1               5

<210>  648
<211>  6
<212>  PRT
<213>  Artificial Sequence

<220>
<223>  Synthetic peptide

<400>  648

Ser Asn Ser Asn Ala Val
1               5


<210>  649
<211>  6
<212>  PRT
<213>  Artificial Sequence

<220>
<223>  Synthetic peptide

<400>  649

Ser Asn Ser Asn Gly Val
1               5


<210>  650
<211>  6
<212>  PRT
<213>  Artificial Sequence

<220>
<223>  Synthetic peptide

<400>  650

Ser Asn Ser Asn Gln Ser
1               5


<210>  651
<211>  6
<212>  PRT
<213>  Artificial Sequence

<220>
<223>  Synthetic peptide

<400>  651

Ser Asn Ser Gln Ala Gly
1               5


<210>  652
<211>  6
<212>  PRT
<213>  Artificial Sequence

<220>
<223>  Synthetic peptide

<400>  652

Ser Asn Ser Gln Ala Gln
1               5


<210>  653
<211>  6
<212>  PRT
<213>  Artificial Sequence

<220>
<223>  Synthetic peptide

<400>  653

Ser Asn Ser Gln Ala Ser
1               5


<210>  654
<211>  6
<212>  PRT
<213>  Artificial Sequence

<220>
<223>  Synthetic peptide

<400>  654

Ser Asn Ser Gln Ala Thr
1               5


<210>  655
<211>  6
<212>  PRT
<213>  Artificial Sequence

<220>
<223>  Synthetic peptide

<400>  655

Ser Asn Ser Gln Ala Val
1               5


<210>  656
<211>  6
<212>  PRT
<213>  Artificial Sequence

<220>
<223>  Synthetic peptide

<400>  656

Ser Asn Ser Gln Gly Gln
1               5

<210>  657
<211>  6
<212>  PRT
<213>  Artificial Sequence

<220>
<223>  Synthetic peptide

<400>  657

Ser Asn Ser Gln Gly Val
1               5


<210>  658
<211>  6
<212>  PRT
<213>  Artificial Sequence

<220>
<223>  Synthetic peptide

<400>  658

Ser Asn Ser Gln Gln Ala
1               5


<210>  659
<211>  6
<212>  PRT
<213>  Artificial Sequence

<220>
<223>  Synthetic peptide

<400>  659

Ser Asn Ser Gln Gln Gly
1               5


<210>  660
<211>  6
<212>  PRT
<213>  Artificial Sequence

<220>
<223>  Synthetic peptide

<400>  660

Ser Asn Ser Gln Gln Gln
1               5


<210>  661
<211>  6
<212>  PRT
<213>  Artificial Sequence

<220>
<223>  Synthetic peptide

<400>  661

Ser Asn Ser Gln Gln Ser
1               5


<210>  662
<211>  6
<212>  PRT
<213>  Artificial Sequence

<220>
<223>  Synthetic peptide

<400>  662

Ser Asn Ser Gln Gln Thr
1               5


<210>  663
<211>  6
<212>  PRT
<213>  Artificial Sequence

<220>
<223>  Synthetic peptide

<400>  663

Ser Asn Ser Gln Gln Val
1               5


<210>  664
<211>  6
<212>  PRT
<213>  Artificial Sequence

<220>
<223>  Synthetic peptide

<400>  664

Ser Asn Ser Gln Ser Gly
1               5


<210>  665
<211>  6
<212>  PRT
<213>  Artificial Sequence

<220>
<223>  Synthetic peptide

<400>  665

Ser Asn Ser Gln Ser Thr
1               5

<210>  666
<211>  6
<212>  PRT
<213>  Artificial Sequence

<220>
<223>  Synthetic peptide

<400>  666

Ser Asn Ser Gln Ser Val
1               5


<210>  667
<211>  6
<212>  PRT
<213>  Artificial Sequence

<220>
<223>  Synthetic peptide

<400>  667

Ser Asn Ser Ser Ala Val
1               5


<210>  668
<211>  6
<212>  PRT
<213>  Artificial Sequence

<220>
<223>  Synthetic peptide

<400>  668

Ser Asn Ser Ser Gly Val
1               5


<210>  669
<211>  6
<212>  PRT
<213>  Artificial Sequence

<220>
<223>  Synthetic peptide

<400>  669

Ser Asn Ser Ser Gln Gly
1               5


<210>  670
<211>  6
<212>  PRT
<213>  Artificial Sequence

<220>
<223>  Synthetic peptide

<400>  670

Ser Asn Ser Ser Gln Gln
1               5


<210>  671
<211>  6
<212>  PRT
<213>  Artificial Sequence

<220>
<223>  Synthetic peptide

<400>  671

Ser Asn Ser Ser Gln Thr
1               5


<210>  672
<211>  6
<212>  PRT
<213>  Artificial Sequence

<220>
<223>  Synthetic peptide

<400>  672

Ser Asn Ser Ser Gln Val
1               5


<210>  673
<211>  6
<212>  PRT
<213>  Artificial Sequence

<220>
<223>  Synthetic peptide

<400>  673

Ser Asn Ser Ser Ser Gln
1               5


<210>  674
<211>  6
<212>  PRT
<213>  Artificial Sequence

<220>
<223>  Synthetic peptide

<400>  674

Ser Asn Ser Ser Ser Val
1               5

<210>  675
<211>  6
<212>  PRT
<213>  Artificial Sequence

<220>
<223>  Synthetic peptide

<400>  675

Ser Asn Ser Thr Ala Gly
1               5


<210>  676
<211>  6
<212>  PRT
<213>  Artificial Sequence

<220>
<223>  Synthetic peptide

<400>  676

Ser Asn Ser Thr Gly Ser
1               5


<210>  677
<211>  6
<212>  PRT
<213>  Artificial Sequence

<220>
<223>  Synthetic peptide

<400>  677

Ser Asn Ser Thr Asn Val
1               5


<210>  678
<211>  6
<212>  PRT
<213>  Artificial Sequence

<220>
<223>  Synthetic peptide

<400>  678

Ser Asn Ser Thr Gln Ala
1               5


<210>  679
<211>  6
<212>  PRT
<213>  Artificial Sequence

<220>
<223>  Synthetic peptide

<400>  679

Ser Asn Ser Thr Gln Gln
1               5


<210>  680
<211>  6
<212>  PRT
<213>  Artificial Sequence

<220>
<223>  Synthetic peptide

<400>  680

Ser Asn Ser Thr Gln Ser
1               5


<210>  681
<211>  6
<212>  PRT
<213>  Artificial Sequence

<220>
<223>  Synthetic peptide

<400>  681

Ser Asn Ser Thr Gln Val
1               5


<210>  682
<211>  6
<212>  PRT
<213>  Artificial Sequence

<220>
<223>  Synthetic peptide

<400>  682

Ser Asn Ser Thr Ser Ala
1               5


<210>  683
<211>  6
<212>  PRT
<213>  Artificial Sequence

<220>
<223>  Synthetic peptide

<400>  683

Ser Asn Ser Thr Ser Val
1               5

<210>  684
<211>  6
<212>  PRT
<213>  Artificial Sequence

<220>
<223>  Synthetic peptide

<400>  684

Ser Asn Ser Thr Val Gly
1               5


<210>  685
<211>  6
<212>  PRT
<213>  Artificial Sequence

<220>
<223>  Synthetic peptide

<400>  685

Ser Asn Ser Val Ala Gln
1               5


<210>  686
<211>  6
<212>  PRT
<213>  Artificial Sequence

<220>
<223>  Synthetic peptide

<400>  686

Ser Asn Ser Val Ala Ser
1               5


<210>  687
<211>  6
<212>  PRT
<213>  Artificial Sequence

<220>
<223>  Synthetic peptide

<400>  687

Ser Asn Ser Val Ala Thr
1               5


<210>  688
<211>  6
<212>  PRT
<213>  Artificial Sequence

<220>
<223>  Synthetic peptide

<400>  688

Ser Asn Ser Val Gly Gln
1               5


<210>  689
<211>  6
<212>  PRT
<213>  Artificial Sequence

<220>
<223>  Synthetic peptide

<400>  689

Ser Asn Ser Val Gly Ser
1               5


<210>  690
<211>  6
<212>  PRT
<213>  Artificial Sequence

<220>
<223>  Synthetic peptide

<400>  690

Ser Asn Ser Val Gly Thr
1               5


<210>  691
<211>  6
<212>  PRT
<213>  Artificial Sequence

<220>
<223>  Synthetic peptide

<400>  691

Ser Asn Ser Val Asn Ser
1               5


<210>  692
<211>  6
<212>  PRT
<213>  Artificial Sequence

<220>
<223>  Synthetic peptide

<400>  692

Ser Asn Ser Val Asn Thr
1               5

<210>  693
<211>  6
<212>  PRT
<213>  Artificial Sequence

<220>
<223>  Synthetic peptide

<400>  693

Ser Asn Ser Val Gln Gln
1               5


<210>  694
<211>  6
<212>  PRT
<213>  Artificial Sequence

<220>
<223>  Synthetic peptide

<400>  694

Ser Asn Ser Val Gln Ser
1               5


<210>  695
<211>  6
<212>  PRT
<213>  Artificial Sequence

<220>
<223>  Synthetic peptide

<400>  695

Ser Asn Ser Val Ser Gly
1               5


<210>  696
<211>  6
<212>  PRT
<213>  Artificial Sequence

<220>
<223>  Synthetic peptide

<400>  696

Ser Asn Ser Val Ser Gln
1               5


<210>  697
<211>  6
<212>  PRT
<213>  Artificial Sequence

<220>
<223>  Synthetic peptide

<400>  697

Ser Asn Ser Val Ser Ser
1               5


<210>  698
<211>  6
<212>  PRT
<213>  Artificial Sequence

<220>
<223>  Synthetic peptide

<400>  698

Ser Asn Ser Val Ser Thr
1               5


<210>  699
<211>  6
<212>  PRT
<213>  Artificial Sequence

<220>
<223>  Synthetic peptide

<400>  699

Ser Asn Ser Val Thr Gly
1               5


<210>  700
<211>  6
<212>  PRT
<213>  Artificial Sequence

<220>
<223>  Synthetic peptide

<400>  700

Ser Gln Asn Val Ala Ser
1               5


<210>  701
<211>  6
<212>  PRT
<213>  Artificial Sequence

<220>
<223>  Synthetic peptide

<400>  701

Ser Gln Asn Val Asn Ser
1               5

<210>  702
<211>  6
<212>  PRT
<213>  Artificial Sequence

<220>
<223>  Synthetic peptide

<400>  702

Ser Gln Gln Gln Ser Gln
1               5


<210>  703
<211>  6
<212>  PRT
<213>  Artificial Sequence

<220>
<223>  Synthetic peptide

<400>  703

Ser Gln Gln Gln Ser Val
1               5


<210>  704
<211>  6
<212>  PRT
<213>  Artificial Sequence

<220>
<223>  Synthetic peptide

<400>  704

Ser Gln Gln Gln Thr Gln
1               5


<210>  705
<211>  6
<212>  PRT
<213>  Artificial Sequence

<220>
<223>  Synthetic peptide

<400>  705

Ser Gln Gln Gln Thr Val
1               5


<210>  706
<211>  6
<212>  PRT
<213>  Artificial Sequence

<220>
<223>  Synthetic peptide

<400>  706

Ser Gln Gln Ser Thr Val
1               5


<210>  707
<211>  6
<212>  PRT
<213>  Artificial Sequence

<220>
<223>  Synthetic peptide

<400>  707

Ser Gln Gln Val Ser Gly
1               5


<210>  708
<211>  6
<212>  PRT
<213>  Artificial Sequence

<220>
<223>  Synthetic peptide

<400>  708

Ser Gln Gln Val Ser Asn
1               5


<210>  709
<211>  6
<212>  PRT
<213>  Artificial Sequence

<220>
<223>  Synthetic peptide

<400>  709

Ser Gln Gln Val Ser Ser
1               5


<210>  710
<211>  6
<212>  PRT
<213>  Artificial Sequence

<220>
<223>  Synthetic peptide

<400>  710

Ser Gln Gln Val Ser Thr
1               5

<210>  711
<211>  6
<212>  PRT
<213>  Artificial Sequence

<220>
<223>  Synthetic peptide

<400>  711

Ser Gln Gln Val Thr Gly
1               5


<210>  712
<211>  6
<212>  PRT
<213>  Artificial Sequence

<220>
<223>  Synthetic peptide

<400>  712

Ser Gln Gln Val Thr Asn
1               5


<210>  713
<211>  6
<212>  PRT
<213>  Artificial Sequence

<220>
<223>  Synthetic peptide

<400>  713

Ser Gln Gln Val Thr Ser
1               5


<210>  714
<211>  6
<212>  PRT
<213>  Artificial Sequence

<220>
<223>  Synthetic peptide

<400>  714

Ser Gln Gln Val Thr Thr
1               5


<210>  715
<211>  6
<212>  PRT
<213>  Artificial Sequence

<220>
<223>  Synthetic peptide

<400>  715

Ser Gln Ser Asn Ala Ser
1               5


<210>  716
<211>  6
<212>  PRT
<213>  Artificial Sequence

<220>
<223>  Synthetic peptide

<400>  716

Ser Gln Ser Asn Ala Val
1               5


<210>  717
<211>  6
<212>  PRT
<213>  Artificial Sequence

<220>
<223>  Synthetic peptide

<400>  717

Ser Gln Ser Asn Gly Val
1               5


<210>  718
<211>  6
<212>  PRT
<213>  Artificial Sequence

<220>
<223>  Synthetic peptide

<400>  718

Ser Gln Ser Asn Gln Thr
1               5


<210>  719
<211>  6
<212>  PRT
<213>  Artificial Sequence

<220>
<223>  Synthetic peptide

<400>  719

Ser Gln Ser Asn Gln Val
1               5

<210>  720
<211>  6
<212>  PRT
<213>  Artificial Sequence

<220>
<223>  Synthetic peptide

<400>  720

Ser Gln Ser Asn Ser Val
1               5


<210>  721
<211>  6
<212>  PRT
<213>  Artificial Sequence

<220>
<223>  Synthetic peptide

<400>  721

Ser Gln Ser Gln Ala Gln
1               5


<210>  722
<211>  6
<212>  PRT
<213>  Artificial Sequence

<220>
<223>  Synthetic peptide

<400>  722

Ser Gln Ser Gln Ala Ser
1               5


<210>  723
<211>  6
<212>  PRT
<213>  Artificial Sequence

<220>
<223>  Synthetic peptide

<400>  723

Ser Gln Ser Gln Ala Val
1               5


<210>  724
<211>  6
<212>  PRT
<213>  Artificial Sequence

<220>
<223>  Synthetic peptide

<400>  724

Ser Gln Ser Gln Gln Gln
1               5


<210>  725
<211>  6
<212>  PRT
<213>  Artificial Sequence

<220>
<223>  Synthetic peptide

<400>  725

Ser Gln Ser Gln Gln Val
1               5


<210>  726
<211>  6
<212>  PRT
<213>  Artificial Sequence

<220>
<223>  Synthetic peptide

<400>  726

Ser Gln Ser Gln Ser Gln
1               5


<210>  727
<211>  6
<212>  PRT
<213>  Artificial Sequence

<220>
<223>  Synthetic peptide

<400>  727

Ser Gln Ser Ser Ala Val
1               5


<210>  728
<211>  6
<212>  PRT
<213>  Artificial Sequence

<220>
<223>  Synthetic peptide

<400>  728

Ser Gln Ser Ser Gly Val
1               5

<210>  729
<211>  6
<212>  PRT
<213>  Artificial Sequence

<220>
<223>  Synthetic peptide

<400>  729

Ser Gln Ser Ser Gln Gly
1               5


<210>  730
<211>  6
<212>  PRT
<213>  Artificial Sequence

<220>
<223>  Synthetic peptide

<400>  730

Ser Gln Ser Ser Gln Val
1               5


<210>  731
<211>  6
<212>  PRT
<213>  Artificial Sequence

<220>
<223>  Synthetic peptide

<400>  731

Ser Gln Ser Ser Ser Gln
1               5


<210>  732
<211>  6
<212>  PRT
<213>  Artificial Sequence

<220>
<223>  Synthetic peptide

<400>  732

Ser Gln Ser Ser Ser Val
1               5


<210>  733
<211>  6
<212>  PRT
<213>  Artificial Sequence

<220>
<223>  Synthetic peptide

<400>  733

Ser Gln Ser Thr Ala Val
1               5


<210>  734
<211>  6
<212>  PRT
<213>  Artificial Sequence

<220>
<223>  Synthetic peptide

<400>  734

Ser Gln Ser Thr Gln Ser
1               5


<210>  735
<211>  6
<212>  PRT
<213>  Artificial Sequence

<220>
<223>  Synthetic peptide

<400>  735

Ser Gln Ser Thr Ser Gly
1               5


<210>  736
<211>  6
<212>  PRT
<213>  Artificial Sequence

<220>
<223>  Synthetic peptide

<400>  736

Ser Gln Ser Thr Ser Val
1               5


<210>  737
<211>  6
<212>  PRT
<213>  Artificial Sequence

<220>
<223>  Synthetic peptide

<400>  737

Ser Gln Ser Val Ala Gly
1               5

<210>  738
<211>  6
<212>  PRT
<213>  Artificial Sequence

<220>
<223>  Synthetic peptide

<400>  738

Ser Gln Ser Val Ala Asn
1               5


<210>  739
<211>  6
<212>  PRT
<213>  Artificial Sequence

<220>
<223>  Synthetic peptide

<400>  739

Ser Gln Ser Val Ala Gln
1               5


<210>  740
<211>  6
<212>  PRT
<213>  Artificial Sequence

<220>
<223>  Synthetic peptide

<400>  740

Ser Gln Ser Val Ala Ser
1               5


<210>  741
<211>  6
<212>  PRT
<213>  Artificial Sequence

<220>
<223>  Synthetic peptide

<400>  741

Ser Gln Ser Val Ala Thr
1               5


<210>  742
<211>  6
<212>  PRT
<213>  Artificial Sequence

<220>
<223>  Synthetic peptide

<400>  742

Ser Gln Ser Val Gly Gly
1               5


<210>  743
<211>  6
<212>  PRT
<213>  Artificial Sequence

<220>
<223>  Synthetic peptide

<400>  743

Ser Gln Ser Val Gly Asn
1               5


<210>  744
<211>  6
<212>  PRT
<213>  Artificial Sequence

<220>
<223>  Synthetic peptide

<400>  744

Ser Gln Ser Val Gly Gln
1               5


<210>  745
<211>  6
<212>  PRT
<213>  Artificial Sequence

<220>
<223>  Synthetic peptide

<400>  745

Ser Gln Ser Val Gly Ser
1               5


<210>  746
<211>  6
<212>  PRT
<213>  Artificial Sequence

<220>
<223>  Synthetic peptide

<400>  746

Ser Gln Ser Val Gly Thr
1               5

<210>  747
<211>  6
<212>  PRT
<213>  Artificial Sequence

<220>
<223>  Synthetic peptide

<400>  747

Ser Gln Ser Val Asn Gly
1               5


<210>  748
<211>  6
<212>  PRT
<213>  Artificial Sequence

<220>
<223>  Synthetic peptide

<400>  748

Ser Gln Ser Val Asn Ser
1               5


<210>  749
<211>  6
<212>  PRT
<213>  Artificial Sequence

<220>
<223>  Synthetic peptide

<400>  749

Ser Gln Ser Val Gln Gly
1               5


<210>  750
<211>  6
<212>  PRT
<213>  Artificial Sequence

<220>
<223>  Synthetic peptide

<400>  750

Ser Gln Ser Val Gln Asn
1               5


<210>  751
<211>  6
<212>  PRT
<213>  Artificial Sequence

<220>
<223>  Synthetic peptide

<400>  751

Ser Gln Ser Val Gln Gln
1               5


<210>  752
<211>  6
<212>  PRT
<213>  Artificial Sequence

<220>
<223>  Synthetic peptide

<400>  752

Ser Gln Ser Val Gln Ser
1               5


<210>  753
<211>  6
<212>  PRT
<213>  Artificial Sequence

<220>
<223>  Synthetic peptide

<400>  753

Ser Gln Ser Val Gln Thr
1               5


<210>  754
<211>  6
<212>  PRT
<213>  Artificial Sequence

<220>
<223>  Synthetic peptide

<400>  754

Ser Gln Ser Val Ser Gly
1               5


<210>  755
<211>  6
<212>  PRT
<213>  Artificial Sequence

<220>
<223>  Synthetic peptide

<400>  755

Ser Gln Ser Val Ser Asn
1               5

<210>  756
<211>  6
<212>  PRT
<213>  Artificial Sequence

<220>
<223>  Synthetic peptide

<400>  756

Ser Gln Ser Val Ser Ser
1               5


<210>  757
<211>  6
<212>  PRT
<213>  Artificial Sequence

<220>
<223>  Synthetic peptide

<400>  757

Ser Gln Ser Val Ser Thr
1               5


<210>  758
<211>  6
<212>  PRT
<213>  Artificial Sequence

<220>
<223>  Synthetic peptide

<400>  758

Ser Ser Asn Gly Thr Val
1               5


<210>  759
<211>  6
<212>  PRT
<213>  Artificial Sequence

<220>
<223>  Synthetic peptide

<400>  759

Ser Ser Asn Gln Asn Gln
1               5


<210>  760
<211>  6
<212>  PRT
<213>  Artificial Sequence

<220>
<223>  Synthetic peptide

<400>  760

Ser Ser Asn Gln Asn Val
1               5


<210>  761
<211>  6
<212>  PRT
<213>  Artificial Sequence

<220>
<223>  Synthetic peptide

<400>  761

Ser Ser Asn Gln Thr Gln
1               5


<210>  762
<211>  6
<212>  PRT
<213>  Artificial Sequence

<220>
<223>  Synthetic peptide

<400>  762

Ser Ser Asn Gln Thr Val
1               5


<210>  763
<211>  6
<212>  PRT
<213>  Artificial Sequence

<220>
<223>  Synthetic peptide

<400>  763

Ser Ser Asn Ser Thr Val
1               5


<210>  764
<211>  6
<212>  PRT
<213>  Artificial Sequence

<220>
<223>  Synthetic peptide

<400>  764

Ser Ser Asn Thr Asn Val
1               5

<210>  765
<211>  6
<212>  PRT
<213>  Artificial Sequence

<220>
<223>  Synthetic peptide

<400>  765

Ser Ser Asn Thr Gln Val
1               5


<210>  766
<211>  6
<212>  PRT
<213>  Artificial Sequence

<220>
<223>  Synthetic peptide

<400>  766

Ser Ser Asn Thr Val Gly
1               5


<210>  767
<211>  6
<212>  PRT
<213>  Artificial Sequence

<220>
<223>  Synthetic peptide

<400>  767

Ser Ser Asn Val Ala Gln
1               5


<210>  768
<211>  6
<212>  PRT
<213>  Artificial Sequence

<220>
<223>  Synthetic peptide

<400>  768

Ser Ser Asn Val Ala Ser
1               5


<210>  769
<211>  6
<212>  PRT
<213>  Artificial Sequence

<220>
<223>  Synthetic peptide

<400>  769

Ser Ser Asn Val Gly Thr
1               5


<210>  770
<211>  6
<212>  PRT
<213>  Artificial Sequence

<220>
<223>  Synthetic peptide

<400>  770

Ser Ser Asn Val Asn Gly
1               5


<210>  771
<211>  6
<212>  PRT
<213>  Artificial Sequence

<220>
<223>  Synthetic peptide

<400>  771

Ser Ser Asn Val Asn Gln
1               5


<210>  772
<211>  6
<212>  PRT
<213>  Artificial Sequence

<220>
<223>  Synthetic peptide

<400>  772

Ser Ser Asn Val Asn Ser
1               5


<210>  773
<211>  6
<212>  PRT
<213>  Artificial Sequence

<220>
<223>  Synthetic peptide

<400>  773

Ser Ser Asn Val Asn Thr
1               5

<210>  774
<211>  6
<212>  PRT
<213>  Artificial Sequence

<220>
<223>  Synthetic peptide

<400>  774

Ser Ser Asn Val Gln Ser
1               5


<210>  775
<211>  6
<212>  PRT
<213>  Artificial Sequence

<220>
<223>  Synthetic peptide

<400>  775

Ser Ser Asn Val Gln Thr
1               5


<210>  776
<211>  6
<212>  PRT
<213>  Artificial Sequence

<220>
<223>  Synthetic peptide

<400>  776

Ser Ser Asn Val Thr Gly
1               5


<210>  777
<211>  6
<212>  PRT
<213>  Artificial Sequence

<220>
<223>  Synthetic peptide

<400>  777

Ser Ser Asn Val Thr Gln
1               5


<210>  778
<211>  6
<212>  PRT
<213>  Artificial Sequence

<220>
<223>  Synthetic peptide

<400>  778

Ser Ser Asn Val Thr Ser
1               5


<210>  779
<211>  6
<212>  PRT
<213>  Artificial Sequence

<220>
<223>  Synthetic peptide

<400>  779

Ser Ser Gln Asn Thr Val
1               5


<210>  780
<211>  6
<212>  PRT
<213>  Artificial Sequence

<220>
<223>  Synthetic peptide

<400>  780

Ser Ser Gln Gln Ser Val
1               5


<210>  781
<211>  6
<212>  PRT
<213>  Artificial Sequence

<220>
<223>  Synthetic peptide

<400>  781

Ser Ser Gln Gln Thr Gln
1               5


<210>  782
<211>  6
<212>  PRT
<213>  Artificial Sequence

<220>
<223>  Synthetic peptide

<400>  782

Ser Ser Gln Gln Thr Val
1               5

<210>  783
<211>  6
<212>  PRT
<213>  Artificial Sequence

<220>
<223>  Synthetic peptide

<400>  783

Ser Ser Gln Ser Thr Gln
1               5


<210>  784
<211>  6
<212>  PRT
<213>  Artificial Sequence

<220>
<223>  Synthetic peptide

<400>  784

Ser Ser Gln Thr Ser Val
1               5


<210>  785
<211>  6
<212>  PRT
<213>  Artificial Sequence

<220>
<223>  Synthetic peptide

<400>  785

Ser Ser Gln Val Asn Ser
1               5


<210>  786
<211>  6
<212>  PRT
<213>  Artificial Sequence

<220>
<223>  Synthetic peptide

<400>  786

Ser Ser Gln Val Ser Asn
1               5


<210>  787
<211>  6
<212>  PRT
<213>  Artificial Sequence

<220>
<223>  Synthetic peptide

<400>  787

Ser Ser Gln Val Ser Gln
1               5


<210>  788
<211>  6
<212>  PRT
<213>  Artificial Sequence

<220>
<223>  Synthetic peptide

<400>  788

Ser Ser Gln Val Ser Ser
1               5


<210>  789
<211>  6
<212>  PRT
<213>  Artificial Sequence

<220>
<223>  Synthetic peptide

<400>  789

Ser Ser Gln Val Ser Thr
1               5


<210>  790
<211>  6
<212>  PRT
<213>  Artificial Sequence

<220>
<223>  Synthetic peptide

<400>  790

Ser Ser Gln Val Thr Gly
1               5


<210>  791
<211>  6
<212>  PRT
<213>  Artificial Sequence

<220>
<223>  Synthetic peptide

<400>  791

Ser Ser Gln Val Thr Gln
1               5

<210>  792
<211>  6
<212>  PRT
<213>  Artificial Sequence

<220>
<223>  Synthetic peptide

<400>  792

Ser Ser Gln Val Thr Ser
1               5


<210>  793
<211>  6
<212>  PRT
<213>  Artificial Sequence

<220>
<223>  Synthetic peptide

<400>  793

Ser Ser Gln Val Thr Thr
1               5


<210>  794
<211>  6
<212>  PRT
<213>  Artificial Sequence

<220>
<223>  Synthetic peptide

<400>  794

Ser Ser Ser Asn Ala Val
1               5


<210>  795
<211>  6
<212>  PRT
<213>  Artificial Sequence

<220>
<223>  Synthetic peptide

<400>  795

Ser Ser Ser Asn Gln Val
1               5


<210>  796
<211>  6
<212>  PRT
<213>  Artificial Sequence

<220>
<223>  Synthetic peptide

<400>  796

Ser Ser Ser Asn Ser Val
1               5


<210>  797
<211>  6
<212>  PRT
<213>  Artificial Sequence

<220>
<223>  Synthetic peptide

<400>  797

Ser Ser Ser Gln Ala Gln
1               5


<210>  798
<211>  6
<212>  PRT
<213>  Artificial Sequence

<220>
<223>  Synthetic peptide

<400>  798

Ser Ser Ser Gln Asn Gln
1               5


<210>  799
<211>  6
<212>  PRT
<213>  Artificial Sequence

<220>
<223>  Synthetic peptide

<400>  799

Ser Ser Ser Gln Gln Gln
1               5


<210>  800
<211>  6
<212>  PRT
<213>  Artificial Sequence

<220>
<223>  Synthetic peptide

<400>  800

Ser Ser Ser Gln Gln Thr
1               5

<210>  801
<211>  6
<212>  PRT
<213>  Artificial Sequence

<220>
<223>  Synthetic peptide

<400>  801

Ser Ser Ser Gln Gln Val
1               5


<210>  802
<211>  6
<212>  PRT
<213>  Artificial Sequence

<220>
<223>  Synthetic peptide

<400>  802

Ser Ser Ser Gln Ser Gln
1               5


<210>  803
<211>  6
<212>  PRT
<213>  Artificial Sequence

<220>
<223>  Synthetic peptide

<400>  803

Ser Ser Ser Gln Ser Val
1               5


<210>  804
<211>  6
<212>  PRT
<213>  Artificial Sequence

<220>
<223>  Synthetic peptide

<400>  804

Ser Ser Ser Ser Gly Gln
1               5


<210>  805
<211>  6
<212>  PRT
<213>  Artificial Sequence

<220>
<223>  Synthetic peptide

<400>  805

Ser Ser Ser Ser Gly Val
1               5


<210>  806
<211>  6
<212>  PRT
<213>  Artificial Sequence

<220>
<223>  Synthetic peptide

<400>  806

Ser Ser Ser Ser Asn Val
1               5


<210>  807
<211>  6
<212>  PRT
<213>  Artificial Sequence

<220>
<223>  Synthetic peptide

<400>  807

Ser Ser Ser Ser Gln Gln
1               5


<210>  808
<211>  6
<212>  PRT
<213>  Artificial Sequence

<220>
<223>  Synthetic peptide

<400>  808

Ser Ser Ser Ser Gln Ser
1               5


<210>  809
<211>  6
<212>  PRT
<213>  Artificial Sequence

<220>
<223>  Synthetic peptide

<400>  809

Ser Ser Ser Thr Ala Val
1               5

<210>  810
<211>  6
<212>  PRT
<213>  Artificial Sequence

<220>
<223>  Synthetic peptide

<400>  810

Ser Ser Ser Thr Asn Val
1               5


<210>  811
<211>  6
<212>  PRT
<213>  Artificial Sequence

<220>
<223>  Synthetic peptide

<400>  811

Ser Ser Ser Thr Ser Val
1               5


<210>  812
<211>  6
<212>  PRT
<213>  Artificial Sequence

<220>
<223>  Synthetic peptide

<400>  812

Ser Ser Ser Val Ala Gln
1               5


<210>  813
<211>  6
<212>  PRT
<213>  Artificial Sequence

<220>
<223>  Synthetic peptide

<400>  813

Ser Ser Ser Val Ala Ser
1               5


<210>  814
<211>  6
<212>  PRT
<213>  Artificial Sequence

<220>
<223>  Synthetic peptide

<400>  814

Ser Ser Ser Val Gly Gly
1               5


<210>  815
<211>  6
<212>  PRT
<213>  Artificial Sequence

<220>
<223>  Synthetic peptide

<400>  815

Ser Ser Ser Val Gly Gln
1               5


<210>  816
<211>  6
<212>  PRT
<213>  Artificial Sequence

<220>
<223>  Synthetic peptide

<400>  816

Ser Ser Ser Val Gly Ser
1               5


<210>  817
<211>  6
<212>  PRT
<213>  Artificial Sequence

<220>
<223>  Synthetic peptide

<400>  817

Ser Ser Ser Val Asn Gly
1               5


<210>  818
<211>  6
<212>  PRT
<213>  Artificial Sequence

<220>
<223>  Synthetic peptide

<400>  818

Ser Ser Ser Val Asn Ser
1               5

<210>  819
<211>  6
<212>  PRT
<213>  Artificial Sequence

<220>
<223>  Synthetic peptide

<400>  819

Ser Ser Ser Val Asn Thr
1               5


<210>  820
<211>  6
<212>  PRT
<213>  Artificial Sequence

<220>
<223>  Synthetic peptide

<400>  820

Ser Ser Ser Val Gln Gly
1               5


<210>  821
<211>  6
<212>  PRT
<213>  Artificial Sequence

<220>
<223>  Synthetic peptide

<400>  821

Ser Ser Ser Val Gln Asn
1               5


<210>  822
<211>  6
<212>  PRT
<213>  Artificial Sequence

<220>
<223>  Synthetic peptide

<400>  822

Ser Ser Ser Val Gln Ser
1               5


<210>  823
<211>  6
<212>  PRT
<213>  Artificial Sequence

<220>
<223>  Synthetic peptide

<400>  823

Ser Ser Ser Val Gln Thr
1               5


<210>  824
<211>  6
<212>  PRT
<213>  Artificial Sequence

<220>
<223>  Synthetic peptide

<400>  824

Ser Ser Ser Val Ser Gly
1               5


<210>  825
<211>  6
<212>  PRT
<213>  Artificial Sequence

<220>
<223>  Synthetic peptide

<400>  825

Ser Ser Ser Val Ser Asn
1               5


<210>  826
<211>  6
<212>  PRT
<213>  Artificial Sequence

<220>
<223>  Synthetic peptide

<400>  826

Ser Ser Ser Val Ser Gln
1               5


<210>  827
<211>  6
<212>  PRT
<213>  Artificial Sequence

<220>
<223>  Synthetic peptide

<400>  827

Ser Ser Ser Val Ser Ser
1               5

<210>  828
<211>  6
<212>  PRT
<213>  Artificial Sequence

<220>
<223>  Synthetic peptide

<400>  828

Ser Ser Ser Val Ser Thr
1               5


<210>  829
<211>  6
<212>  PRT
<213>  Artificial Sequence

<220>
<223>  Synthetic peptide

<400>  829

Ser Thr Asn Gly Ser Val
1               5


<210>  830
<211>  6
<212>  PRT
<213>  Artificial Sequence

<220>
<223>  Synthetic peptide

<400>  830

Ser Thr Asn Ser Gly Val
1               5


<210>  831
<211>  6
<212>  PRT
<213>  Artificial Sequence

<220>
<223>  Synthetic peptide

<400>  831

Ser Thr Asn Ser Asn Val
1               5


<210>  832
<211>  6
<212>  PRT
<213>  Artificial Sequence

<220>
<223>  Synthetic peptide

<400>  832

Ser Thr Asn Thr Asn Val
1               5


<210>  833
<211>  6
<212>  PRT
<213>  Artificial Sequence

<220>
<223>  Synthetic peptide

<400>  833

Ser Thr Asn Thr Gln Val
1               5


<210>  834
<211>  6
<212>  PRT
<213>  Artificial Sequence

<220>
<223>  Synthetic peptide

<400>  834

Ser Thr Asn Val Gly Ser
1               5


<210>  835
<211>  6
<212>  PRT
<213>  Artificial Sequence

<220>
<223>  Synthetic peptide

<400>  835

Ser Thr Asn Val Asn Gly
1               5


<210>  836
<211>  6
<212>  PRT
<213>  Artificial Sequence

<220>
<223>  Synthetic peptide

<400>  836

Ser Thr Asn Val Asn Ser
1               5

<210>  837
<211>  6
<212>  PRT
<213>  Artificial Sequence

<220>
<223>  Synthetic peptide

<400>  837

Ser Thr Asn Val Ser Gly
1               5


<210>  838
<211>  6
<212>  PRT
<213>  Artificial Sequence

<220>
<223>  Synthetic peptide

<400>  838

Ser Thr Asn Val Ser Ser
1               5


<210>  839
<211>  6
<212>  PRT
<213>  Artificial Sequence

<220>
<223>  Synthetic peptide

<400>  839

Ser Thr Asn Val Thr Gly
1               5


<210>  840
<211>  6
<212>  PRT
<213>  Artificial Sequence

<220>
<223>  Synthetic peptide

<400>  840

Ser Thr Asn Val Thr Ser
1               5


<210>  841
<211>  6
<212>  PRT
<213>  Artificial Sequence

<220>
<223>  Synthetic peptide

<400>  841

Ser Thr Gln Gln Ser Gly
1               5


<210>  842
<211>  6
<212>  PRT
<213>  Artificial Sequence

<220>
<223>  Synthetic peptide

<400>  842

Ser Thr Gln Gln Ser Gln
1               5


<210>  843
<211>  6
<212>  PRT
<213>  Artificial Sequence

<220>
<223>  Synthetic peptide

<400>  843

Ser Thr Gln Gln Ser Val
1               5


<210>  844
<211>  6
<212>  PRT
<213>  Artificial Sequence

<220>
<223>  Synthetic peptide

<400>  844

Ser Thr Gln Gln Thr Gln
1               5


<210>  845
<211>  6
<212>  PRT
<213>  Artificial Sequence

<220>
<223>  Synthetic peptide

<400>  845

Ser Thr Gln Gln Thr Ser
1               5

<210>  846
<211>  6
<212>  PRT
<213>  Artificial Sequence

<220>
<223>  Synthetic peptide

<400>  846

Ser Thr Gln Gln Thr Val
1               5


<210>  847
<211>  6
<212>  PRT
<213>  Artificial Sequence

<220>
<223>  Synthetic peptide

<400>  847

Ser Thr Gln Ser Asn Val
1               5


<210>  848
<211>  6
<212>  PRT
<213>  Artificial Sequence

<220>
<223>  Synthetic peptide

<400>  848

Ser Thr Gln Ser Thr Val
1               5


<210>  849
<211>  6
<212>  PRT
<213>  Artificial Sequence

<220>
<223>  Synthetic peptide

<400>  849

Ser Thr Gln Thr Ser Ser
1               5


<210>  850
<211>  6
<212>  PRT
<213>  Artificial Sequence

<220>
<223>  Synthetic peptide

<400>  850

Ser Thr Gln Thr Ser Val
1               5


<210>  851
<211>  6
<212>  PRT
<213>  Artificial Sequence

<220>
<223>  Synthetic peptide

<400>  851

Ser Thr Gln Val Asn Ser
1               5


<210>  852
<211>  6
<212>  PRT
<213>  Artificial Sequence

<220>
<223>  Synthetic peptide

<400>  852

Ser Thr Gln Val Ser Gly
1               5


<210>  853
<211>  6
<212>  PRT
<213>  Artificial Sequence

<220>
<223>  Synthetic peptide

<400>  853

Ser Thr Gln Val Ser Asn
1               5


<210>  854
<211>  6
<212>  PRT
<213>  Artificial Sequence

<220>
<223>  Synthetic peptide

<400>  854

Ser Thr Gln Val Ser Thr
1               5

<210>  855
<211>  6
<212>  PRT
<213>  Artificial Sequence

<220>
<223>  Synthetic peptide

<400>  855

Ser Thr Gln Val Thr Gly
1               5


<210>  856
<211>  6
<212>  PRT
<213>  Artificial Sequence

<220>
<223>  Synthetic peptide

<400>  856

Ser Thr Gln Val Thr Asn
1               5


<210>  857
<211>  6
<212>  PRT
<213>  Artificial Sequence

<220>
<223>  Synthetic peptide

<400>  857

Ser Thr Gln Val Thr Gln
1               5


<210>  858
<211>  6
<212>  PRT
<213>  Artificial Sequence

<220>
<223>  Synthetic peptide

<400>  858

Ser Thr Gln Val Thr Ser
1               5


<210>  859
<211>  6
<212>  PRT
<213>  Artificial Sequence

<220>
<223>  Synthetic peptide

<400>  859

Ser Thr Gln Val Thr Thr
1               5


<210>  860
<211>  6
<212>  PRT
<213>  Artificial Sequence

<220>
<223>  Synthetic peptide

<400>  860

Ser Thr Ser Gly Asn Val
1               5


<210>  861
<211>  6
<212>  PRT
<213>  Artificial Sequence

<220>
<223>  Synthetic peptide

<400>  861

Ser Thr Ser Asn Ala Ser
1               5


<210>  862
<211>  6
<212>  PRT
<213>  Artificial Sequence

<220>
<223>  Synthetic peptide

<400>  862

Ser Thr Ser Asn Gly Val
1               5


<210>  863
<211>  6
<212>  PRT
<213>  Artificial Sequence

<220>
<223>  Synthetic peptide

<400>  863

Ser Thr Ser Asn Gln Val
1               5

<210>  864
<211>  6
<212>  PRT
<213>  Artificial Sequence

<220>
<223>  Synthetic peptide

<400>  864

Ser Thr Ser Asn Ser Val
1               5


<210>  865
<211>  6
<212>  PRT
<213>  Artificial Sequence

<220>
<223>  Synthetic peptide

<400>  865

Ser Thr Ser Gln Ala Gln
1               5


<210>  866
<211>  6
<212>  PRT
<213>  Artificial Sequence

<220>
<223>  Synthetic peptide

<400>  866

Ser Thr Ser Gln Gly Val
1               5


<210>  867
<211>  6
<212>  PRT
<213>  Artificial Sequence

<220>
<223>  Synthetic peptide

<400>  867

Ser Thr Ser Gln Asn Gln
1               5


<210>  868
<211>  6
<212>  PRT
<213>  Artificial Sequence

<220>
<223>  Synthetic peptide

<400>  868

Ser Thr Ser Gln Gln Gln
1               5


<210>  869
<211>  6
<212>  PRT
<213>  Artificial Sequence

<220>
<223>  Synthetic peptide

<400>  869

Ser Thr Ser Gln Gln Val
1               5


<210>  870
<211>  6
<212>  PRT
<213>  Artificial Sequence

<220>
<223>  Synthetic peptide

<400>  870

Ser Thr Ser Gln Ser Gln
1               5


<210>  871
<211>  6
<212>  PRT
<213>  Artificial Sequence

<220>
<223>  Synthetic peptide

<400>  871

Ser Thr Ser Ser Ala Val
1               5


<210>  872
<211>  6
<212>  PRT
<213>  Artificial Sequence

<220>
<223>  Synthetic peptide

<400>  872

Ser Thr Ser Ser Gly Val
1               5

<210>  873
<211>  6
<212>  PRT
<213>  Artificial Sequence

<220>
<223>  Synthetic peptide

<400>  873

Ser Thr Ser Ser Gln Gln
1               5


<210>  874
<211>  6
<212>  PRT
<213>  Artificial Sequence

<220>
<223>  Synthetic peptide

<400>  874

Ser Thr Ser Ser Gln Ser
1               5


<210>  875
<211>  6
<212>  PRT
<213>  Artificial Sequence

<220>
<223>  Synthetic peptide

<400>  875

Ser Thr Ser Ser Gln Val
1               5


<210>  876
<211>  6
<212>  PRT
<213>  Artificial Sequence

<220>
<223>  Synthetic peptide

<400>  876

Ser Thr Ser Ser Ser Gln
1               5


<210>  877
<211>  6
<212>  PRT
<213>  Artificial Sequence

<220>
<223>  Synthetic peptide

<400>  877

Ser Thr Ser Thr Ala Val
1               5


<210>  878
<211>  6
<212>  PRT
<213>  Artificial Sequence

<220>
<223>  Synthetic peptide

<400>  878

Ser Thr Ser Thr Gly Val
1               5


<210>  879
<211>  6
<212>  PRT
<213>  Artificial Sequence

<220>
<223>  Synthetic peptide

<400>  879

Ser Thr Ser Thr Gln Ala
1               5


<210>  880
<211>  6
<212>  PRT
<213>  Artificial Sequence

<220>
<223>  Synthetic peptide

<400>  880

Ser Thr Ser Thr Gln Gly
1               5


<210>  881
<211>  6
<212>  PRT
<213>  Artificial Sequence

<220>
<223>  Synthetic peptide

<400>  881

Ser Thr Ser Thr Gln Thr
1               5

<210>  882
<211>  6
<212>  PRT
<213>  Artificial Sequence

<220>
<223>  Synthetic peptide

<400>  882

Ser Thr Ser Thr Gln Val
1               5


<210>  883
<211>  6
<212>  PRT
<213>  Artificial Sequence

<220>
<223>  Synthetic peptide

<400>  883

Ser Thr Ser Thr Ser Gln
1               5


<210>  884
<211>  6
<212>  PRT
<213>  Artificial Sequence

<220>
<223>  Synthetic peptide

<400>  884

Ser Thr Ser Thr Ser Val
1               5


<210>  885
<211>  6
<212>  PRT
<213>  Artificial Sequence

<220>
<223>  Synthetic peptide

<400>  885

Ser Thr Ser Val Ala Gly
1               5


<210>  886
<211>  6
<212>  PRT
<213>  Artificial Sequence

<220>
<223>  Synthetic peptide

<400>  886

Ser Thr Ser Val Ala Asn
1               5


<210>  887
<211>  6
<212>  PRT
<213>  Artificial Sequence

<220>
<223>  Synthetic peptide

<400>  887

Ser Thr Ser Val Ala Gln
1               5


<210>  888
<211>  6
<212>  PRT
<213>  Artificial Sequence

<220>
<223>  Synthetic peptide

<400>  888

Ser Thr Ser Val Ala Ser
1               5


<210>  889
<211>  6
<212>  PRT
<213>  Artificial Sequence

<220>
<223>  Synthetic peptide

<400>  889

Ser Thr Ser Val Ala Thr
1               5


<210>  890
<211>  6
<212>  PRT
<213>  Artificial Sequence

<220>
<223>  Synthetic peptide

<400>  890

Ser Thr Ser Val Gly Gly
1               5

<210>  891
<211>  6
<212>  PRT
<213>  Artificial Sequence

<220>
<223>  Synthetic peptide

<400>  891

Ser Thr Ser Val Gly Asn
1               5


<210>  892
<211>  6
<212>  PRT
<213>  Artificial Sequence

<220>
<223>  Synthetic peptide

<400>  892

Ser Thr Ser Val Gly Gln
1               5


<210>  893
<211>  6
<212>  PRT
<213>  Artificial Sequence

<220>
<223>  Synthetic peptide

<400>  893

Ser Thr Ser Val Gly Ser
1               5


<210>  894
<211>  6
<212>  PRT
<213>  Artificial Sequence

<220>
<223>  Synthetic peptide

<400>  894

Ser Thr Ser Val Asn Gly
1               5


<210>  895
<211>  6
<212>  PRT
<213>  Artificial Sequence

<220>
<223>  Synthetic peptide

<400>  895

Ser Thr Ser Val Asn Asn
1               5


<210>  896
<211>  6
<212>  PRT
<213>  Artificial Sequence

<220>
<223>  Synthetic peptide

<400>  896

Ser Thr Ser Val Asn Ser
1               5


<210>  897
<211>  6
<212>  PRT
<213>  Artificial Sequence

<220>
<223>  Synthetic peptide

<400>  897

Ser Thr Ser Val Gln Gly
1               5


<210>  898
<211>  6
<212>  PRT
<213>  Artificial Sequence

<220>
<223>  Synthetic peptide

<400>  898

Ser Thr Ser Val Gln Asn
1               5


<210>  899
<211>  6
<212>  PRT
<213>  Artificial Sequence

<220>
<223>  Synthetic peptide

<400>  899

Ser Thr Ser Val Gln Gln
1               5

<210>  900
<211>  6
<212>  PRT
<213>  Artificial Sequence

<220>
<223>  Synthetic peptide

<400>  900

Ser Thr Ser Val Gln Ser
1               5


<210>  901
<211>  6
<212>  PRT
<213>  Artificial Sequence

<220>
<223>  Synthetic peptide

<400>  901

Ser Thr Ser Val Gln Thr
1               5


<210>  902
<211>  6
<212>  PRT
<213>  Artificial Sequence

<220>
<223>  Synthetic peptide

<400>  902

Ser Thr Ser Val Ser Gly
1               5


<210>  903
<211>  6
<212>  PRT
<213>  Artificial Sequence

<220>
<223>  Synthetic peptide

<400>  903

Ser Thr Ser Val Ser Asn
1               5


<210>  904
<211>  6
<212>  PRT
<213>  Artificial Sequence

<220>
<223>  Synthetic peptide

<400>  904

Ser Thr Ser Val Ser Gln
1               5


<210>  905
<211>  6
<212>  PRT
<213>  Artificial Sequence

<220>
<223>  Synthetic peptide

<400>  905

Ser Thr Ser Val Ser Ser
1               5


<210>  906
<211>  6
<212>  PRT
<213>  Artificial Sequence

<220>
<223>  Synthetic peptide

<400>  906

Ser Thr Ser Val Ser Thr
1               5


<210>  907
<211>  6
<212>  PRT
<213>  Artificial Sequence

<220>
<223>  Synthetic peptide

<400>  907

Ser Val Asn Gly Ser Thr
1               5


<210>  908
<211>  6
<212>  PRT
<213>  Artificial Sequence

<220>
<223>  Synthetic peptide

<400>  908

Ser Val Asn Gly Thr Ser
1               5

<210>  909
<211>  6
<212>  PRT
<213>  Artificial Sequence

<220>
<223>  Synthetic peptide

<400>  909

Ser Val Asn Gln Ala Gln
1               5


<210>  910
<211>  6
<212>  PRT
<213>  Artificial Sequence

<220>
<223>  Synthetic peptide

<400>  910

Ser Val Asn Gln Gln Gln
1               5


<210>  911
<211>  6
<212>  PRT
<213>  Artificial Sequence

<220>
<223>  Synthetic peptide

<400>  911

Ser Val Asn Ser Gly Thr
1               5


<210>  912
<211>  6
<212>  PRT
<213>  Artificial Sequence

<220>
<223>  Synthetic peptide

<400>  912

Ser Val Asn Ser Asn Gln
1               5


<210>  913
<211>  6
<212>  PRT
<213>  Artificial Sequence

<220>
<223>  Synthetic peptide

<400>  913

Ser Val Asn Ser Asn Ser
1               5


<210>  914
<211>  6
<212>  PRT
<213>  Artificial Sequence

<220>
<223>  Synthetic peptide

<400>  914

Ser Val Asn Ser Thr Gly
1               5


<210>  915
<211>  6
<212>  PRT
<213>  Artificial Sequence

<220>
<223>  Synthetic peptide

<400>  915

Ser Val Asn Ser Thr Ser
1               5


<210>  916
<211>  6
<212>  PRT
<213>  Artificial Sequence

<220>
<223>  Synthetic peptide

<400>  916

Ser Val Asn Thr Gly Ser
1               5


<210>  917
<211>  6
<212>  PRT
<213>  Artificial Sequence

<220>
<223>  Synthetic peptide

<400>  917

Ser Val Asn Thr Ser Gly
1               5

<210>  918
<211>  6
<212>  PRT
<213>  Artificial Sequence

<220>
<223>  Synthetic peptide

<400>  918

Ser Val Gln Gln Ser Gln
1               5


<210>  919
<211>  6
<212>  PRT
<213>  Artificial Sequence

<220>
<223>  Synthetic peptide

<400>  919

Ser Val Gln Gln Ser Thr
1               5


<210>  920
<211>  6
<212>  PRT
<213>  Artificial Sequence

<220>
<223>  Synthetic peptide

<400>  920

Ser Val Gln Gln Thr Gln
1               5


<210>  921
<211>  6
<212>  PRT
<213>  Artificial Sequence

<220>
<223>  Synthetic peptide

<400>  921

Ser Val Gln Gln Thr Ser
1               5


<210>  922
<211>  6
<212>  PRT
<213>  Artificial Sequence

<220>
<223>  Synthetic peptide

<400>  922

Ser Val Gln Gln Thr Thr
1               5


<210>  923
<211>  6
<212>  PRT
<213>  Artificial Sequence

<220>
<223>  Synthetic peptide

<400>  923

Ser Val Gln Ser Asn Gln
1               5


<210>  924
<211>  6
<212>  PRT
<213>  Artificial Sequence

<220>
<223>  Synthetic peptide

<400>  924

Ser Val Gln Ser Asn Ser
1               5


<210>  925
<211>  6
<212>  PRT
<213>  Artificial Sequence

<220>
<223>  Synthetic peptide

<400>  925

Ser Val Gln Ser Ser Gly
1               5


<210>  926
<211>  6
<212>  PRT
<213>  Artificial Sequence

<220>
<223>  Synthetic peptide

<400>  926

Ser Val Gln Ser Ser Gln
1               5

<210>  927
<211>  6
<212>  PRT
<213>  Artificial Sequence

<220>
<223>  Synthetic peptide

<400>  927

Ser Val Gln Ser Ser Ser
1               5


<210>  928
<211>  6
<212>  PRT
<213>  Artificial Sequence

<220>
<223>  Synthetic peptide

<400>  928

Ser Val Gln Ser Thr Gly
1               5


<210>  929
<211>  6
<212>  PRT
<213>  Artificial Sequence

<220>
<223>  Synthetic peptide

<400>  929

Ser Val Gln Ser Thr Gln
1               5


<210>  930
<211>  6
<212>  PRT
<213>  Artificial Sequence

<220>
<223>  Synthetic peptide

<400>  930

Ser Val Gln Ser Thr Ser
1               5


<210>  931
<211>  6
<212>  PRT
<213>  Artificial Sequence

<220>
<223>  Synthetic peptide

<400>  931

Ser Val Gln Thr Ser Gly
1               5


<210>  932
<211>  6
<212>  PRT
<213>  Artificial Sequence

<220>
<223>  Synthetic peptide

<400>  932

Ser Val Gln Thr Ser Asn
1               5


<210>  933
<211>  6
<212>  PRT
<213>  Artificial Sequence

<220>
<223>  Synthetic peptide

<400>  933

Ser Val Gln Thr Ser Ser
1               5


<210>  934
<211>  6
<212>  PRT
<213>  Artificial Sequence

<220>
<223>  Synthetic peptide

<400>  934

Ser Val Gln Val Ser Asn
1               5


<210>  935
<211>  6
<212>  PRT
<213>  Artificial Sequence

<220>
<223>  Synthetic peptide

<400>  935

Ser Val Ser Gly Asn Thr
1               5

<210>  936
<211>  6
<212>  PRT
<213>  Artificial Sequence

<220>
<223>  Synthetic peptide

<400>  936

Ser Val Ser Gly Gln Ser
1               5


<210>  937
<211>  6
<212>  PRT
<213>  Artificial Sequence

<220>
<223>  Synthetic peptide

<400>  937

Ser Val Ser Asn Ala Gln
1               5


<210>  938
<211>  6
<212>  PRT
<213>  Artificial Sequence

<220>
<223>  Synthetic peptide

<400>  938

Ser Val Ser Asn Ala Ser
1               5


<210>  939
<211>  6
<212>  PRT
<213>  Artificial Sequence

<220>
<223>  Synthetic peptide

<400>  939

Ser Val Ser Asn Gly Ser
1               5


<210>  940
<211>  6
<212>  PRT
<213>  Artificial Sequence

<220>
<223>  Synthetic peptide

<400>  940

Ser Val Ser Asn Gly Thr
1               5


<210>  941
<211>  6
<212>  PRT
<213>  Artificial Sequence

<220>
<223>  Synthetic peptide

<400>  941

Ser Val Ser Asn Gln Gly
1               5


<210>  942
<211>  6
<212>  PRT
<213>  Artificial Sequence

<220>
<223>  Synthetic peptide

<400>  942

Ser Val Ser Asn Gln Ser
1               5


<210>  943
<211>  6
<212>  PRT
<213>  Artificial Sequence

<220>
<223>  Synthetic peptide

<400>  943

Ser Val Ser Asn Gln Thr
1               5


<210>  944
<211>  6
<212>  PRT
<213>  Artificial Sequence

<220>
<223>  Synthetic peptide

<400>  944

Ser Val Ser Asn Ser Thr
1               5

<210>  945
<211>  6
<212>  PRT
<213>  Artificial Sequence

<220>
<223>  Synthetic peptide

<400>  945

Ser Val Ser Asn Thr Gly
1               5


<210>  946
<211>  6
<212>  PRT
<213>  Artificial Sequence

<220>
<223>  Synthetic peptide

<400>  946

Ser Val Ser Gln Ala Gln
1               5


<210>  947
<211>  6
<212>  PRT
<213>  Artificial Sequence

<220>
<223>  Synthetic peptide

<400>  947

Ser Val Ser Gln Ala Ser
1               5


<210>  948
<211>  6
<212>  PRT
<213>  Artificial Sequence

<220>
<223>  Synthetic peptide

<400>  948

Ser Val Ser Gln Gly Gln
1               5


<210>  949
<211>  6
<212>  PRT
<213>  Artificial Sequence

<220>
<223>  Synthetic peptide

<400>  949

Ser Val Ser Gln Asn Gln
1               5


<210>  950
<211>  6
<212>  PRT
<213>  Artificial Sequence

<220>
<223>  Synthetic peptide

<400>  950

Ser Val Ser Gln Gln Gly
1               5


<210>  951
<211>  6
<212>  PRT
<213>  Artificial Sequence

<220>
<223>  Synthetic peptide

<400>  951

Ser Val Ser Gln Gln Gln
1               5


<210>  952
<211>  6
<212>  PRT
<213>  Artificial Sequence

<220>
<223>  Synthetic peptide

<400>  952

Ser Val Ser Gln Gln Ser
1               5


<210>  953
<211>  6
<212>  PRT
<213>  Artificial Sequence

<220>
<223>  Synthetic peptide

<400>  953

Ser Val Ser Gln Ser Gly
1               5

<210>  954
<211>  6
<212>  PRT
<213>  Artificial Sequence

<220>
<223>  Synthetic peptide

<400>  954

Ser Val Ser Gln Ser Ser
1               5


<210>  955
<211>  6
<212>  PRT
<213>  Artificial Sequence

<220>
<223>  Synthetic peptide

<400>  955

Ser Val Ser Gln Thr Gln
1               5


<210>  956
<211>  6
<212>  PRT
<213>  Artificial Sequence

<220>
<223>  Synthetic peptide

<400>  956

Ser Val Ser Ser Ala Gln
1               5


<210>  957
<211>  6
<212>  PRT
<213>  Artificial Sequence

<220>
<223>  Synthetic peptide

<400>  957

Ser Val Ser Ser Ala Ser
1               5


<210>  958
<211>  6
<212>  PRT
<213>  Artificial Sequence

<220>
<223>  Synthetic peptide

<400>  958

Ser Val Ser Ser Gly Gly
1               5


<210>  959
<211>  6
<212>  PRT
<213>  Artificial Sequence

<220>
<223>  Synthetic peptide

<400>  959

Ser Val Ser Ser Gly Ser
1               5


<210>  960
<211>  6
<212>  PRT
<213>  Artificial Sequence

<220>
<223>  Synthetic peptide

<400>  960

Ser Val Ser Ser Gly Thr
1               5


<210>  961
<211>  6
<212>  PRT
<213>  Artificial Sequence

<220>
<223>  Synthetic peptide

<400>  961

Ser Val Ser Ser Asn Gly
1               5


<210>  962
<211>  6
<212>  PRT
<213>  Artificial Sequence

<220>
<223>  Synthetic peptide

<400>  962

Ser Val Ser Ser Asn Gln
1               5

<210>  963
<211>  6
<212>  PRT
<213>  Artificial Sequence

<220>
<223>  Synthetic peptide

<400>  963

Ser Val Ser Ser Asn Ser
1               5


<210>  964
<211>  6
<212>  PRT
<213>  Artificial Sequence

<220>
<223>  Synthetic peptide

<400>  964

Ser Val Ser Ser Asn Thr
1               5


<210>  965
<211>  6
<212>  PRT
<213>  Artificial Sequence

<220>
<223>  Synthetic peptide

<400>  965

Ser Val Ser Ser Gln Gly
1               5


<210>  966
<211>  6
<212>  PRT
<213>  Artificial Sequence

<220>
<223>  Synthetic peptide

<400>  966

Ser Val Ser Ser Gln Gln
1               5


<210>  967
<211>  6
<212>  PRT
<213>  Artificial Sequence

<220>
<223>  Synthetic peptide

<400>  967

Ser Val Ser Ser Gln Ser
1               5


<210>  968
<211>  6
<212>  PRT
<213>  Artificial Sequence

<220>
<223>  Synthetic peptide

<400>  968

Ser Val Ser Ser Gln Thr
1               5


<210>  969
<211>  6
<212>  PRT
<213>  Artificial Sequence

<220>
<223>  Synthetic peptide

<400>  969

Ser Val Ser Ser Gln Val
1               5


<210>  970
<211>  6
<212>  PRT
<213>  Artificial Sequence

<220>
<223>  Synthetic peptide

<400>  970

Ser Val Ser Ser Ser Gly
1               5


<210>  971
<211>  6
<212>  PRT
<213>  Artificial Sequence

<220>
<223>  Synthetic peptide

<400>  971

Ser Val Ser Ser Ser Gln
1               5

<210>  972
<211>  6
<212>  PRT
<213>  Artificial Sequence

<220>
<223>  Synthetic peptide

<400>  972

Ser Val Ser Ser Ser Ser
1               5


<210>  973
<211>  6
<212>  PRT
<213>  Artificial Sequence

<220>
<223>  Synthetic peptide

<400>  973

Ser Val Ser Thr Ala Gly
1               5


<210>  974
<211>  6
<212>  PRT
<213>  Artificial Sequence

<220>
<223>  Synthetic peptide

<400>  974

Ser Val Ser Thr Ala Ser
1               5


<210>  975
<211>  6
<212>  PRT
<213>  Artificial Sequence

<220>
<223>  Synthetic peptide

<400>  975

Ser Val Ser Thr Ala Thr
1               5


<210>  976
<211>  6
<212>  PRT
<213>  Artificial Sequence

<220>
<223>  Synthetic peptide

<400>  976

Ser Val Ser Thr Gly Ser
1               5


<210>  977
<211>  6
<212>  PRT
<213>  Artificial Sequence

<220>
<223>  Synthetic peptide

<400>  977

Ser Val Ser Thr Asn Ser
1               5


<210>  978
<211>  6
<212>  PRT
<213>  Artificial Sequence

<220>
<223>  Synthetic peptide

<400>  978

Ser Val Ser Thr Asn Thr
1               5


<210>  979
<211>  6
<212>  PRT
<213>  Artificial Sequence

<220>
<223>  Synthetic peptide

<400>  979

Ser Val Ser Thr Gln Gln
1               5


<210>  980
<211>  6
<212>  PRT
<213>  Artificial Sequence

<220>
<223>  Synthetic peptide

<400>  980

Ser Val Ser Thr Gln Ser
1               5

<210>  981
<211>  6
<212>  PRT
<213>  Artificial Sequence

<220>
<223>  Synthetic peptide

<400>  981

Ser Val Ser Thr Gln Thr
1               5


<210>  982
<211>  6
<212>  PRT
<213>  Artificial Sequence

<220>
<223>  Synthetic peptide

<400>  982

Ser Val Ser Thr Ser Gly
1               5


<210>  983
<211>  6
<212>  PRT
<213>  Artificial Sequence

<220>
<223>  Synthetic peptide

<400>  983

Ser Val Ser Thr Ser Asn
1               5


<210>  984
<211>  6
<212>  PRT
<213>  Artificial Sequence

<220>
<223>  Synthetic peptide

<400>  984

Ser Val Ser Thr Ser Ser
1               5


<210>  985
<211>  6
<212>  PRT
<213>  Artificial Sequence

<220>
<223>  Synthetic peptide

<400>  985

Ser Val Ser Thr Ser Thr
1               5


<210>  986
<211>  6
<212>  PRT
<213>  Artificial Sequence

<220>
<223>  Synthetic peptide

<400>  986

Thr Ala Ser Gln Ser Gln
1               5


<210>  987
<211>  6
<212>  PRT
<213>  Artificial Sequence

<220>
<223>  Synthetic peptide

<400>  987

Thr Ala Ser Gln Val Gln
1               5


<210>  988
<211>  6
<212>  PRT
<213>  Artificial Sequence

<220>
<223>  Synthetic peptide

<400>  988

Thr Gly Ser Asn Ser Val
1               5


<210>  989
<211>  6
<212>  PRT
<213>  Artificial Sequence

<220>
<223>  Synthetic peptide

<400>  989

Thr Gly Ser Asn Val Ser
1               5

<210>  990
<211>  6
<212>  PRT
<213>  Artificial Sequence

<220>
<223>  Synthetic peptide

<400>  990

Thr Gly Ser Ser Asn Val
1               5


<210>  991
<211>  6
<212>  PRT
<213>  Artificial Sequence

<220>
<223>  Synthetic peptide

<400>  991

Thr Gly Ser Val Asn Ser
1               5


<210>  992
<211>  6
<212>  PRT
<213>  Artificial Sequence

<220>
<223>  Synthetic peptide

<400>  992

Thr Gly Ser Val Ser Asn
1               5


<210>  993
<211>  6
<212>  PRT
<213>  Artificial Sequence

<220>
<223>  Synthetic peptide

<400>  993

Thr Asn Ser Gly Ser Val
1               5


<210>  994
<211>  6
<212>  PRT
<213>  Artificial Sequence

<220>
<223>  Synthetic peptide

<400>  994

Thr Asn Ser Gly Val Ser
1               5



<210>  995
<211>  6
<212>  PRT
<213>  Artificial Sequence

<220>
<223>  Synthetic peptide

<400>  995

Thr Asn Ser Gln Val Gln
1               5



<210>  996
<211>  6
<212>  PRT
<213>  Artificial Sequence

<220>
<223>  Synthetic peptide

<400>  996

Thr Asn Ser Ser Gly Val
1               5



<210>  997
<211>  6
<212>  PRT
<213>  Artificial Sequence

<220>
<223>  Synthetic peptide

<400>  997

Thr Asn Ser Val Gly Ser
1               5



<210>  998
<211>  6
<212>  PRT
<213>  Artificial Sequence

<220>
<223>  Synthetic peptide

<400>  998

Thr Asn Ser Val Ser Gly
1               5

<210>  999
<211>  6
<212>  PRT
<213>  Artificial Sequence

<220>
<223>  Synthetic peptide

<400>  999

Thr Gln Ser Gln Ser Gln
1               5


<210>  1000
<211>  6
<212>  PRT
<213>  Artificial Sequence

<220>
<223>  Synthetic peptide

<400>  1000

Thr Gln Ser Gln Val Gln
1               5


<210>  1001
<211>  6
<212>  PRT
<213>  Artificial Sequence

<220>
<223>  Synthetic peptide

<400>  1001

Thr Gln Ser Thr Val Ser
1               5


<210>  1002
<211>  6
<212>  PRT
<213>  Artificial Sequence

<220>
<223>  Synthetic peptide

<400>  1002

Thr Gln Ser Val Ser Asn
1               5


<210>  1003
<211>  6
<212>  PRT
<213>  Artificial Sequence

<220>
<223>  Synthetic peptide

<400>  1003

Thr Gln Ser Val Ser Ser
1               5


<210>  1004
<211>  6
<212>  PRT
<213>  Artificial Sequence

<220>
<223>  Synthetic peptide

<400>  1004

Thr Ser Asn Gly Val Ser
1               5


<210>  1005
<211>  6
<212>  PRT
<213>  Artificial Sequence

<220>
<223>  Synthetic peptide

<400>  1005

Thr Ser Asn Val Gly Ser
1               5


<210>  1006
<211>  6
<212>  PRT
<213>  Artificial Sequence

<220>
<223>  Synthetic peptide

<400>  1006

Thr Ser Asn Val Ser Gly
1               5


<210>  1007
<211>  6
<212>  PRT
<213>  Artificial Sequence

<220>
<223>  Synthetic peptide

<400>  1007

Thr Ser Ser Gly Asn Val
1               5

<210>  1008
<211>  6
<212>  PRT
<213>  Artificial Sequence

<220>
<223>  Synthetic peptide

<400>  1008

Thr Ser Ser Gly Val Asn
1               5


<210>  1009
<211>  6
<212>  PRT
<213>  Artificial Sequence

<220>
<223>  Synthetic peptide

<400>  1009

Thr Ser Ser Asn Gly Val
1               5


<210>  1010
<211>  6
<212>  PRT
<213>  Artificial Sequence

<220>
<223>  Synthetic peptide

<400>  1010

Thr Ser Ser Asn Ser Val
1               5


<210>  1011
<211>  6
<212>  PRT
<213>  Artificial Sequence

<220>
<223>  Synthetic peptide

<400>  1011

Thr Ser Ser Asn Val Gly
1               5


<210>  1012
<211>  6
<212>  PRT
<213>  Artificial Sequence

<220>
<223>  Synthetic peptide

<400>  1012

Thr Ser Ser Gln Ser Gln
1               5


<210>  1013
<211>  6
<212>  PRT
<213>  Artificial Sequence

<220>
<223>  Synthetic peptide

<400>  1013

Thr Ser Ser Ser Val Gln
1               5


<210>  1014
<211>  6
<212>  PRT
<213>  Artificial Sequence

<220>
<223>  Synthetic peptide

<400>  1014

Thr Ser Ser Val Gly Asn
1               5


<210>  1015
<211>  6
<212>  PRT
<213>  Artificial Sequence

<220>
<223>  Synthetic peptide

<400>  1015

Thr Ser Ser Val Asn Gly
1               5


<210>  1016
<211>  6
<212>  PRT
<213>  Artificial Sequence

<220>
<223>  Synthetic peptide

<400>  1016

Thr Ser Ser Val Ser Gly
1               5

<210>  1017
<211>  6
<212>  PRT
<213>  Artificial Sequence

<220>
<223>  Synthetic peptide

<400>  1017

Thr Ser Ser Val Ser Ser
1               5


<210>  1018
<211>  6
<212>  PRT
<213>  Artificial Sequence

<220>
<223>  Synthetic peptide

<400>  1018

Thr Ser Ser Val Thr Ser
1               5


<210>  1019
<211>  6
<212>  PRT
<213>  Artificial Sequence

<220>
<223>  Synthetic peptide

<400>  1019

Thr Val Asn Ser Gly Ser
1               5


<210>  1020
<211>  6
<212>  PRT
<213>  Artificial Sequence

<220>
<223>  Synthetic peptide

<400>  1020

Thr Val Asn Ser Ser Gly
1               5


<210>  1021
<211>  6
<212>  PRT
<213>  Artificial Sequence

<220>
<223>  Synthetic peptide

<400>  1021

Thr Val Ser Gly Asn Ser
1               5


<210>  1022
<211>  6
<212>  PRT
<213>  Artificial Sequence

<220>
<223>  Synthetic peptide

<400>  1022

Thr Val Ser Gly Ser Asn
1               5


<210>  1023
<211>  6
<212>  PRT
<213>  Artificial Sequence

<220>
<223>  Synthetic peptide

<400>  1023

Thr Val Ser Asn Gly Ser
1               5


<210>  1024
<211>  6
<212>  PRT
<213>  Artificial Sequence

<220>
<223>  Synthetic peptide

<400>  1024

Thr Val Ser Asn Ser Gly
1               5


<210>  1025
<211>  6
<212>  PRT
<213>  Artificial Sequence

<220>
<223>  Synthetic peptide

<400>  1025

Thr Val Ser Asn Ser Thr
1               5

<210>  1026
<211>  6
<212>  PRT
<213>  Artificial Sequence

<220>
<223>  Synthetic peptide

<400>  1026

Thr Val Ser Asn Val Gly
1               5


<210>  1027
<211>  6
<212>  PRT
<213>  Artificial Sequence

<220>
<223>  Synthetic peptide

<400>  1027

Thr Val Ser Gln Asn Gln
1               5


<210>  1028
<211>  6
<212>  PRT
<213>  Artificial Sequence

<220>
<223>  Synthetic peptide

<400>  1028

Thr Val Ser Gln Gln Val
1               5


<210>  1029
<211>  6
<212>  PRT
<213>  Artificial Sequence

<220>
<223>  Synthetic peptide

<400>  1029

Thr Val Ser Gln Ser Gly
1               5


<210>  1030
<211>  6
<212>  PRT
<213>  Artificial Sequence

<220>
<223>  Synthetic peptide

<400>  1030

Thr Val Ser Gln Ser Gln
1               5


<210>  1031
<211>  6
<212>  PRT
<213>  Artificial Sequence

<220>
<223>  Synthetic peptide

<400>  1031

Thr Val Ser Ser Gly Asn
1               5


<210>  1032
<211>  6
<212>  PRT
<213>  Artificial Sequence

<220>
<223>  Synthetic peptide

<400>  1032

Thr Val Ser Ser Asn Gly
1               5


<210>  1033
<211>  6
<212>  PRT
<213>  Artificial Sequence

<220>
<223>  Synthetic peptide

<400>  1033

Thr Val Ser Ser Asn Gln
1               5


<210>  1034
<211>  6
<212>  PRT
<213>  Artificial Sequence

<220>
<223>  Synthetic peptide

<400>  1034

Thr Val Ser Ser Asn Ser
1               5

<210>  1035
<211>  6
<212>  PRT
<213>  Artificial Sequence

<220>
<223>  Synthetic peptide

<400>  1035

Thr Val Ser Ser Ser Gln
1               5


<210>  1036
<211>  6
<212>  PRT
<213>  Artificial Sequence

<220>
<223>  Synthetic peptide

<400>  1036

Thr Val Ser Thr Ser Gly
1               5


<210>  1037
<211>  6
<212>  PRT
<213>  Artificial Sequence

<220>
<223>  Synthetic peptide

<400>  1037

Thr Val Ser Thr Ser Asn
1               5


<210>  1038
<211>  6
<212>  PRT
<213>  Artificial Sequence

<220>
<223>  Synthetic peptide

<400>  1038

Thr Val Ser Thr Ser Ser
1               5


<210>  1039
<211>  6
<212>  PRT
<213>  Artificial Sequence

<220>
<223>  Synthetic peptide

<400>  1039

Thr Val Ser Thr Ser Thr
1               5


<210>  1040
<211>  6
<212>  PRT
<213>  Artificial Sequence

<220>
<223>  Synthetic peptide

<400>  1040

Val Gly Ser Thr Asn Ser
1               5


<210>  1041
<211>  6
<212>  PRT
<213>  Artificial Sequence

<220>
<223>  Synthetic peptide

<400>  1041

Val Asn Ser Thr Ser Gly
1               5


<210>  1042
<211>  6
<212>  PRT
<213>  Artificial Sequence

<220>
<223>  Synthetic peptide

<400>  1042

Val Asn Ser Thr Ser Asn
1               5


<210>  1043
<211>  6
<212>  PRT
<213>  Artificial Sequence

<220>
<223>  Synthetic peptide

<400>  1043

Val Ser Ser Gln Ser Gln
1               5

<210>  1044
<211>  6
<212>  PRT
<213>  Artificial Sequence

<220>
<223>  Synthetic peptide

<400>  1044

Val Ser Ser Gln Val Gln
1               5


<210>  1045
<211>  6
<212>  PRT
<213>  Artificial Sequence

<220>
<223>  Synthetic peptide

<400>  1045

Val Ser Ser Thr Asn Gly
1               5


<210>  1046
<211>  6
<212>  PRT
<213>  Artificial Sequence

<220>
<223>  Synthetic peptide

<400>  1046

Val Thr Ser Asn Ser Gly
1               5


<210>  1047
<211>  6
<212>  PRT
<213>  Artificial Sequence

<220>
<223>  Synthetic peptide

<400>  1047

Val Thr Ser Gln Ser Gln
1               5


<210>  1048
<211>  6
<212>  PRT
<213>  Artificial Sequence

<220>
<223>  Synthetic peptide

<400>  1048

Val Glu Ala Leu Tyr Leu
1               5


<210>  1049
<211>  6
<212>  PRT
<213>  Artificial Sequence

<220>
<223>  Synthetic peptide

<400>  1049

Leu Tyr Gln Leu Glu Asn
1               5


<210>  1050
<211>  6
<212>  PRT
<213>  Artificial Sequence

<220>
<223>  Synthetic peptide

<400>  1050

Gly Tyr Val Ile Ile Lys
1               5


<210>  1051
<211>  6
<212>  PRT
<213>  Artificial Sequence

<220>
<223>  Synthetic peptide

<400>  1051

Ser Asn Gln Asn Asn Phe
1               5


<210>  1052
<211>  6
<212>  PRT
<213>  Artificial Sequence

<220>
<223>  Synthetic peptide

<400>  1052

Ser Val Leu Thr Ser Leu
1               5

<210>  1053
<211>  6
<212>  PRT
<213>  Artificial Sequence

<220>
<223>  Synthetic peptide

<400>  1053

Ser Ser Thr Asn Val Gly
1               5


<210>  1054
<211>  6
<212>  PRT
<213>  Artificial Sequence

<220>
<223>  Synthetic peptide

<400>  1054

Ser Val Ser Ser Ser Tyr
1               5


<210>  1055
<211>  6
<212>  PRT
<213>  Artificial Sequence

<220>
<223>  Synthetic peptide

<400>  1055

Gly Ala Ile Leu Ser Ser
1               5


<210>  1056
<211>  6
<212>  PRT
<213>  Artificial Sequence

<220>
<223>  Synthetic peptide

<400>  1056

Gly Ala Ile Ile Gly Leu
1               5


<210>  1057
<211>  6
<212>  PRT
<213>  Artificial Sequence

<220>
<223>  Synthetic peptide

<400>  1057

Ala Ile Ile Gly Leu Met
1               5


<210>  1058
<211>  6
<212>  PRT
<213>  Artificial Sequence

<220>
<223>  Synthetic peptide

<400>  1058

Gly Gly Val Val Ile Ala
1               5


<210>  1059
<211>  6
<212>  PRT
<213>  Artificial Sequence

<220>
<223>  Synthetic peptide

<400>  1059

Ser Val Asn Asp Leu Val
1               5


<210>  1060
<211>  6
<212>  PRT
<213>  Artificial Sequence

<220>
<223>  Synthetic peptide

<400>  1060

Leu Lys Val Lys Val Leu
1               5


<210>  1061
<211>  6
<212>  PRT
<213>  Artificial Sequence

<220>
<223>  Synthetic peptide

<400>  1061

Asn Lys Gly Ala Ile Ile
1               5

<210>  1062
<211>  6
<212>  PRT
<213>  Artificial Sequence

<220>
<223>  Synthetic peptide

<400>  1062

Asn Val Ser Thr Ser Gly
1               5


<210>  1063
<211>  41
<212>  PRT
<213>  Homo sapiens

<400>  1063

Ala Glu Phe Arg His Asp Ser Gly Tyr Glu Val His His Gln Lys Leu
1               5                   10                  15

Val Phe Phe Ala Glu Asp Val Gly Ser Asn Lys Gly Ala Ile Ile Gly
            20                  25                  30

Leu Met Val Gly Gly Val Val Ile Ala
        35                  40


<210>  1064
<211>  40
<212>  PRT
<213>  Homo sapiens

<400>  1064

Asp Ala Glu Phe Arg His Asp Ser Gly Tyr Glu Val His His Gln Lys
1               5                   10                  15


Leu Val Phe Phe Ala Glu Asp Val Gly Ser Asn Lys Gly Ala Ile Ile
            20                  25                  30


Gly Leu Met Val Gly Gly Val Val
        35                  40


<210>  1065
<211>  21
<212>  PRT
<213>  Homo sapiens

<400>  1065

Met Glu Val Gly Trp Tyr Arg Ser Pro Phe Ser Arg Val Val His Leu
1               5                   10                  15

Tyr Arg Asn Gly Lys
             20